# EUROPEAN PATENT APPLICATION

(11) **EP 4 810 038 A1**
(43) Date of publication of application: **23.09.2026**
(21) Application number: 24890763.6
(22) Date of filing: 14.11.2024
(51) Int. Cl.: A61K 31/43, C07D 498/18, A61P 35/00

(54) **METHOD FOR TREATING ALK-POSITIVE OR ROS1-POSITIVE NON-SMALL CELL LUNG CANCER**

(30) Priority: 15.11.2023 CN 202311531707; 05.11.2024 CN 202411573923
(71) Applicant: Shenzhen TargetRx Co., Ltd., Shenzhen, Guangdong 518057 (CN)
(72) Inventor: WANG, Yihan, Shenzhen, Guangdong 518057 (CN); CAO, Jingrong, Shenzhen, Guangdong 518057 (CN); LIU, Yun, Shenzhen, Guangdong 518057 (CN); LI, Huanyin, Shenzhen, Guangdong 518057 (CN)
(74) Representative: Novitas Patent AB
(86) International application number: PCT/CN2024/132017
(87) International publication number: WO 2025/103408

(57) **Abstract**

Provided is a method for treating ALK-positive non-small cell lung cancer in a subject by means of administering a compound A or a pharmaceutically acceptable salt thereof to the subject. Further provided is a method for treating ROS1-positive non-small cell lung cancer in a subject by means of administering compound A or a pharmaceutically acceptable salt thereof to the subject.

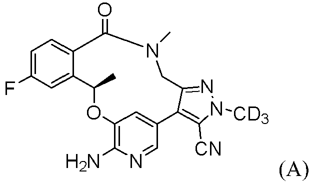

## Description

### TECHNICAL FIELD

The present disclosure belongs to the pharmaceutical field, specifically relates to a method of treating non-small cell lung cancer, such as ALK-positive and/or ROS1-positive non-small cell lung cancer, with a macrocyclic compound (10R)-7-amino-12-fluoro-2-(methyl-d3)-10,16-dimethyl-15-oxo-10,15,16,17-tetrahydro-2H-8,4-(metheno)pyrazolo[4,3-h][2,5,11]benzoxadiazacyclotetradecine-3-carbonitrile (compound A) or a pharmaceutically acceptable salt thereof.

### BACKGROUND

Anaplastic lymphoma kinase (ALK) is a receptor-type protein tyrosine kinase belonging to the insulin receptor superfamily. It was discovered in 1994 by Morris and Shiota et al. as a product of chromosomal rearrangement in anaplastic large cell lymphoma (ALCL). The most common fusion mode is the fusion of the NPM (Nucleophosmin) gene on chromosome 5 with the ALK gene on chromosome 2. The NPM-ALK fusion protein is detected in nearly 75% of the ALK-positive ALCL patients. Some subsequent studies have found that different ALK fusion forms were found in many types of cancer, including inflammatory myofibroblastic tumor and diffuse large B-cell lymphoma. Nevertheless, the importance of ALK as an effective target for the anti-tumor drugs is not fully recognized. It wasn't until 2007, when Soda et al. discovered that the EML4-ALK fusion protein accounted for 5% of non-small cell lung cancer (NSCLC), that the importance of ALK as a target for the anti-tumor drugs became apparent. This is because the number of cancer patients worldwide is enormous, with lung cancer being the most prevalent. The United States sees over 8,000 new cases of ALK-positive lung cancer annually, while China has over 65,000 new cases each year, and the global 5-year survival rate for lung cancer is only 15%. What attracts attention is that, patients who are positive for the EML4-ALK gene generally do not carry epidermal growth factor receptor (EGFR) or Kirsten rat sarcoma virus (KRAS) mutations, making the EML4-ALK fusion gene a unique molecular target for NSCLC. Furthermore, amplification or a point mutation of ALK gene has been found in neuroblastomas, anaplastic thyroid cancer, and ovarian cancer.

ROS1 is a proto-oncogene receptor tyrosine kinase belonging to the insulin receptor subfamily, and it is involved in the cell proliferation and differentiation processes. ROS1 is expressed in the epithelial cells of various tissues of human. ROS1 expression and/or activation have been found in glioblastoma and tumors of the central nervous system. The resulting abnormal fusion protein of ROS1 kinase involves the genetic alterations in ROS1, including the FIG-ROS1 deletion and translocation in glioblastoma and non-small cell lung cancer (NSCLC), SLC34A2-ROS1 translocation in NSCLC, and CD74-ROS1 translocation in NSCLC and cholangiocarcinoma. Other fusions have also been found in tumor samples from lung cancer patients, including TPM3-ROS1, SDC4-ROS1, EZR-ROS1 and LRTG3-ROS1.

### SUMMARY

The present disclosure provides use and method of a compound A or a pharmaceutically acceptable salt thereof in treating non-small cell lung cancer, such as ALK-positive and/or ROS1-positive non-small cell lung cancer.

In one aspect, the present disclosure discloses use of a compound A or a pharmaceutically acceptable salt thereof in the manufacture of a medicament for the treatment of non-small cell lung cancer, wherein the compound A has the following structure:

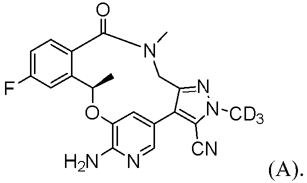

In another aspect, the present disclosure discloses a method of treating non-small cell lung cancer in a patient, comprising administering to the patient a therapeutically effective amount of a compound A or a pharmaceutically acceptable salt thereof, wherein the compound A has the following structure:

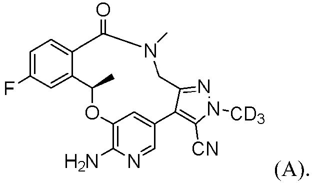

In another aspect, the present disclosure discloses a compound A or a pharmaceutically acceptable salt thereof, for use in the treatment of non-small cell lung cancer, wherein the compound A has the following structure:

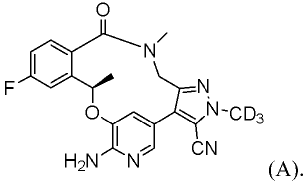

In another aspect, the present disclosure discloses a method of determining the concentration of the compound A in the CNS fluid of a subject with ALK-positive and/or ROS1-positive CNS metastatic non-small cell lung cancer, comprising:
a. collecting a cerebrospinal fluid sample; and
b. measuring the concentration of the compound A in the CSF sample to determine the concentration of the compound A present in the CNS fluid.

### BRIEF DESCRIPTION OF DRAWINGS

Fig. 1 shows the growth curves of tumor volume in the mice of the solvent control group, the positive control group of crizotinib (100 mg/kg), and the high-dose (10 mg/kg), medium-dose (3 mg/kg), and low-dose (1 mg/kg) groups of the assay drug compound A in the Ba/F3(EML4-ALK L1196M) subcutaneous tumor model.
Fig. 2 shows the weight change curves over treatment time of the solvent control group, the positive control group of crizotinib (100 mg/kg), and the high-dose (10 mg/kg), medium-dose (3 mg/kg), and low-dose (1 mg/kg) groups of the assay drug compound A in the Ba/F3(EML4-ALK L1196M) subcutaneous tumor model.
Fig. 3 shows the percentage change curves in body weight over treatment time of the solvent control group, the positive control group of crizotinib (100 mg/kg), and the high-dose (10 mg/kg), medium-dose (3 mg/kg), and low-dose (1 mg/kg) groups of the assay drug compound A in the Ba/F3 (EML4-ALK L1196M) subcutaneous tumor model.
Fig. 4 shows the growth curves of tumor volume in the mice of the solvent control group, the positive control group of crizotinib (100 mg/kg), and the high-dose (15 mg/kg), medium-dose (5 mg/kg), and low-dose (1.5 mg/kg) groups of the assay drug compound A in the Ba/F3(EML4-ALK G1202R) subcutaneous tumor model.
Fig. 5 shows the weight change curves over treatment time of the solvent control group, the positive control group of crizotinib (100 mg/kg), and the high-dose (15 mg/kg), medium-dose (5 mg/kg), and low-dose (1.5 mg/kg) groups of the assay drug compound A in the Ba/F3(EML4-ALK G1202R) subcutaneous tumor model.
Fig. 6 shows the percentage change curves in body weight over treatment time of the solvent control group, the positive control group of crizotinib (100 mg/kg), and the high-dose (15 mg/kg), medium-dose (5 mg/kg), and low-dose (1.5 mg/kg) groups of the assay drug compound A in the Ba/F3(EML4-ALK G1202R) subcutaneous tumor model.
Fig. 7 shows the growth curves of tumor volume in the mice of the solvent control group, the positive control group of crizotinib (100 mg/kg), and the high-dose (15 mg/kg), medium-dose (5 mg/kg), and low-dose (1.5 mg/kg) groups of the assay drug compound A in the Ba/F3 (SLC34A2-ROS1G2032R) subcutaneous tumor model.
Fig. 8 shows the body weight change curves over treatment time of the solvent control group, the positive control group of crizotinib (100 mg/kg), and the high-dose (15 mg/kg), medium-dose (5 mg/kg), and low-dose (1.5 mg/kg) groups of the assay drug compound A in the Ba/F3 (SLC34A2-ROS1 G2032R) subcutaneous tumor model.
Fig. 9 shows the percentage change curves in body weight over treatment time of the solvent control group, the positive control group of crizotinib (100 mg/kg), and the high-dose (15 mg/kg), medium-dose (5 mg/kg), and low-dose (1.5 mg/kg) groups of the assay drug compound A in the Ba/F3 (SLC34A2-ROS1 G2032R) subcutaneous tumor model.
Fig. 10 is a waterfall chart of the best change percentages of the sum of the whole-body diameters of tumor relative to the baseline in the ALK-positive subjects evaluated for efficacy after baseline during the indication expansion phase - full analysis set (RP2D dose group), wherein Fig. 10a shows the results of 93 subjects who had previously received a second-generation TKI treatment, Fig. 10b shows the results of 14 subjects who had previously received crizotinib treatment, and Fig. 10c shows the results of 33 ALK TKI treatment-naive patients.
Fig. 11a shows the results of the systemic progression-free survival of the ALK-positive subjects during the indication expansion phase.
Fig. 11b shows the Kaplan-Meier curve of the progression-free survival of the ALK-positive subjects during the indication expansion phase - full analysis set (primary analysis).
Fig. 12a shows the results of the intracranial progression-free survival of the ALK-positive subjects during the indication expansion phase, wherein group A cohort 2 includes 5 subjects with intracranial metastases in the 60mg group in the dose escalation and expansion phases.
Fig. 12b shows the Kaplan-Meier curve of the overall survival of the ALK-positive subjects during the indication expansion phase - full analysis set (primary analysis).
Fig. 12c shows the Kaplan-Meier curve of the progression-free survival of the ROS1-positive subjects during the indication expansion phase - full analysis set (primary analysis).
Fig. 12d shows the Kaplan-Meier curve of the overall survival of the ROS1-positive subjects during the indication expansion phase - full analysis set (primary analysis).
Fig. 13 shows the progression-free survival (PFS) of the patients with mutant or wild-type TP53 treated with the compound A in the ALK TKI drug resistance subgroup.
Fig. 14 is a waterfall chart of the best change percentages of the sum of the whole-body tumor diameters relative to the baseline (IRC assessment results) - full analysis set.
Fig. 15 shows the Kaplan-Meier curve of the PFS (IRC assessment results) - full analysis set.
Fig. 16 is a waterfall chart of the best change percentages of the sum of the intracranial tumor diameters relative to the baseline (IRC assessment results) - full analysis set.
Fig. 17 shows the Kaplan-Meier curve of the IC-PFS (IRC assessment results) - full analysis set.
Fig. 18 is a forest plot of the objective response rate (%) for each subgroup (IRC assessment results) - full analysis set.

### DETAILED DESCRIPTION

The present disclosure discovered that compound A can be used in the treatment of ALK-positive and/or ROS1-positive non-small cell lung cancer. Therefore, the present disclosure discloses use and method of the compound A or a pharmaceutically acceptable salt thereof in treating ALK-positive and/or ROS1-positive non-small cell lung cancer.

Abbreviations

| **English abbreviations** | **Full English name** |
|---|---|
| AE | Adverse Event |
| ALK | Anaplastic lymphoma kinase |
| ALT | Alanine transaminase |
| ALP | Alkaline phosphatase |
| ANC | Absolute neutrophil count |
| APTT | Activated partial thromboplastin time |
| AST | Aspartate amino transferase |
| AUC | Area under the curve |
| BOR | Best over response |
| IC-BOR | Intracranial Best over response |
| CYP3A4 | Cytochrome P450 Family 3 Subfamily A Member 4 |
| CI | Confidence interval |
| CL | Systemic clearance |
| CL/F | Apparent clearance |
| Cₘₐₓ | Maximum concentration |
| Cₘᵢₙ | Minimum concentration |
| CNS | Central nervous system |
| CR | Complete Remission |
| CSCO | Chinese Society of Clinical Oncology |
| CSF | Cerebrospinal fluid |
| DDI | Drug-Drug Interaction |
| DOR | Duration of Response |
| IC-DOR | Intracranial Duration of Response |
| ECOG | Eastern Cooperative Oncology Group |
| eCRF | electronic Case Report Form |
| FAS | Full Analysis Set |
| FISH | Fluorescence in situ hybridization |
| HBV | Hepatitis B virus |
| HCV | Hepatitis C virus |
| HIV | Human Immunodeficiency Virus |
| IHC | Immunohistochemistry |
| INR | International Normalized Ratio |
| IRC | Independent Reading Committee |
| MedDRA | Medical Dictionary for Regulatory Activities |
| MTD | Maximum Tolerance dose |
| NSCLC | Nonsmall-cell lung cancer |
| ORR | Objective Response Rate/Overall Response Rate |
| IC-ORR | Intracranial objective response rate |
| DCR | Disease control rate |
| IC-DCR | Intracranial disease control rate |
| OS | Overall Survival |
| PD | Progressive Disease/Progressed Disease |
| PFS | Progress Free Survival |
| IC-PFS | Intracranial Progress Free Survival |
| PK | Pharmacokinetic |
| PR | Partial Remission/Partial Response |
| QD | Quaque die |
| RECIST | Response Evaluation Criteria In Solid Tumors |
| ROS1 | C-ros oncogene 1 receptor tyrosine kinase |
| RT-PCR | Reverse Transcription-Polymerase Chain Reaction |
| SD | Stable Disease |
| T_{1/2} | Half-life |
| Tₘₐₓ | Peak time |
| TGI | The Tumor Growth Inhibition value |
| TKI | Tyrosine kinase inhibitors |
| TTR | Time To Response |
| IC-TTR | Intracranial Time To Response |
| TRAE | Treatment-related adverse events |
| ULN | Upper limit of normal |
| DLT | Dose-limiting toxicity |
| NE | Not evaluable |
| NR | Not reached |
| GM | geometric mean |
| CV | coefficient of variation |

### Definition

The present disclosure can be more readily understood by referring to the following detailed description of the embodiments of the present disclosure and the examples included herein. It should be understood that the terms used herein are only for the purpose of describing the specific embodiments and are not intended to be limiting. It should be further understood that, unless specifically defined in the text, the terms used herein should be given their ordinary meanings known in the relevant art.

As used herein, unless otherwise indicated, the singular forms "a", "an" and "the" include plural references. For example, "a" compound includes one or more compounds.

As used herein, unless otherwise indicated, the use of "or" means "and/or".

The term "include", "comprise", "contain" and "have" and other forms of use, such as "including", "comprising", "containing" and "having" are not restrictive.

The section headings used herein are for organizational purposes only and are not intended to be interpreted as limiting the object being described. All documents or parts thereof cited herein, including but not limited to patents, patent applications, articles, books, guidelines and discussions, are expressly incorporated herein by reference in their entirety for any purpose.

A "subject" includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In some embodiments, the subject is a human. In some embodiments, the subject is a non-human animal. The terms "human", "patient", and "subject" are used interchangeably herein.

"Disease", "disorder" and "condition" are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplate an action that occurs before a subject begins to suffer from the specified disease, disorder or condition ("prophylactic treatment").

"Therapeutically effective amount" refers to a dosage that is sufficient to stop, delay the progression of, or cause the regression of a disease, or that can reduce the symptoms caused by a disease, for example, a cancer, such as lung cancer (e.g., NSCLC).

"Non-small cell lung cancer (NSCLC)" refers to any type of lung cancer that differs from small cell lung cancer. NSCLC includes squamous cell carcinoma (SQCC), adenocarcinoma (ADC) and large-cell carcinoma. Both ADC and large-cell carcinoma are classified as non-squamous cell carcinoma. ADC can be divided into subtypes, including acinar adenocarcinoma, papillary adenocarcinoma, bronchioloalveolar carcinoma (BAC), solid adenocarcinoma, and mixed subtypes (2004 World Health Organization classification of lung tumors, Beasley et al., Semin. Roentgenol. 40:90-97, 2004). ADC accounts for approximately 40% of all lung cancers and is the most common form of lung cancer among the never-smokers. ADC is classified as a non-squamous cell type of NSCLC. Histologically, ADC shows the glandular formation, papillary structures, or solid growth with mucin production. Large-cell carcinoma includes subtypes such as giant cell carcinoma, clear cell carcinoma, adenosquamous carcinoma, and undifferentiated carcinoma.

A "sample" is a biological specimen obtained from a subject that contains genomic DNA, RNA (including mRNA), protein, or other combinations thereof. Examples include a specimen containing at least one type of NSCLC cells ("NSCLC sample"), such as tissue or tumor biopsy, fine-needle aspiration, bronchoalveolar lavage fluid, pleural fluid, sputum, surgical specimen, lymph node, NSCLC metastasis, peripheral blood, or autopsy material. In other examples, a sample includes a control, such as a non-NSCLC cell or tissue sample.

"ALK" is a transmembrane receptor tyrosine kinase belonging to the insulin receptor subfamily. ALK receptor tyrosine kinase (RTK) was initially identified due to its involvement in a subtype of human non-Hodgkin's lymphoma known as anaplastic large cell lymphoma (ALCL). ALK typically has a restricted distribution in mammalian cells, and its significant presence only in the nervous system during embryonic development suggests a role for ALK in the brain development.

In addition to its role in normal development, full-length normal ALK expression has been detected in the cell lines derived from various tumors, such as glioblastoma, neuroectodermal tumors and glioblastoma, as well as breast cancer and melanoma cell lines.

Like other RTKs, translocations affect the ALK gene, thereby leading to the expression of fusion kinases in the original cells, most commonly NPM-ALK. For example, approximately 60 percent of anaplastic large cell lymphomas (ALCL) are associated with the chromosomal mutations that produce a fusion protein consisting of the intracellular domain of nucleophosmin (NMP) ALK. This mutant protein, NPM-ALK, possesses a constitutively active tyrosine kinase domain, which is responsible for its oncogenic properties by activating the downstream effectors. Experimental data have demonstrated that the abnormal expression of constitutively active ALK is directly involved in the pathogenesis of ALCL, and that such inhibition of ALK can significantly hinder the growth of ALK-positive lymphoma cells. Constitutively activated chimeric ALK has been shown to be present in approximately 60% of the inflammatory myofibroblastic tumors (IMT) (a slow-growing sarcoma that primarily affects children and young adults). In addition, current reports have described the occurrence of a variant ALK fusion TPM4-ALK in the case of squamous cell carcinoma (SCC) of the esophagus. Therefore, ALK is one of the few instances of RTK involved in the tumorigenesis in both non-hematopoietic and hematopoietic malignancies. Recently, it has been shown that an inversion within chromosome 2p leads to the formation of part of a fusion gene in the non-small cell lung cancer (NSCLC) cells containing the echinoderm microtubule-associated protein-like 4 (EML4) gene and the anaplastic lymphoma kinase (ALK) gene. Exemplary ALK fusions in NSCLC include EML4-ALK, KIF5B-ALK, KLC1-ALK, PTPN3-ALK, HIP1-ALK, TPR-ALK, STRN-ALK, PPM1B-ALK, EIF2AK3-ALK, SPDYA-ALK, ASXL2-ALK, BIRC6-ALK, PICALM-ALK, CRIM1-ALK, CUX-ALK, FAM179A-ALK, COL25A1-ALK, BIRC6-ALK, PICALM-ALK, etc., among which EML4-ALK is more common. Detection of the ALK fusion gene can be performed using IHC, FISH, RT-PCR, or HTS method.

"ALK-positive" refers to the chromosomal rearrangements and/or ALK overexpression and/or ALK point mutations in tumors with ALK fusion genes. In some embodiments, an ALK-positive result requires a test result from one of the following tests: FISH, RT-PCR, IHC, or NGS (high-throughput sequencing) as a diagnostic evidence.

"ROS1" belongs to the sevenless subfamily of the insulin receptor genes of the receptor tyrosine kinase. The protein encoded by this gene is a type I integral membrane protein with tyrosine kinase activity. ROS1 shares structural similarities with ALK proteins. Gene rearrangements involving ROS1 have been identified in various cancers. The small-molecule tyrosine kinase inhibitor crizotinib has been approved for the treatment of patients with metastatic NSCLC, whose tumors are either ROS1-positive or ALK-positive. In non-small cell lung cancer, exemplary ROS1 fusions include SLC34A2-ROS1, CD74-ROS1, TPM3-ROS1, SDC4-ROS1, EZR-ROS1, LRIG3-ROS1, KDELR2-ROS1, CCDC6-ROS1, FIG-ROS1, TPD52L1-ROS1, FYN-ROS1, MKX-ROS1, CLTC-ROS1, LIMA1-ROS1, etc., among which SLC34A2-ROS1, CD74-ROS1, EZR-ROS1 or SDC4-ROS1 is more common.

"SLC34A2-ROS1 fusion protein or gene" refers to a somatic gene fusion of the partner SLC34A2 and ROS1. In some embodiments, the SLC34A2-ROS1 fusion protein includes an N-terminal domain of a fusion partner such as SLC34A2 and a C-terminal kinase domain of the ROS1 protein. The N-terminal domain of the fusion partner may be located at the N-terminus of the fusion protein, and the C-terminal kinase domain of the ROS1 protein may be located at the C-terminus of the fusion protein. The fusion partner may be the N-terminal domain of the SLC34A2 protein located at the N-terminus of the fusion protein. In this case, the fusion protein may be represented as the SLC34A2-ROS1 protein comprising the N-terminal domain of the SLC34A2 protein at the N-terminus and the C-terminal kinase domain of the ROS1 protein at the C-terminus. In another embodiment, a fusion gene encoding a fusion protein is provided, wherein the gene encoding the N-terminal domain of the fusion partner is located at the 5' end, and the gene encoding the C-terminal kinase domain of the ROS1 protein is located at the 3' end. In some embodiments, a portion of the SLC34A2 protein and a portion of the ROS1 protein containing a functional kinase domain are in-frame fused due to an aberrant chromosomal translocation resulting from a ROS1 chromosomal rearrangement in the presence of SLC34A2. In some embodiments, the SLC34A2-ROS1 fusion protein may include fusion exon 4 of SLC34A2 and fusion exon 32 of ROS1. In some embodiments, the SLC34A2-ROS1 fusion protein may include fusion exon 4 of SLC34A2 and fusion exon 34 of ROS1.

"ROS1-positive" refers to having a chromosomal rearrangement of ROS1 fusion gene and/or ROS1 overexpression in the tumor. In some embodiments, a ROS1-positive result requires a test result from one of the following tests: FISH, RT-PCR, or NGS, as a diagnostic evidence.

"ALK inhibitor" is a molecule that inhibits or reduces the activity of ALK, such as the tyrosine kinase activity of ALK. In some examples, the ALK inhibitor can be a small molecule, a protein (such as an antibody), or a nucleic acid (such as an antisense molecule). An ALK inhibitor can inhibit or reduce the binding of a ligand (such as a pleiotropic protein) to ALK, and thus reducing the tyrosine kinase activity of ALK. An ALK inhibitor can also directly inhibit or reduce the tyrosine kinase activity of ALK, for example, an ATP-competitive inhibitor (such as crizotinib). Molecules that reduce or inhibit ALK expression, such as antisense molecules, are also ALK inhibitors. In some examples, an ALK inhibitor inhibits or reduces the activity of genetically altered ALK, such as ALK gene fusions (including but not limited to ALK-EML4 gene fusion). An ALK inhibitor can specifically inhibit the tyrosine kinase activity of ALK, or in addition to inhibiting the tyrosine kinase activity of ALK, can inhibit the activity of other receptor tyrosine kinases (such as the activity of c-Met/HGFR). Hua Gao et al., (Synthesis and clinical application of small-molecule inhibitors and PROTACs of anaplastic lymphoma kinase. Bioorganic Chemistry 140 (2023) 106807) disclosed the small-molecule ALK inhibitors that are already on the market and in various stages of development in Table 1. This article is incorporated herein by reference in its entirety for all purposes.

"Generation one, generation two or generation three ALK inhibitors" refers to the first-generation, second-generation or third-generation ALK inhibitors.

For the information on "the first-line, the second-line, the third-line, the fourth-line, the fifth-line therapies, etc. for ALK-positive or ROS1-positive non-small cell lung cancer", please refer to the "Chinese Guidelines on the Diagnosis and Treatment of Primary Lung Cancer" updated by the Chinese Society of Clinical Oncology (CSCO) Expert Committee in 2022.

"Advanced non-small cell lung cancer" is defined as non-small cell lung cancer in stage IIIB, IIIC, or IV, which can be referenced in the "Chinese Guidelines on the Diagnosis and Treatment of Primary Lung Cancerr" updated in 2022.

The PK parameters for single-dose administration mainly include peak concentration (Cₘₐₓ), time to peak concentration (Tₘₐₓ), half-life (T_{1/2}), systemic clearance (CL), volume of distribution (Vd), AUC₀₋ₜ, and AUC_{0-∞}. The PK parameters for multiple-dose administration mainly include the area under the steady-state plasma concentration-time curve (AUCₛₛ), steady-state peak plasma concentration (C_{max, ss}), steady-state trough plasma concentration (C_{min, ss}), and steady-state time to peak plasma concentration (T_{max, ss}).

An adverse event will be described using MedDRA (Medical Dictionary for Regulatory Activities). Analysis of an adverse event will be based on a treatment emergent adverse event (TEAE). TEAE is defined as an adverse event that occur or worsen after a treatment/medication.

The anti-tumor efficacy is evaluated according to RECIST version 1.1. For patients initially evaluated as having a complete response (CR) or partial response (PR), efficacy should be confirmed at least 4 weeks later. In the patients with asymptomatic central nervous system (CNS) metastases, in addition to evaluating 5 extracranial target lesions, up to 5 intracranial target lesions will also be evaluated. The specific indicators are as follows:

"CR" is defined as the disappearance of all target lesions, and the diameters of the short axis of all the pathological lymph nodes (whether target or non-target lymph nodes) must be reduced to 10 mm or less.

"PR" is defined as a reduction of at least 30% in the total diameter of the target lesions, with the total diameter of the baseline as a reference value.

"PD" is defined as an increase of at least 20% in the total diameter of the target lesions, using the minimum total diameter in the study as a reference value (this value may include the total diameter of the baseline). In addition, the absolute value of the total diameter must increase by at least 5 mm (the appearance of one or more new lesions is also considered as progressive disease).

"SD" is defined as a state in which the reduction in target lesions is insufficient to achieve partial response and the increase is insufficient to achieve progressive disease.

"ORR" includes CR and PR, and is defined as the proportion of patients who achieve complete response or partial response.

"IC-ORR" includes CR or PR for confirmed intracranial lesions, and is defined as the proportion of the patients with complete response or partial response of intracranial lesions among the patients with intracranial lesions.

"DCR" is defined as the proportion of the patients who achieve response (PR+CR) and stable disease (SD) after a treatment.

"IC-DCR" is defined as the proportion of the patients who achieve intracranial response (PR+CR), intracranial stable disease (SD), and non-target lesion CR/non-target lesion PD after a treatment among the patients with intracranial lesions.

"DOR" is defined as the time from the first assessment of CR or PR to progressive disease or death, and is only applicable to the patients who achieve response.

"IC-DOR" is defined as the time from the first assessment of intracranial CR or intracranial PR to progressive disease or death, and is only applicable to the patients whose intracranial lesions achieve response.

"TTR" is defined as the time from patient enrollment (first dose of investigational drug) to tumor response (CR/PR), based on the patients with objective response.

"IC-TTR" is defined as the time from patient enrollment (first dose of investigational drug) to intracranial tumor response (IC-CR/PR), based on the patients with intracranial objective response.

"PFS" is defined as the time from the first administration of the investigational drug to progressive disease (PD) or death from any cause, whichever comes first.

"IC-PFS" is defined as the time from the first administration of the investigational drug to intracranial progressive tumor (PD) or death from any cause, whichever comes first.

"OS" is defined as the time from the first administration of the investigational drug to death from any cause.

### Compound A

The present disclosure relates to a compound A of the following formula: 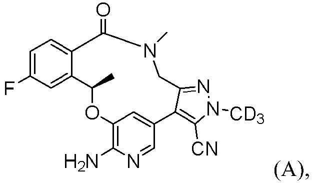 or a pharmaceutically acceptable salt thereof.

Compound A, chemically named (10R)-7-amino-12-fluoro-2-(methyl-d3)-10,16-dimethyl-15-oxo-10,15,16,17-tetrahydro-2H-8,4-(metheno)pyrazolo[4,3-h][2,5,11]benzoxadiazacyclotetradecine-3-carbonitrile, is a potent third-generation ALK/ROS1 dual tyrosine kinase inhibitor. It inhibits the overexpression of ALK and ROS1 in NSCLC cells by disrupting the ALK and ROS1-mediated signaling pathways, thereby achieving the effect of inhibiting the proliferation of NSCLC cells. It is sensitive to most of the secondary mutations in the ALK kinase domains, including the mutations in the G1202R solvant-front region, and can cross the blood-brain barrier. Compound A and the preparation thereof were first disclosed in WO2017/148325A1. Then, a preparation method thereof was disclosed in WO2020/108522A1. Subsequently, a salt form, a crystal form and a pharmaceutical composition thereof were disclosed in WO2021/233296A1. These three patents were incorporated herein by reference in their entireties for all purposes.

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describe pharmaceutically acceptable salts in detail in J. Pharmaceutical Sciences (1977) 66:1-19. Pharmaceutically acceptable salts of the compounds disclosed herein include those derived from suitable inorganic and organic acids and bases. Examples of pharmaceutically acceptable, nontoxic acid addition salts are salts formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid. Salts formed using conventional methods in the art such as ion exchange are also included. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and N⁺(C₁₋₄ alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

In some embodiments, the X-ray powder diffraction pattern of compound A obtained using CuKα radiation includes at least the characteristic peaks represented by the following °2θ: 16.175±0.2, 17.299±0.2, and 21.218±0.2. In a specific embodiment, the X-ray powder diffraction pattern of compound A further includes the characteristic peaks represented by the following °2θ: 9.637±0.2, 12.555±0.2, 14.343±0.2, and 19.366±0.2. In a specific embodiment, the X-ray powder diffraction pattern of compound A further includes the characteristic peaks represented by the following °2θ: 7.435±0.2, 10.11±0.2, 11.808±0.2, 14.922±0.2, 18.359±0.2, 19.859±0.2, 23.401±0.2, 23.939±0.2, 25.117±0.2, 25.727±0.2, 26.831±0.2, and 28.862±0.2.

### Pharmaceutical composition

A composition of compound A and a method of preparing the composition are described in WO2021/233296A1, which is incorporated herein by reference in its entirety for all purposes.

In one aspect, the present disclosure provides use and method of a pharmaceutical composition in treating ALK-positive non-small cell lung cancer, wherein the pharmaceutical composition comprises the compound A or a pharmaceutically acceptable salt thereof, and a pharmaceutically acceptable excipient.

In another aspect, the present disclosure provides use and method of a pharmaceutical composition in treating ALK-positive CNS metastatic non-small cell lung cancer, wherein the pharmaceutical composition comprises the compound A or a pharmaceutically acceptable salt thereof, and a pharmaceutically acceptable excipient.

In another aspect, the present disclosure provides use and method of a pharmaceutical composition in treating non-small cell lung cancer with EML4-ALK L1196M mutation, wherein the pharmaceutical composition comprises the compound A or a pharmaceutically acceptable salt thereof, and a pharmaceutically acceptable excipient.

In another aspect, the present disclosure provides use and method of a pharmaceutical composition in treating CNS metastatic non-small cell lung cancer with EML4-ALK L1196M mutation, wherein the pharmaceutical composition comprises the compound A or a pharmaceutically acceptable salt thereof, and a pharmaceutically acceptable excipient.

In another aspect, the present disclosure provides use and method of a pharmaceutical composition in treating non-small cell lung cancer with EML4-ALK G1202R mutation, wherein the pharmaceutical composition comprises the compound A or a pharmaceutically acceptable salt thereof, and a pharmaceutically acceptable excipient.

In another aspect, the present disclosure provides use and method of a pharmaceutical composition in treating CNS metastatic non-small cell lung cancer with EML4-ALK G1202R mutation, wherein the pharmaceutical composition comprises the compound A or a pharmaceutically acceptable salt thereof, and a pharmaceutically acceptable excipient.

In another aspect, the present disclosure provides use and method of a pharmaceutical composition in treating ROS1-positive non-small cell lung cancer, wherein the pharmaceutical composition comprises the compound A or a pharmaceutically acceptable salt thereof, and a pharmaceutically acceptable excipient.

In another aspect, the present disclosure provides use and method of a pharmaceutical composition in treating ROS1-positive CNS metastatic non-small cell lung cancer, wherein the pharmaceutical composition comprises the compound A or a pharmaceutically acceptable salt thereof, and a pharmaceutically acceptable excipient.

In another aspect, the present disclosure provides use and method of a pharmaceutical composition in treating non-small cell lung cancer with SLC34A2-ROS1 G2032R mutation, wherein the pharmaceutical composition comprises the compound A or a pharmaceutically acceptable salt thereof, and a pharmaceutically acceptable excipient.

In another aspect, the present disclosure provides use and method of a pharmaceutical composition in treating CNS metastatic non-small cell lung cancer with RSLC34A2-ROS1 G2032R mutation, wherein the pharmaceutical composition comprises the compound A or a pharmaceutically acceptable salt thereof, and a pharmaceutically acceptable excipient.

In some embodiments, the pharmaceutical composition disclosed herein comprises (i) a pharmaceutically active ingredient: a crystal form of the free base of compound A or a crystal form of a pharmaceutically acceptable salt thereof, (ii) a diluent, (iii) a disintegrant, (iv) a binder, and (v) a lubricant.

In a specific embodiment, the present disclosure provides the above pharmaceutical composition, wherein the crystal form accounts for 1 to 30%, alternatively 2 to 20%, alternatively 3 to 15%, yet alternatively, approximately 4%, 5%, 6%, 7%, 8%, 9% or 10%, yet alternatively, approximately 4%, 5%, 6%, 7%, 8%, 9%, 10%, 12%, 14%, 15% or 16%, yet alternatively, approximately 6.25% or 15.38% of the total weight of the pharmaceutical composition, calculated based on the weight of the free base of the compound; alternatively, wherein the content of the crystal form in a unit dose is 1 to 100 mg, alternatively 2 to 50 mg, alternatively 3 to 40 mg, alternatively 5 to 60 mg, alternatively 10 to 100 mg, alternatively approximately 5, 10, 15, 20, 25, 30, 35 or 40 mg, alternatively approximately 5, 10, 15, 20, 25, 30, 35, 40, 50, 55, 60, 65, 70 or 80 mg, alternatively approximately 5, 20, 25 or 60 mg.

In a specific embodiment, the present disclosure provides the above pharmaceutical composition, wherein the diluent accounts for 65 to 95%, alternatively 70 to 90%, alternatively approximately 80%, 81%, 82%, 83%, 84%, 85%, 86%, 87%, 88%, 89%, or 90% of the total weight of the pharmaceutical composition; alternatively, wherein the content of the diluent in a unit dose is 50 to 380 mg, alternatively 60 to 360 mg, alternatively 70 to 350 mg, for example, approximately 70 mg or 350 mg.

In a specific embodiment, the present disclosure provides the above pharmaceutical composition, wherein the diluent is selected from microcrystalline cellulose, anhydrous calcium hydrogen phosphate and mannitol, for example, microcrystalline cellulose 102, mannitol 100SD and mannitol 50C, and mixtures thereof; alternatively, when microcrystalline cellulose 102 and mannitol 50C are present simultaneously, the weight ratio of microcrystalline cellulose 102 to mannitol 50C is 5:1 to 1:5, alternatively 3:1 to 1:2, alternatively approximately 2:1.

In a specific embodiment, the present disclosure provides the above pharmaceutical composition, wherein the disintegrant accounts for 1 to 5%, alternatively 2 to 4%, alternatively approximately 2%, 2.5%, 3%, 3.5% or 4% of the total weight of the pharmaceutical composition; alternatively, wherein the content of the disintegrant in a unit dose is 1 to 20 mg, alternatively 2 to 16 mg, alternatively approximately 2, 2.5, 3, 6, 9 or 12 mg.

In a specific embodiment, the present disclosure provides the above pharmaceutical composition, wherein the disintegrant is croscarmellose sodium or crospovidone XL-10, alternatively croscarmellose sodium.

In a specific embodiment, the present disclosure provides the above pharmaceutical composition, wherein the binder accounts for 1 to 5%, alternatively 2 to 4%, alternatively approximately 2%, 2.5%, 3%, 3.5% or 4% of the total weight of the pharmaceutical composition; alternatively, wherein the content of the binder in a unit dose is 1 to 20 mg, alternatively 2 to 16 mg, alternatively approximately 2, 2.5, 3, 6, 9 or 12 mg.

In a specific embodiment, the present disclosure provides the above pharmaceutical composition, wherein the binder is hydroxypropyl cellulose EXF or povidone K30, alternatively hydroxypropyl cellulose EXF.

In a specific embodiment, the present disclosure provides the above pharmaceutical composition, wherein the lubricant accounts for 0.1 to 5%, alternatively 0.5 to 2%, alternatively approximately 1% of the total weight of the pharmaceutical composition; alternatively, wherein the content of the lubricant in a unit dose is 0.1 to 20 mg, alternatively 0.5 to 8 mg, alternatively approximately 0.5, 1, 2, 3, 4, 5, 6, 7 or 8 mg.

In a specific embodiment, the present disclosure provides the above pharmaceutical composition, wherein the lubricant is magnesium stearate or sodium stearyl fumarate PRUV, alternatively magnesium stearate.

In some embodiments, the present disclosure provides the above pharmaceutical composition, comprising the following components:
(i) compound A with a weight percentage of 1 to 30%,
(ii) microcrystalline cellulose 102 and mannitol 50C (in a weight ratio of approximately 2:1) with a weight percentage of 65 to 95%,
(iii) croscarmellose sodium with a weight percentage of 2 to 4%,
(iv) hydroxypropyl cellulose EXF with a weight percentage of 2 to 4%, and
(v) magnesium stearate with a weight percentage of 0.1 to 5%.

In some embodiments, the present disclosure provides the above pharmaceutical composition, wherein a unit dose comprises the following components:
(i) approximately 5 mg of compound A,
(ii) approximately 46.2 mg of microcrystalline cellulose 102 and approximately 23.2 mg of mannitol 50C,
(iii) approximately 2.4 mg of croscarmellose sodium,
(iv) approximately 2.4 mg of hydroxypropyl cellulose EXF, and
(v) approximately 0.8 mg of magnesium stearate.

In some embodiments, the present disclosure provides the above pharmaceutical composition, wherein a unit dose comprises the following components:
(i) approximately 25 mg of compound A,
(ii) approximately 231 mg of microcrystalline cellulose 102 and approximately 116 mg of mannitol 50 C,
(iii) approximately 12 mg of croscarmellose sodium,
(iv) approximately 12 mg of hydroxypropyl cellulose EXF, and
(v) approximately 4 mg of magnesium stearate.

In a specific embodiment, the X-ray powder diffraction pattern of compound A in the pharmaceutical composition obtained using CuKα radiation includes at least the characteristic peaks represented by the following °2θ: 16.175±0.2, 17.299±0.2, and 21.218±0.2. In another embodiment, the X-ray powder diffraction pattern of compound A further includes the characteristic peaks represented by the following °2θ: 9.637±0.2, 12.555±0.2, 14.343±0.2, and 19.366±0.2. In some embodiments, the X-ray powder diffraction pattern of compound A further includes the characteristic peaks represented by the following °2θ: 7.435±0.2, 10.11±0.2, 11.808±0.2, 14.922±0.2, 18.359±0.2, 19.859±0.2, 23.401±0.2, 23.939±0.2, 25.117±0.2, 25.727±0.2, 26.831±0.2, and 28.862±0.2.

In some embodiments, the present disclosure provides the above pharmaceutical composition as a tablet, alternatively a coated tablet; alternatively, the coating agent is Opadry II 85F620077.

The pharmaceutical compositions provided by the present disclosure can be administered by a variety of routes including, but not limited to, oral (enteral) administration, parenteral administration (via injection), rectal administration, transdermal administration, intradermal administration, intrathecal administration, subcutaneous (SC) administration, intravenous (IV) administration, intramuscular (IM) administration, and intranasal administration. In one embodiment, the pharmaceutical composition disclosed herein is administered orally.

The pharmaceutical compositions provided herein can also be administered chronically ("chronic administration"). Chronic administration refers to administration of a compound or pharmaceutical composition thereof over an extended period of time, for example, over 3 months, 6 months, 1 year, 2 years, 3 years, 5 years, etc, or may be continued indefinitely, for example, for the rest of the subject's life. In one embodiment, the chronic administration is intended to provide a constant level of the compound in the blood, e.g., within the therapeutic window over the extended period of time.

The pharmaceutical compositions provided herein can be presented in unit dosage forms to facilitate precise administration. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions.

In another embodiment, the pharmaceutical composition provided herein is administered to the subject in a solid dosage form. In one specific embodiment, the solid dosage form is a tablet.

In another embodiment, the compounds provided herein may be administered as a single active agent, or they may be administered in combination with other active agents.

### Use and treatment methods

### ALK-positive non-small cell lung cancer

In some embodiments, the present disclosure relates to use and method of the compound A or a pharmaceutically acceptable salt thereof in treating ALK-positive non-small cell lung cancer. In a specific embodiment, the non-small cell lung cancer is advanced non-small cell lung cancer. In one specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has an EML4-ALK L1196M mutation or an EML4-ALK G1202R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to use and method of the compound A or a pharmaceutically acceptable salt thereof in treating ALK-positive non-small cell lung cancer, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has an EML4-ALK L1196M mutation or an EML4-ALK G1202R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

### ALK-positive CNS metastatic non-small cell lung cancer

In one embodiment, the present disclosure relates to use and method of the compound A or a pharmaceutically acceptable salt thereof in treating ALK-positive CNS metastatic non-small cell lung cancer. In one specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has an EML4-ALK L1196M mutation or an EML4-ALK G1202R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to a method of treating ALK-positive CNS metastatic non-small cell lung cancer with the compound A or a pharmaceutically acceptable salt thereof, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has an EML4-ALK L1196M mutation or an EML4-ALK G1202R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, death, or discontinuation due to the individual choice.

In another embodiment, the present disclosure relates to a method of determining the concentration of the compound A in the CNS fluid of a subject with ALK-positive CNS metastatic non-small cell lung cancer, comprising:
a. collecting a cerebrospinal fluid sample; and
b. measuring the concentration of the compound A in the CSF sample to determine the concentration of the compound A present in the CNS fluid.

### Non-small cell lung cancer with EML4-ALK L1196M mutation

In one embodiment, the present disclosure relates to use and method of the compound A or a pharmaceutically acceptable salt thereof in treating non-small cell lung cancer with EML4-ALK L1196M mutation. In one specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In one embodiment, the present disclosure relates to use and method of the compound A or a pharmaceutically acceptable salt thereof in treating non-small cell lung cancer with EML4-ALK L1196M mutation, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment witha an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

### CNS metastatic non-small cell lung cancer with EML4-ALK L1196M mutation

In one embodiment, the present disclosure relates to use and method of the compound A or a pharmaceutically acceptable salt thereof in treating CNS metastatic non-small cell lung cancer with EML4-ALK L1196M mutation. In one specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to use and method of an effective amount of the compound A or a pharmaceutically acceptable salt thereof in treating CNS metastatic non-small cell lung cancer with EML4-ALK L1196M mutation, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to a method of determining the concentration of the compound A in the CNS fluid of a subject with CNS metastatic non-small cell lung cancer with EML4-ALK L1196M mutation, comprising:
a. collecting a cerebrospinal fluid sample; and
b. measuring the concentration of the compound A in the CSF sample to determine the concentration of the compound A present in the CNS fluid.

### Non-small cell lung cancer with EML4-ALK G1202R mutation

In one embodiment, the present disclosure relates to use and method of the compound A or a pharmaceutically acceptable salt thereof in treating non-small cell lung cancer with EML4-ALK G1202R mutation. In one specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to use and method of the compound A or a pharmaceutically acceptable salt thereof in treating non-small cell lung cancer with EML4-ALK G1202R mutation, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

### CNS metastatic non-small cell lung cancer with EML4-ALK G1202R mutation

In one embodiment, the present disclosure relates to use and method of the compound A or a pharmaceutically acceptable salt thereof in treating CNS metastatic non-small cell lung cancer with EML4-ALK G1202R mutation. In one specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to use and method of the compound A or a pharmaceutically acceptable salt thereof in treating CNS metastatic non-small cell lung cancer with EML4-ALK G1202R mutation, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to a method of determining the concentration of the compound A in the CNS fluid of a subject with CNS metastatic non-small cell lung cancer with EML4-ALK G1202R mutation, comprising:
a. collecting a cerebrospinal fluid sample; and
b. measuring the concentration of the compound A in the CSF sample to determine the concentration of the compound A present in the CNS fluid.

### TP53-positive ALK-positive non-small cell lung cancer

In some embodiments, the present disclosure relates to use and method of the compound A or a pharmaceutically acceptable salt thereof in treating TP53-positive ALK-positive non-small cell lung cancer. In a specific embodiment, the non-small cell lung cancer is advanced non-small cell lung cancer. In one specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has an EML4-ALK L1196M mutation or an EML4-ALK G1202R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to use and method of the compound A or a pharmaceutically acceptable salt thereof in treating TP53-positive ALK-positive non-small cell lung cancer, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib as the only ALK inhibitor. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with an ALK inhibitor. In another specific embodiment, the subject has not previously received a treatment with a third-generation ALK inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has an EML4-ALK L1196M mutation or an EML4-ALK G1202R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

### ROS1-positive non-small cell lung cancer

In one embodiment, the present disclosure relates to use and method of the compound A or a pharmaceutically acceptable salt thereof in treating ROS1-positive non-small cell lung cancer. In one specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with a ROS1 inhibitor. In another specific embodiment, the subject has not previously received a treatment with a second-generation ROS1 inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has a SLC34A2-ROS1 G2032R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to use and method of the compound A or a pharmaceutically acceptable salt thereof in treating ROS1-positive non-small cell lung cancer, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with a ROS1 inhibitor. In another specific embodiment, the subject has not previously received a treatment with a second-generation ROS1 inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has a SLC34A2-ROS1 G2032R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

### ROS1-positive CNS metastatic non-small cell lung cancer

In one embodiment, the present disclosure relates to use and method of the compound A or a pharmaceutically acceptable salt thereof in treating ROS1-positive CNS metastatic non-small cell lung cancer. In one specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with a ROS1 inhibitor. In another specific embodiment, the subject has not previously received a treatment with a second-generation ROS1 inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has a SLC34A2-ROS1 G2032R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to use and method of the compound A or a pharmaceutically acceptable salt thereof in treating ROS1-positive CNS metastatic non-small cell lung cancer, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with a ROS1 inhibitor. In another specific embodiment, the subject has not previously received a treatment with a second-generation ROS1 inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the non-small cell lung cancer has a SLC34A2-ROS1 G2032R mutation. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to a method of determining the concentration of the compound A in the CNS fluid of a subject with ROS1-positive CNS metastatic non-small cell lung cancer, comprising:
a. collecting a cerebrospinal fluid sample; and
b. measuring the concentration of the compound A in the CSF sample to determine the concentration of the compound A present in the CNS fluid.

### Non-small cell lung cancer with SLC34A2-ROS1 G2032R mutation

In one embodiment, the present disclosure relates to use and method of the compound A or a pharmaceutically acceptable salt thereof in treating non-small cell lung cancer with SLC34A2-ROS1 G2032R mutation. In one specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with a ROS1 inhibitor. In another specific embodiment, the subject has not previously received a treatment with a second-generation ROS1 inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to use and method of the compound A or a pharmaceutically acceptable salt thereof in treating non-small cell lung cancer with SLC34A2-ROS1 G2032R mutation, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the non-small cell lung cancer is metastatic non-small cell lung cancer. In another specific embodiment, the metastatic non-small cell lung cancer is CNS metastatic non-small cell lung cancer, bone metastatic non-small cell lung cancer, liver metastatic non-small cell lung cancer, adrenal gland metastatic non-small cell lung cancer, or other metastatic non-small cell lung cancers. In another embodiment, the non-small cell lung cancer is CNS metastatic non-small cell lung cancer. In another embodiment, the non-small cell lung cancer is metastatic advanced non-small cell lung cancer. In another embodiment, the non-small cell lung cancer is CNS metastatic advanced non-small cell lung cancer. In another specific embodiment, the subject has previously experienced progressive disease or intolerance after a continuous treatment with crizotinib. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with a ROS1 inhibitor. In another specific embodiment, the subject has not previously received a treatment with a second-generation ROS1 inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

### CNS metastatic non-small cell lung cancer with SLC34A2-ROS1 G2032R mutation

In one embodiment, the present disclosure relates to use and method of the compound A or a pharmaceutically acceptable salt thereof in treating CNS metastatic non-small cell lung cancer with SLC34A2-ROS1 G2032R mutation. In one specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with a ROS1 inhibitor. In another specific embodiment, the subject has not previously received a treatment with a second-generation ROS1 inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, death, or discontinuation due to the individual choice.

In one embodiment, the present disclosure relates to use and method of the compound A or a pharmaceutically acceptable salt thereof in treating CNS metastatic non-small cell lung cancer with SLC34A2-ROS1 G2032R mutation, wherein the non-small cell lung cancer has reached progressive disease after another therapy. In one specific embodiment, the another therapy is radiotherapy, pharmacotherapy, or surgical therapy. In another specific embodiment, the pharmacotherapy is chemotherapy, targeted therapy, or immunotherapy. In another specific embodiment, the pharmacotherapy is chemotherapy. In another specific embodiment, the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody. In another specific embodiment, the non-small cell lung cancer is advanced non-small cell lung cancer. In another specific embodiment, the non-small cell lung cancer is adenocarcinoma. In another specific embodiment, the subject has previously experienced progressive disease or intolerance after a continuous treatment with crizotinib. In another specific embodiment, the subject has previously experienced progressive disease or intolerance after a continuous treatment with at least a second-generation ALK inhibitor. In another specific embodiment, the subject has previously experienced progressive disease or intolerance after a continuous treatment with a second-generation ALK inhibitor. In another specific embodiment, the second-generation ALK inhibitor is selected from ceritinib, alectinib, brigatinib, ensartinib, iruplinalkib and entrectinib. In another specific embodiment, the second-generation ALK inhibitor is selected from alectinib, ceritinib, ensartinib and brigatinib. In another specific embodiment, the subject has not previously received a treatment with a ROS1 inhibitor. In another specific embodiment, the subject has not previously received a treatment with a second-generation ROS1 inhibitor. In another specific embodiment, the subject has previously experienced progressive disease or intolerance after a first-line or a higher-line therapy. In another specific embodiment, the subject has previously experienced progressive disease or intolerance after a first-line, a second-line, a third-line, a fourth-line, or a fifth-line therapy. In another specific embodiment, the subject has previously experienced progressive disease or intolerance after a first-line or a second-line therapy. In another specific embodiment, the dosage of compound A is from approximately 5 mg/day to approximately 180 mg/day. In another specific embodiment, the dosage of compound A is from approximately 5 mg/day to approximately 125 mg/day. In another specific embodiment, the dosage of compound A is selected from approximately 5 mg/day, approximately 10 mg/day, approximately 20 mg/day, approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day, approximately 80 mg/day, approximately 100 mg/day and approximately 125 mg/day. In another specific embodiment, the dosage of compound A is selected from approximately 40 mg/day, approximately 60 mg/day and approximately 80 mg/day. In another specific embodiment, the dosage of compound A is approximately 60 mg/day. In another specific embodiment, the compound A is administered orally. In another specific embodiment, the compound A is administered orally on an empty stomach. In another specific embodiment, the compound A is administered orally once daily, twice daily, or three times daily. In another specific embodiment, the compound A is administered orally once daily. In another specific embodiment, the compound A is administered until progressive disease, intolerable toxicity, or death.

In another embodiment, the present disclosure relates to a method of determining the concentration of the compound A in the CNS fluid of a subject with CNS metastatic non-small cell lung cancer with SLC34A2-ROS1 G2032R mutation, comprising:
a. collecting a cerebrospinal fluid sample; and
b. measuring the concentration of the compound A in the CSF sample to determine the concentration of the compound A present in the CNS fluid.

More specifically, the present disclosure relates to the following technical solutions:
1. Use of a compound A or a pharmaceutically acceptable salt thereof in the manufacture of a medicament for the treatment of non-small cell lung cancer, wherein the compound A has the following structure: 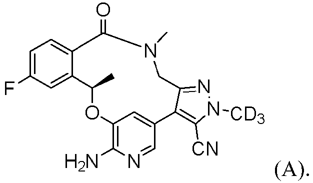
2. A method of treating non-small cell lung cancer in a patient, comprising administering to the patient a therapeutically effective amount of a compound A or a pharmaceutically acceptable salt thereof, wherein the compound A has the following structure: 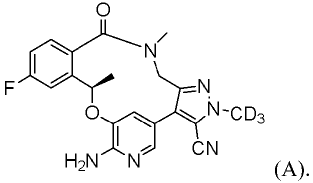
3. A compound A or a pharmaceutically acceptable salt thereof, for use in the treatment of non-small cell lung cancer, wherein the compound A has the following structure: 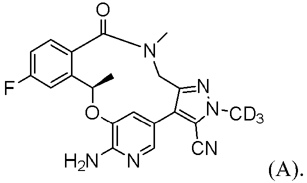
4. The use according to technical solution 1, or the method according to technical solution 2, or the compound for use according to technical solution 3, wherein the non-small cell lung cancer is ALK-positive non-small cell lung cancer; alternatively, wherein the non-small cell lung cancer is advanced ALK-positive non-small cell lung cancer; alternatively, wherein the ALK is ALK L1196M or ALK G1202R mutation; alternatively, wherein the non-small cell lung cancer is TP53-positive (including mutant and wild-type) ALK-positive non-small cell lung cancer; alternatively, the non-small cell lung cancer is TP53-mutated ALK-positive non-small cell lung cancer.
5. The use, method, or compound for use according to any one of technical solutions 1 to 4, wherein the non-small cell lung cancer is adenocarcinoma.
6. The use, method, or compound for use according to any one of technical solutions 1 to 5, wherein the non-small cell lung cancer is metastatic non-small cell lung cancer; alternatively, wherein the non-small cell lung cancer is CNS metastatic non-small cell lung cancer (e.g., intracranial metastatic non-small cell lung cancer).
7. The use, method, or compound for use according to any one of technical solutions 1 to 6, wherein the non-small cell lung cancer is untreated.
8. The use, method, or compound for use according to any one of technical solutions 1 to 6, wherein the non-small cell lung cancer has reached progressive disease after a treatment with another therapy;
   alternatively, wherein the another therapy is selected from radiotherapy, pharmacotherapy and surgical therapy; alternatively, wherein the pharmacotherapy is chemotherapy, targeted therapy, or immunotherapy;
   alternatively, wherein the pharmacotherapy is chemotherapy;
   alternatively, wherein the pharmacotherapy is targeted therapy;
   alternatively, wherein the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody.
9. The use, method, or compound for use according to technical solution 8, wherein the non-small cell lung cancer has reached progressive disease after a treatment with an ALK inhibitor.
10. The use, method, or compound for use according to technical solution 9, wherein the ALK inhibitor is a first-generation or a second-generation ALK inhibitor; alternatively, the ALK inhibitor is selected from crizotinib, alectinib, ceritinib, ensartinib and brigatinib.
11. The use, method, or compound for use according to any one of technical solutions 1 to 10, wherein the non-small cell lung cancer is in clinical stage III or IV.
12. The use, method, or compound for use according to any one of technical solutions 1 to 11, wherein the patient has received a first-line or a higher-line therapy; alternatively, wherein the patient has received a second-line or a higher-line therapy; alternatively, the patient has received a first-line, a second-line, a third-line, a fourth-line, or a fifth-line therapy.
13. The use, method or compound for use according to technical solution 12, wherein the patient has experienced progressive disease or intolerance after receiving a first-line or a higher-line therapy; alternatively, wherein the patient has experienced progressive disease or intolerance after receiving a first-line or a second-line therapy.
14. The use, method, or compound for use according to technical solution 12 or 13, wherein the first-line or the higher-line therapy is selected from radiotherapy, pharmacotherapy and surgical therapy;
   alternatively, wherein the pharmacotherapy is chemotherapy, targeted therapy, or immunotherapy;
   alternatively, wherein the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody.
15. The use, method, or compound for use according to technical solution 14, wherein the patient has previously received chemotherapy; alternatively, wherein the chemotherapy drug is selected from carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, and pyridoxine, alternatively carboplatin, cisplatin, nedaplatin, lobaplatin, pemetrexed, docetaxel, endostatin, sodium cantharidate, or pyridoxine.
16. The use, method, or compound for use according to technical solution 14, wherein the patient has previously received targeted therapy; alternatively, the patient has previously received a treatment with an ALK inhibitor; alternatively, the patient has previously received a treatment with a second-generation ALK inhibitor, such as alectinib, ceritinib, ensartinib, or brigatinib; alternatively, the patient has previously received a treatment with crizotinib as the only ALK inhibitor.
17. The use, method, or compound for use according to technical solution 14, wherein the patient has not previously received a treatment with an ALK inhibitor (e.g., a third-generation ALK inhibitor).
18. The use, method, or compound for use according to any one of technical solutions 1 to 17, wherein the dosage of the compound A is approximately 5 mg/day to approximately 180 mg/day;
   alternatively, the dosage of the compound A is approximately 5 mg/day to approximately 125 mg/day;
   alternatively, the dosage of the compound A is selected from approximately 5 mg/day, approximately 10 mg/day, approximately 20 mg/day, approximately 40 mg/day, approximately 60 mg/day, approximately 80 mg/day, approximately 100 mg/day, and approximately 125 mg/day;
   alternatively, the dosage of the compound A is selected from approximately 40 mg/day, approximately 60 mg/day, approximately 80 mg/day, approximately 100 mg/day, and approximately 125 mg/day;
   alternatively, the dosage of the compound A is selected from approximately 40 mg/day, approximately 60 mg/day, and approximately 80 mg/day;
   alternatively, the dosage of the compound A is approximately 60 mg/day.
19. The use, method, or compound for use according to any one of technical solutions 1 to 18, wherein the compound A is administered orally.
20. The use, method, or compound for use according to any one of technical solutions 1 to 19, wherein the compound A is administered orally once daily, twice daily, or three times daily;
   alternatively, the compound A is administered orally once daily.
21. The use, method, or compound for use according to any one of technical solutions 1 to 20, wherein the compound A is administered until progressive disease, intolerable toxicity, or death.
22. The use, method, or compound for use according to any one of technical solutions 1 to 21, wherein the patient has a CSF/plasma concentration ratio greater than approximately 40% of the compound A after the administration of the compound A; alternatively, has a CSF/plasma concentration ratio greater than approximately 50% of the compound A; alternatively, has a CSF/plasma concentration ratio greater than approximately 60% of the compound A.
23. The use according to technical solution 1, or the method according to technical solution 2, or the compound for use according to technical solution 3, wherein the non-small cell lung cancer is ROS1-positive non-small cell lung cancer; alternatively, wherein the non-small cell lung cancer is advanced ROS1-positive non-small cell lung cancer; alternatively, wherein the ROS1 is ROS1 G2032R mutation.
24. The use, method, or compound for use according to any one of technical solutions 1 to 3 and 23, wherein the non-small cell lung cancer is adenocarcinoma.
25. The use, method, or compound for use according to any one of technical solutions 1 to 3, 23 and 24, wherein the non-small cell lung cancer is metastatic non-small cell lung cancer; alternatively, wherein the non-small cell lung cancer is CNS metastatic non-small cell lung cancer (e.g., intracranial metastatic non-small cell lung cancer).
26. The use, method, or compound for use according to any one of technical solutions 1 to 3 and 23 to 25, wherein the non-small cell lung cancer is untreated.
27. The use, method, or compound for use according to any one of technical solutions 1 to 3 and 23 to 26, wherein the non-small cell lung cancer has reached progressive disease after a treatment with another therapy;
   alternatively, wherein the another therapy is selected from radiotherapy, pharmacotherapy and surgical therapy; alternatively, wherein the pharmacotherapy is chemotherapy, targeted therapy, or immunotherapy;
   alternatively, wherein the pharmacotherapy is chemotherapy;
   alternatively, wherein the pharmacotherapy is targeted therapy;
   alternatively, wherein the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody.
28. The use, method, or compound for use according to technical solution 27, wherein the non-small cell lung cancer has reached progressive disease after a treatment with a ROS1 inhibitor.
29. The use, method, or compound for use according to technical solution 28, wherein the ROS1 inhibitor is a first-generation ROS1 inhibitor; alternatively, the ROS1 inhibitor is selected from crizotinib, alectinib, ceritinib, ensartinib and brigatinib.
30. The use, method, or compound for use according to any one of technical solutions 1 to 3 and 23 to 29, wherein the non-small cell lung cancer is in clinical stage III or IV, alternatively clinical stage IV.
31. The use, method, or compound for use according to any one of technical solutions 1 to 3 and 23 to 30, wherein the patient has received a first-line or a higher-line therapy; alternatively, wherein the patient has received a second-line therapy.
32. The use, method or compound for use according to technical solution 31, wherein the patient has experienced progressive disease or intolerance after receiving a first-line or a higher-line therapy; alternatively, wherein the patient has experienced progressive disease or intolerance after receiving a second-line therapy.
33. The use, method, or compound for use according to technical solution 31 or 32, wherein the first-line or the higher-line therapy is selected from radiotherapy, pharmacotherapy and surgical therapy;
   alternatively, wherein the pharmacotherapy is chemotherapy, targeted therapy, or immunotherapy;
   alternatively, wherein the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody.
34. The use, method, or compound for use according to technical solution 33, wherein the patient has previously received chemotherapy; alternatively, wherein the chemotherapy drug is selected from carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, and pyridoxine, alternatively carboplatin, cisplatin, nedaplatin, lobaplatin, pemetrexed, docetaxel, endostatin, sodium cantharidate, or pyridoxine.
35. The use, method, or compound for use according to technical solution 33, wherein the patient has previously received targeted therapy; alternatively, the patient has previously received a treatment with a ROS1 inhibitor, such as alectinib, ceritinib, ensartinib, or brigatinib; alternatively, the patient has previously received a treatment with crizotinib as the only ROS1 inhibitor.
36. The use, method, or compound for use according to technical solution 33, wherein the patient has not previously received a treatment with a ROS1 inhibitor (e.g., a second-generation ROS1 inhibitor).
37. The use, method, or compound for use according to any one of technical solutions 1 to 3 and 23 to 36, wherein the dosage of the compound A is approximately 5 mg/day to approximately 180 mg/day;
   alternatively, the dosage of the compound A is approximately 5 mg/day to approximately 125 mg/day;
   alternatively, the dosage of the compound A is selected from approximately 5 mg/day, approximately 10 mg/day, approximately 20 mg/day, approximately 40 mg/day, approximately 60 mg/day, approximately 80 mg/day, approximately 100 mg/day, and approximately 125 mg/day;
   alternatively, the dosage of the compound A is selected from approximately 40 mg/day, approximately 60 mg/day, approximately 80 mg/day, approximately 100 mg/day, and approximately 125 mg/day;
   alternatively, the dosage of the compound A is selected from approximately 40 mg/day, approximately 60 mg/day, and approximately 80 mg/day;
   alternatively, the dosage of the compound A is approximately 60 mg/day.
38. The use, method, or compound for use according to any one of technical solutions 1 to 3 and 23 to 37, wherein the compound A is administered orally.
39. The use, method, or compound for use according to any one of technical solutions 1 to 3 and 23 to 38, wherein the compound A is administered orally once daily, twice daily, or three times daily;
   alternatively, the compound A is administered orally once daily.
40. The use, method, or compound for use according to any one of technical solutions 1 to 3 and 23 to 39, wherein the compound A is administered until progressive disease, intolerable toxicity, or death.
41. The use, method, or compound for use according to any one of technical solutions 1 to 3 and 23 to 40, wherein the patient has a CSF/plasma concentration ratio greater than approximately 40% of the compound A after the administration of the compound A; alternatively, has a CSF/plasma concentration ratio greater than approximately 50% of the compound A; alternatively, has a CSF/plasma concentration ratio greater than approximately 60% of the compound A.
42. A method of determining the concentration of the compound A in the CNS fluid of a subject with ALK-positive or ROS1-positive CNS metastatic non-small cell lung cancer, comprising:
   a. collecting a cerebrospinal fluid sample; and
   b. measuring the concentration of the compound A in the CSF sample to determine the concentration of the compound A present in the CNS fluid.

### Examples

The present disclosure will be further elaborated with specific examples below. It should be understood that these examples are only used to illustrate the present disclosure and are not used to limit the scope of the present disclosure. The experimental methods without specific conditions in the following examples are usually in accordance with the conventional conditions or the conditions suggested by the manufacturer. Unless otherwise specified, parts and percentages are parts by weight and percentages by weight.

Example 1: The anti-tumor effect in the subcutaneous transplanted animal model of Ba/F3 (EML4-ALK L1196M) cell line in female NOD-SCID mice.

Laboratory animals: NOD-SCID mice, female, 7 to 8 weeks old (the age of mice at tumor cell inoculation), weighing 16.0 to 22.0 g, and 50 mice (with additional 32 surplus mice). The mice were purchased from Beijing Ankai Yibo Biotechnology Co., Ltd., production license No.: SCXK (Beijing) 2017-0006, animal qualification certificate No.: 1103301911000908. Housing environment: SPF grade.

Environmental conditions of the housing room of the laboratory animals: All the laboratory animals were housed in individually ventilated cages with constant temperature and constant humidity. The temperature of the housing room was 20 to 26 °C, the humidity was 40 to 70%, the ventilation was 10 to 20 times/hour, and the day/night light/dark cycle was 12h/12h. The animals were continuously provided with cobalt-60 irradiated complete pelleted mouse feed with unlimited free access. The animals drank tap water (used after being autoclaved), which was provided continuously from water bottles, allowing for free access. The cages for raising the mice were polysulfone mice cages, which were autoclaved before use, and measured 325 mm × 210 mm × 180 mm. The bedding was autoclaved corn cobs. Each cage contained 5 animals. The cage cards were marked with the IACUC approval number, experiment number, start time of the experiment, project leader, experimenters, source of the animals, group, and animal number. The laboratory animals were marked with ear tags or ear labels.

Cell culture: Ba/F3 (EML4-ALK L1196M) cells (cell number: TC-00136) were cultured in RPMI 1640 culture medium containing 10% fetal bovine serum and 2 µg/mL Blasticidin S. The Ba/F3 (EML4-ALK L1196M) cells in the exponential growth phase were collected, resuspended in PBS to a suitable concentration, and then subcutaneously inoculated into nude mice for tumor formation.

Assay method: Female NOD/SCID mice were subcutaneously inoculated with 5×10⁶ Ba/F3 (EML4-ALK L1196M) cells on the right side to establish a subcutaneous tumor transplantation model. The mice were divided into 5 groups in the assay, including a high-dose (10 mg/kg) of compound A group, a medium-dose (3 mg/kg) of compound A group, a low-dose (1 mg/kg) of compound A group, a crizotinib positive control group (100 mg/kg), and a solvent control group, with 10 mice in each group. The mice were administered orally via gavage once daily. The efficacy was evaluated based on the relative tumor growth inhibition rate (TGI), and the safety was evaluated based on the changes in the body weight and mortality of the animals.

All the assay protocols for the animal experiment were reviewed and approved by the Institutional Animal Care and Use Committee (IACUC) of Crown Bioscience Inc. (CrownBio). During the assay, all the procedures of the animal experiment were complied with the requirements of the Association for Assessment and Accreditation of Laboratory Animal Care (AAALAC). All the procedures, including drug administration, tumor measurement, weight measurement, etc., were performed in a biosafety cabinet or on a clean bench.

The relative tumor growth rate (T/C%) was the percentage value of the relative tumor volume between a treatment group and the control group at a certain time point, or the percentage value of the tumor weight between a treatment group and the control group. The calculation formula was: T/C% = T_{RTV} / C_{RTV} × 100% (T_{RTV}: the average RTV of the treatment group; C_{RTV}: the average RTV of the solvent control group; RTV = Vt / V₀, wherein V₀ is the tumor volume of the animal at the time of grouping, and Vₜ is the tumor volume of the animal after treatment); or T/C% = T_{TW} / C_{TW} × 100% (T_{TW}: the average tumor weight at the end of the assay in the treatment group; C_{TW}: the average tumor weight at the end of the assay in the solvent control group).

The relative tumor growth inhibition rate (TGI%) was calculated using the formula: TGI% = 1 - T/C.

Statistical analysis: To compare the tumor volume on a given day across the different treatment groups, we first used the Bartlett's test to verify the assumption of homogeneity of variance among all the groups. When the p-value of the Bartlett's test was not less than 0.05, one-way ANOVA was used to test whether the means of all the groups were equal. If the p-value of the one-way ANOVA was less than 0.05, we would use the Tukey's HSD test for the pairwise comparisons between all the groups, or use the Dunnett's test for a pairwise comparison between each treatment group and the control group. When the p-value of the Bartlett's test was less than 0.05, the Kruskal-Wallis test was used to test whether the medians of all the groups were equal. If the p-value of the Kruskal-Wallis test was less than 0.05, we would use the Conover test for the pairwise comparisons between all the groups or a pairwise comparison between each treatment group and the control group, and adjust the p-value accordingly based on the number of groups in the multiple testing. All the statistical analyses and graphical plotting were performed in the R language environment (version 3.3.1). Unless otherwise specified, all the tests were two-tailed tests, and a p-value less than 0.05 was considered statistically significant.

### Assay result:

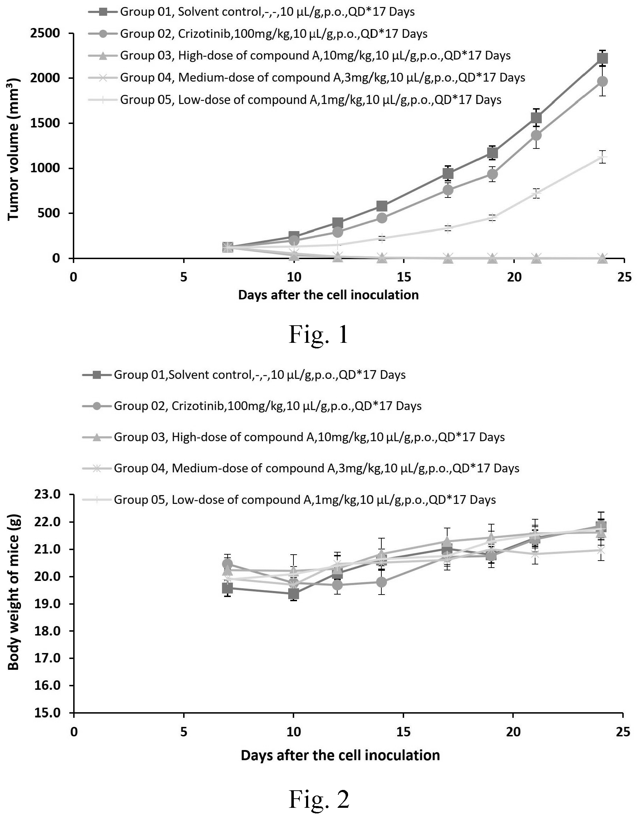

On the 17^{th} day after administration (i.e., at the end of the assay: the administration started on day 7 and ended on day 24), the average tumor volume of the mice in the solvent control group was 2222.44 mm³. In contrast, the assay drug compound A was administered under the regimens of p.o. once daily at 10 mg/kg, 3 mg/kg, and 1 mg/kg, and the average tumor volumes were 0 mm³, 0 mm³, and 1125.94 mm³, respectively, with the relative tumor growth inhibition rates (TGI) of 100%, 100%, and 49.53%, respectively. All of the results showed a statistically significant difference compared with the solvent control group (P<0.001). At the end of the assay, in the treatment groups of compound A at 10 mg/kg and 3 mg/kg, the tumor volumes of all (10/10, 10/10) the tumor-bearing mice reduced to 0 and the tumors completely disappeared. The positive control drug crizotinib was administered under an administration regimen of p.o. once daily at up to 100 mg/kg, and the average tumor volume was 1965.17 mm³, with a relative tumor growth inhibition rate (TGI) of 12.00%. The results showed no statistically significant difference compared with the solvent control group (P>0.05). The therapeutic effect was worse than that of the 1 mg/kg dose group of compound A. The tumor growth in each treatment group and the control group is shown in Fig. 1.

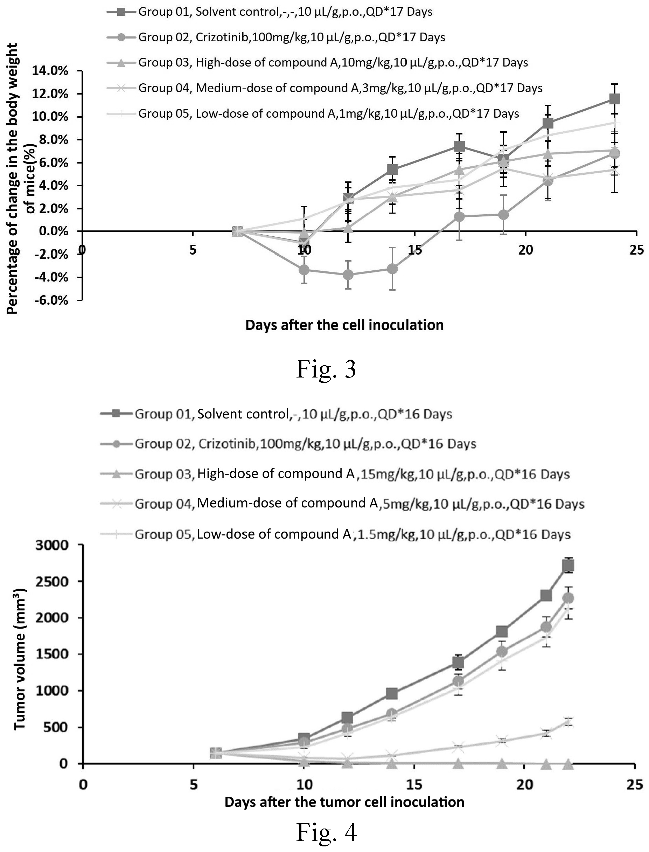

During the assay, no animals died in any of the treatment groups, and no obvious drug toxicity was observed. The animals were well tolerated during the treatment period. The body weight changes after the administration in the treatment and control groups are shown in Fig. 2 and Fig. 3.

Example 2: The anti-tumor effect in the subcutaneous transplanted animal model of Ba/F3 (EML4-ALK G1202R) cell line in female NOD-SCID mice.

Laboratory animals: NOD-SCID mice, female, 7 to 8 weeks old (the age of mice at tumor cell inoculation), weighing 16.0 to 20.3 g, and 50 mice (with additional 30 surplus mice). The mice were purchased from Beijing Ankai Yibo Biotechnology Co., Ltd., production license No.: SCXK (Beijing) 2017-0006, animal qualification certificate No.: 1103301911000914. Housing environment: SPF grade.

Environmental conditions of the housing room of the laboratory animals: All the laboratory animals were housed in individually ventilated cages with constant temperature and constant humidity. The temperature of the housing room was 20 to 26 °C, the humidity was 40 to 70%, the ventilation was 10 to 20 times/hour, and the day/night light/dark cycle was 12h/12h. The animals were continuously provided with cobalt-60 irradiated complete pelleted mouse feed with unlimited free access. The animals drank tap water (used after being autoclaved), which was provided continuously from water bottles, allowing for free access. The cages for raising the mice were polysulfone mice cages, which were autoclaved before use, and measured 325 mm × 210 mm × 180 mm. The bedding was autoclaved corn cobs. Each cage contained 5 animals. The cage cards were marked with the IACUC approval number, experiment number, start time of the experiment, project leader, experimenters, source of the animals, group, and animal number. The laboratory animals were marked with ear tags or ear labels.

Cell culture: Ba/F3 (EML4-ALK G1202R) cells (cell number: ECL-00031) were cultured in RPMI 1640 culture medium containing 10% fetal bovine serum. The Ba/F3 (EML4-ALK G1202R) cells in the exponential growth phase were collected, resuspended in PBS to a suitable concentration, and then subcutaneously inoculated for tumor formation.

Assay method: Female NOD/SCID mice were subcutaneously inoculated with 2×10⁶ Ba/F3 (EML4-ALK G1202R) cells on the right side to establish a subcutaneous tumor transplantation model. The mice were divided into 5 groups in the assay, including a high-dose (15 mg/kg) of compound A group, a medium-dose (5 mg/kg) of compound A group, a low-dose (1.5 mg/kg) of compound A group, a crizotinib positive control group (100 mg/kg), and a solvent control group, with 10 mice in each group. The mice were administered orally via gavage once daily. The efficacy was evaluated based on the relative tumor growth inhibition rate (TGI), and the safety was evaluated based on the changes in the body weight and mortality of the animals.

All the assay protocols for the animal experiment were reviewed and approved by the IACUC of CrownBio. During the assay, all the procedures of the animal experiment were complied with the requirements of the AAALAC. All the procedures, including drug administration, tumor measurement, weight measurement, etc., were performed in a biosafety cabinet or on a clean bench.

The relative tumor growth rate (T/C%) was the percentage value of the relative tumor volume between a treatment group and the control group at a certain time point, or the percentage value of the tumor weight between a treatment group and the control group. The calculation formula was: T/C% = T_{RTV} / C_{RTV} × 100% (T_{RTV}: the average RTV of the treatment group; C_{RTV}: the average RTV of the solvent control group; RTV = Vt / V₀, wherein V₀ is the tumor volume of the animal at the time of grouping, and Vₜ is the tumor volume of the animal after treatment); or T/C% = T_{TW} / C_{TW} × 100% (T_{TW}: the average tumor weight at the end of the assay in the treatment group; C_{TW}: the average tumor weight at the end of the assay in the solvent control group).

The relative tumor growth inhibition rate (TGI%) was calculated using the formula: TGI% = 1 - T/C.

Statistical analysis: To compare the tumor volume on a given day across the different treatment groups, we first used the Bartlett's test to verify the assumption of homogeneity of variance among all the groups. When the p-value of the Bartlett's test was not less than 0.05, one-way ANOVA was used to test whether the means of all the groups were equal. If the p-value of the one-way ANOVA was less than 0.05, we would use the Tukey's HSD test for the pairwise comparisons between all the groups, or use the Dunnett's test for a pairwise comparison between each treatment group and the control group. When the p-value of the Bartlett's test was less than 0.05, the Kruskal-Wallis test was used to test whether the medians of all the groups were equal. If the p-value of the Kruskal-Wallis test was less than 0.05, we would use the Conover test for the pairwise comparisons between all the groups or a pairwise comparison between each treatment group and the control group, and adjust the p-value accordingly based on the number of groups in the multiple testing. All the statistical analyses and graphical plotting were performed in the R language environment (version 3.3.1). Unless otherwise specified, all the tests were two-tailed tests, and a p-value less than 0.05 was considered statistically significant.

### Assay result:

On the 16^{th} day after administration (i.e., at the end of the assay: the administration started on day 6 and ended on day 22), the average tumor volume of the mice in the solvent control group was 2723.66 mm³. In contrast, the assay drug compound A was administered under the administration regimens of p.o. once daily at 15 mg/kg, 5 mg/kg, and 1.5 mg/kg, and the average tumor volumes were 0.25 mm³, 576.11 mm³, and 2139.18 mm³, respectively, with the relative tumor growth inhibition rates (TGI) of 99.99%, 79.17%, and 21.50%, respectively. All of the results showed a statistically significant difference compared with the solvent control group (P<0.001 for both the 15 mg/kg and 5 mg/kg treatment groups, and P<0.05 for the 1.5 mg/kg treatment group). At the end of the assay, in the treatment group of compound A at 15 mg/kg, 9/10 of the tumor-bearing mice had their tumor volumes reduced to 0, and the tumors completely disappeared. The positive control drug crizotinib was administered under the administration regimen of p.o. once daily at up to 100 mg/kg, and the average tumor volume was 2275.28 mm³, with a relative tumor growth inhibition rate (TGI) of 17.32%. The results showed a statistically significant difference compared with the solvent control group (P<0.05). The therapeutic effect was worse than that of the 1.5 mg/kg dose group of compound A. The tumor growth in each treatment group and the control group is shown in Fig. 4.

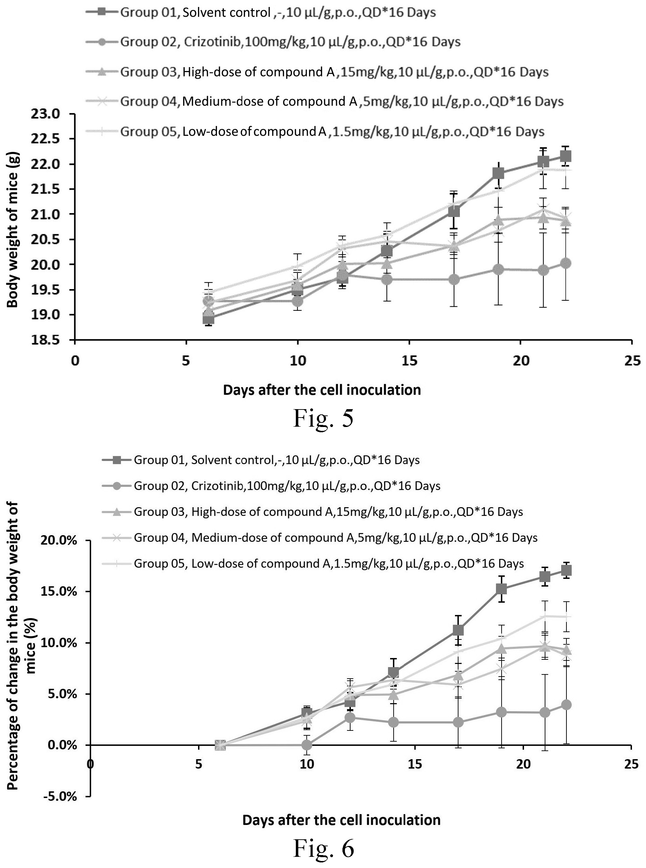

During the assay, no animals died in any of the treatment groups, and no obvious drug toxicity was observed. The animals were well tolerated during the treatment period. The body weight changes after the administration in the treatment and control groups are shown in Fig. 5 and Fig. 6.

Example 3: The anti-tumor effect in the subcutaneous transplanted animal model of Ba/F3 (SLC34A2-ROS1 G2032R) cell line in female NOD-SCID mice.

Laboratory animals: NOD-SCID mice, female, 7 to 8 weeks old (the age of mice at tumor cell inoculation), weighing 13.4 to 20.2 g, and 50 mice (with additional 30 surplus mice). The mice were purchased from Beijing Ankai Yibo Biotechnology Co., Ltd., production license No.: SCXK (Beijing) 2017-0006, animal qualification certificate No.: 1103301911000909. Housing environment: SPF grade.

Environmental conditions of the housing room of the laboratory animals: All the laboratory animals were housed in individually ventilated cages with constant temperature and constant humidity. The temperature of the housing room was 20 to 26 °C, the humidity was 40 to 70%, the ventilation was 10 to 20 times/hour, and the day/night light/dark cycle was 12h/12h. The animals were continuously provided with cobalt-60 irradiated complete pelleted mouse feed with unlimited free access. The animals drank tap water (used after being autoclaved), which was provided continuously from water bottles, allowing for free access. The cages for raising the mice were polysulfone mice cages, which were autoclaved before use, and measured 325 mm × 210 mm × 180 mm. The bedding was autoclaved corn cobs. Each cage contained 5 animals. The cage cards were marked with the IACUC approval number, experiment number, start time of the experiment, project leader, experimenters, source of the animals, group, and animal number. The laboratory animals were marked with ear tags or ear labels.

Cell culture: Ba/F3 (SLC34A2-ROS1 G2032R) cells (cell number: ECL-00019) were cultured in RPMI 1640 culture medium containing 10% fetal bovine serum. The Ba/F3 (SLC34A2-ROS1 G2032R) cells in the exponential growth phase were collected, resuspended in PBS to a suitable concentration, and then subcutaneously inoculated into nude mice for tumor formation.

Assay method: Female NOD/SCID mice were subcutaneously inoculated with 2×10⁶ Ba/F3 (SLC34A2-ROS1 G2032R) cells on the right side to establish a subcutaneous tumor transplantation model. The mice were divided into 5 groups in the assay, including a high-dose (15 mg/kg) of compound A group, a medium-dose (5 mg/kg) of compound A group, a low-dose (1.5 mg/kg) of compound A group, a crizotinib positive control group (100 mg/kg), and a solvent control group, with 10 mice in each group. The mice were administered orally via gavage once daily. The efficacy was evaluated based on the relative tumor growth inhibition rate (TGI), and the safety was evaluated based on the changes in the body weight and mortality of the animals.

All the assay protocols for the animal experiment were reviewed and approved by the IACUC of CrownBio. During the assay, all the procedures of the animal experiment were complied with the requirements of the AAALAC. All the procedures, including drug administration, tumor measurement, weight measurement, etc., were performed in a biosafety cabinet or on a clean bench.

The relative tumor growth rate (T/C%) was the percentage value of the relative tumor volume between a treatment group and the control group at a certain time point, or the percentage value of the tumor weight between a treatment group and the control group. The calculation formula was: T/C% = T_{RTV} / C_{RTV} × 100% (T_{RTV}: the average RTV of the treatment group; C_{RTV}: the average RTV of the solvent control group; RTV = Vt / V₀, wherein V₀ is the tumor volume of the animal at the time of grouping, and Vₜ is the tumor volume of the animal after treatment); or T/C% = T_{TW} / C_{TW} × 100% (T_{TW}: the average tumor weight at the end of the assay in the treatment group; C_{TW}: the average tumor weight at the end of the assay in the solvent control group).

The relative tumor growth inhibition rate (TGI%) was calculated using the formula: TGI% = 1 - T/C.

Statistical analysis: To compare the tumor volume on a given day across the different treatment groups, we first used the Bartlett's test to verify the assumption of homogeneity of variance among all the groups. When the p-value of the Bartlett's test was not less than 0.05, one-way ANOVA was used to test whether the means of all the groups were equal. If the p-value of the one-way ANOVA was less than 0.05, we would use the Tukey's HSD test for the pairwise comparisons between all the groups, or use the Dunnett's test for a pairwise comparison between each treatment group and the control group. When the p-value of the Bartlett's test was less than 0.05, the Kruskal-Wallis test was used to test whether the medians of all the groups were equal. If the p-value of the Kruskal-Wallis test was less than 0.05, we would use the Conover test for the pairwise comparisons between all the groups or a pairwise comparison between each treatment group and the control group, and adjust the p-value accordingly based on the number of groups in the multiple testing. All the statistical analyses and graphical plotting were performed in the R language environment (version 3.3.1). Unless otherwise specified, all the tests were two-tailed tests, and a p-value less than 0.05 was considered statistically significant.

### Assay result:

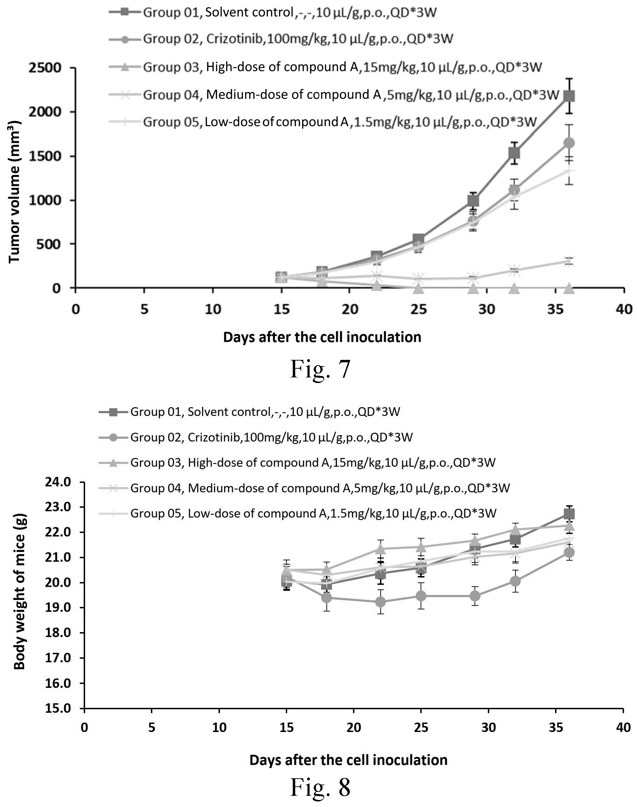

On the 21^{st} day after administration (i.e., at the end of the assay: the administration started on day 15 and ended on day 36), the average tumor volume of the mice in the solvent control group was 2177.24 mm³. In contrast, the assay drug compound A was administered under the administration regimens of p.o. once daily at 15 mg/kg, 5 mg/kg, and 1.5 mg/kg, and the average tumor volumes were 0 mm³, 305.46 mm³, and 1334.38 mm³, respectively, with the relative tumor growth inhibition rates (TGI) of 100%, 85.78%, and 38.05%, respectively. All of the results showed a statistically significant difference compared with the solvent control group (P<0.001 for both the 15 mg/kg and 5 mg/kg treatment groups, and P<0.05 for the 1.5 mg/kg treatment group). At the end of the assay, in the treatment group of compound A at 15 mg/kg, all (10/10) the tumor-bearing mice had their tumor volumes reduced to 0, and the tumors completely disappeared. The positive control drug crizotinib was administered under the administration regimen of p.o. once daily at up to 100 mg/kg, and the average tumor volume was 1651.66 mm³, with a relative tumor growth inhibition rate (TGI) of 25.52%. The results showed no statistically significant difference compared with the solvent control group (P>0.05). The therapeutic effect was worse than that of the 1.5 mg/kg dose group of compound A. The tumor growth in each treatment group and the control group is shown in Fig. 7.

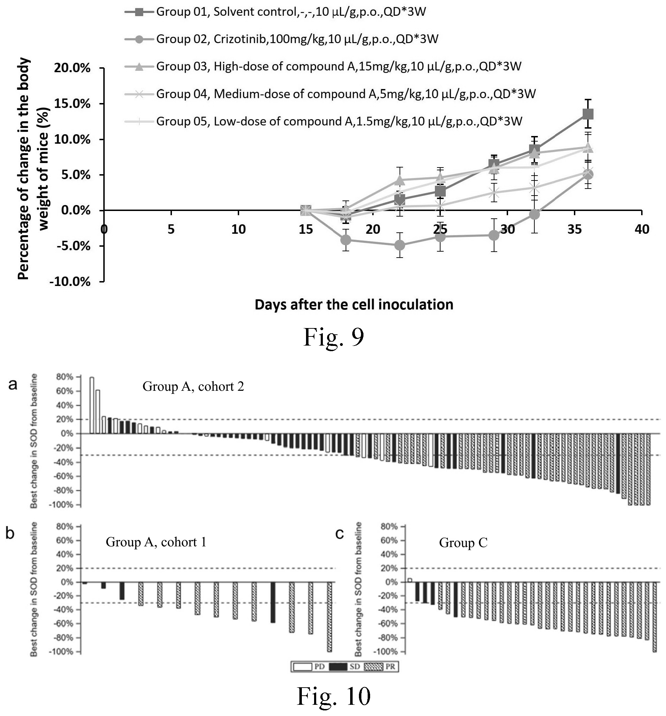

During the assay, no animals died in any of the treatment groups, and no obvious drug toxicity was observed. The animals were well tolerated during the treatment period. The body weight changes after the administration in the treatment and control groups are shown in Fig. 8 and Fig. 9.

Example 4: A dose-escalation and expansion phase I clinical trial evaluating the safety, tolerability, pharmacokinetics, and preliminary efficacy of compound A for the treatment of patients with ALK-positive or ROS1-positive advanced non-small cell lung cancer.

Purpose of the research:
Primary purpose:
   To evaluate the safety and tolerability of the compound A in patients with ALK-positive or ROS1-positive advanced non-small cell lung cancer (NSCLC), and to determine the maximum tolerated dose (MTD) and the recommended phase 2 dose (RP2D).
Secondary purpose:
   1) to evaluate the pharmacokinetic (PK) characteristics of the compound A in patients with ALK-positive or ROS1-positive advanced NSCLC;
   2) to evaluate the preliminary efficacy of the compound A in patients with ALK-positive or ROS1-positive advanced NSCLC.
Exploratory purpose:
   to explore the blood-brain barrier permeability of the compound A in patients with ALK-positive or ROS1-positive advanced NSCLC.

### Overall design

This trial is a multicenter, single-arm, open-label phase I clinical trial, consisting of three phases: dose escalation phase, dose expansion phase, and indication expansion phase, respectively, to explore the safety, tolerability, pharmacokinetics (PK), and preliminary efficacy of compound A in the patients with ALK-positive or ROS1-positive advanced NSCLC.

The dose escalation phase and dose expansion phase were planned to enroll the ALK-positive advanced NSCLC patients who had experienced progressive disease or intolerance after a continuous treatment with at least a second-generation ALK inhibitor (including those already on the market or in clinical trials) and the ROS1-positive advanced NSCLC patients who had failed the treatment with crizotinib.

Approximately eight dose groups in total were set in the dose escalation phase, which were 5 (the starting dose), 10, 20, 40, 60, 80, 100, and 125 mg, respectively. Subjects in all the dose groups received a single dose on day 1 (D1), and the blood samples were collected for pharmacokinetic (PK) assay and tolerability observation. If no grade 2 or a higher-level adverse event related to the investigational drug occurred during the single-dose period (5 to 7 days) (if such events occurred, dose adjustments would be made based on the toxicity according to the protocol), the subject entered the multiple-dose period (i.e., treatment period) 5 to 7 days later (7 days after the first administration in the first dose group (i.e., starting the multiple-dose treatment on day 8), and the intervals for the subsequent dose groups were determined based on the obtained tolerability and PK data). After entering the multiple-dose period, the subjects in the first dose group were initially administered with the drug once daily (QD) (if the PK data for the single-dose period had been obtained, the administration frequency for the multiple-dose period could be determined based on the PK data). The administration frequency for the subjects in the subsequent dose groups after entering the multiple-dose period was determined based on the obtained PK and tolerability data. In this trial, the administration frequency for all the subjects could be adjusted throughout the trial based on the continuously acquired safety and PK data. The four lower dose groups (5 mg, 10 mg, 20 mg and 40 mg) used an accelerated titration design, with one subject enrolled in each dose group. If any grade 2 or a higher-level adverse event related to the investigational drug occurred, the "3+3" dose escalation method would be switched to. The 60 mg dose group and the subsequent dose groups followed a "3+3" dose escalation method, and the MTD was determined. After the evaluation of the 8th dose group, if the researcher and the sponsor agreed that a higher dose group might have better efficacy and manageable safety, then a higher dose could be explored (e.g., the 9th dose group and the 10th dose group, at 150 mg and 180 mg, respectively). During the dose escalation, if a dose group was confirmed to have a preliminary antitumor effect and manageable safety (e.g., no DLT occurred in 3 subjects or only 1 out of 6 subjects experienced DLT), the dose level could be expanded after a discussion and confirmation by the researcher and the sponsor.

The dose expansion phase was expected to be conducted in multiple dose groups, with each dose group expected to expand to approximately 10 cases in total (including the dose escalation phase and the dose expansion phase), thereby determining an appropriate administration regimen for the subsequent indication expansion phase.

In the indication expansion phase, an appropriate administration regimen was determined based on the safety, tolerability, PK and efficacy data from the dose escalation phase and dose expansion phase, and the expansion was performed within the target population. The subjects in the expansion group were assigned to the following different populations based on their previously received treatments and gene mutation status, with the initial sample sizes as follows:
group A: the ALK-positive advanced NSCLC patients who had failed a first-line or a higher-line therapy (including those who had previously experienced progressive disease or intolerance after a continuous treatment with crizotinib or other ALK inhibitors), with at least 20 patients enrolled;
cohort 1: the ALK-positive advanced NSCLC patients who had experienced progressive disease or failure after receiving a continuous treatment with crizotinib as the only ALK inhibitor;
cohort 2: the ALK-positive advanced NSCLC patients who had experienced progressive disease or intolerance after a continuous treatment with at least a second-generation ALK inhibitor (including those already on the market or in clinical trials) (regardless of whether they had previously used crizotinib) (including this type of subjects using the target dose level during the dose escalation phase and dose expansion phase);
group B: ROS1-positive advanced NSCLC patients who had experienced progressive disease or intolerance after a continuous treatment with crizotinib, with at least a total of approximately 20 patients enrolled (including this type of subjects in the target dose group during the dose escalation phase and dose expansion phase);
group C: the ALK-positive advanced NSCLC patients who had not received a treatment with an ALK inhibitor, with at least a total of approximately 25 patients enrolled.

After obtaining the preliminary efficacy data of group A, the Safety Monitoring Committee (SMC) comprehensively considered the risk-benefit assessment and decided whether to initiate the enrollment of group C.

All the subjects in the dose escalation phase and dose expansion phase of this research needed to undergo intensive blood collection for PK study of the compound A, and all the subjects needed to undergo a population PK study of the compound A.

Descriptive statistical methods were used to analyze and compare the changes in the drug concentrations in cerebrospinal fluid and blood at the same time points after administration.

Selection criteria:
Patients meeting all of the following criteria were considered for enrollment:
1) agreeing to follow the treatment protocol and visitation plan of the trial, voluntarily enrolled, and having signed a written informed consent form;
2) age ≥ 18 years old on the day of signing the informed consent form, regardless of gender;
3) the following advanced NSCLC patients who were confirmed to be ALK-positive or ROS1-positive by histopathological or cytological examination, with the requirements for the treatment line for the subjects at different research phases as follows:
   dose escalation phase and dose expansion phase: the ALK-positive advanced NSCLC patients who had previously experienced progressive disease or intolerance after a treatment with at least a second-generation ALK inhibitor (including those already on the market or in clinical trials) and the ROS1-positive advanced NSCLC patients who had failed after a treatment with crizotinib;
   indication expansion phase: group A: the ALK-positive advanced NSCLC patients who had failed after a first-line or a higher-line therapy (including those who had previously experienced progressive disease or intolerance after a continuous treatment with crizotinib or other ALK inhibitors), including: cohort 1: the ALK-positive advanced NSCLC patients who had experienced progressive disease or failure after receiving a continuous treatment with crizotinib as the only ALK inhibitor; cohort 2: the ALK-positive advanced NSCLC patients who had experienced progressive disease or intolerance after a continuous treatment with at least a second-generation ALK inhibitor (including those already on the market or in clinical trials) (regardless of whether they had previously used crizotinib); group B: ROS1-positive advanced NSCLC patients who had experienced progressive disease or intolerance after a continuous treatment with crizotinib; group C: the ALK-positive advanced NSCLC patients who had not received a treatment with an ALK inhibitor.
4) The subjects were able to provide archived tissue samples and/or fresh tumor tissue samples obtained during the screening period for biomarker testing (those with previous biomarker testing results could be exempt from this test); or had previous next-generation sequencing (NGS) results. If a subject was unable to provide a tumor tissue sample (e.g., due to the exhaustion in previous diagnostic tests, high clinical risk of re-puncture, etc.), the subject might participate in this research only with the consent of the medical monitor; if a fresh tissue sample was available or a previous tissue sample was available, it must still be sent to a third-party central laboratory for retesting, and the test results would not affect the previous exemption.
5) Central nervous system metastases were permitted if the following conditions were met: a. asymptomatic: currently not requiring corticosteroid treatment or requiring a stable dose or a dose reduction to ≤10 mg QD of prednisone or equivalent; or b. a previous diagnosis had been made indicating that, treatment had been completed, the acute effects of the radiotherapy or surgery had been fully recovered before the first administration, the corticosteroid treatment for these metastases had been discontinued for at least 4 weeks, and the nervous system was stable;
6) before the first administration of the investigational drug, the patients who had previously received targeted therapy (specifically with ALK or ROS1 inhibitors) must had discontinued the drug for ≥ 5 half-lives;
7) according to the criteria in RECIST version 1.1, there must be at least one measurable target lesion (the longest diameter of a non-lymph node ≥ 10 mm, the shortest diameter of a lymph node ≥ 15 mm); the lesion which had been previously treated with radiotherapy could not be considered as a target lesion unless the lesion had significantly progressed;
8) eastern Cooperative Oncology Group (ECOG) Performance Status (PS): dose escalation phase and dose expansion phase: score 0 to 1; indication expansion phase: score 0 to 2;
9) adequate bone marrow, liver, kidney, coagulation, and pancreatic function (referring to the upper limit of normal at each clinical trial center):
   bone marrow (no blood transfusions or use of medications that help increase the white blood cells, platelets, hemoglobin, or red blood cells within 2 weeks prior to the screening check):
   absolute neutrophil count (ANC) ≥ 1.5 × 10⁹/L;
   platelet count ≥ 100 × 10⁹/L;
   hemoglobin ≥ 90 g/L;
   liver function:
   serum total bilirubin ≤ 1.5 times the upper limit of normal (ULN);
   in the absence of a liver metastasis, alanine aminotransferase (ALT), aspartate aminotransferase (AST), or alkaline phosphatase (ALP) ≤ 2.5 times the ULN; in the presence of a liver metastasis, ALT, AST, or ALP ≤ 5 times the ULN;
   kidney function: serum creatinine ≤ 1.5 times the ULN;
   coagulation function: international normalized ratio (INR) ≤ 1.5 × ULN, the activated partial thromboplastin time (APTT) ≤ 1.5 × ULN;
   pancreatic function:
   serum total amylase ≤ 1.5 times the ULN;
   serum lipase ≤ 1.5 times the ULN;
10) expected survival ≥ 3 months;
11) men with fertility and women of childbearing age were willing to take effective contraception from the date of signing the informed consent form until 6 months after the last administration of the investigational drug; the women of childbearing age included the premenopausal women and women within two years of menopause. The result of a pregnancy test conducted ≤7 days prior to the first administration of the investigational drug must be negative for the women of childbearing age.

Exclusion criteria:
Patients meeting any one of the following criteria were ineligible for enrollment:
1) subjects who had previously received a third-generation ALK inhibitor or a second-generation ROS1 inhibitor other than the compound A;
2) subjects with a known allergy to any active ingredient or excipient of compound A, or a history of known allergy to protein drugs, a history of specific allergic reactions (asthma, rheumatism, eczema), or a history of other severe allergic reactions that the investigators determined were unsuitable for the treatment with compound A;
3) subjects with another malignant tumor other than lung cancer; except for the cervical cancer in situ and non-melanoma skin cancer that had received curative treatment and had not recurred within 5 years;
4) subjects who had undergone a major surgery within 4 weeks prior to the first administration; minor surgical procedures (e.g., port implantation) were not exclusion criteria, but sufficient time was required for adequate wound healing;
5) spinal cord compression, unless the subject had achieved good pain control through treatment and the neurological function had fully recovered within 4 weeks prior to the first administration;
6) gastrointestinal dysfunction, primarily including inability to take oral medications, need for intravenous nutrition, previous surgical procedures (including total gastrectomy or gastric banding surgery) affecting the absorption, active inflammatory gastrointestinal disease, chronic diarrhea, symptomatic diverticulosis, treatment of active peptic ulcer disease within the past 6 months, malabsorption syndrome, etc.;
7) patients with a history of active pneumonia or clinically significant interstitial lung disease, or patients with a radiation or drug-induced pulmonary disease requiring treatment; the patients with radiation pneumonitis who were 3 months post-radiotherapy and had no clinical symptoms could be enrolled;
8) patients with cardiac dysfunction, including but not limited to those with a left ventricular ejection fraction (LVEF) <50%, a history of congestive heart failure or a history of ventricular arrhythmias requiring treatment, hypokalemia, hereditary long QT syndrome, or a history of myocardial infarction or unstable angina within 6 months prior to the screening; patients with heart failure classified by the New York Heart Association as class ≥2;
9) patients with clinically significant abnormalities in QTc in the electrocardiogram (ECG) examination (QTc >450 msec [male] or QTc >470 msec [female] obtained in the ECG examination at rest); concomitant use of any medication known to prolong the QT interval and cause torsades de pointes ventricular tachycardia;
10) patients with uncontrolled hypertension (systolic blood pressure ≥ 160 mmHg or diastolic blood pressure ≥ 100 mmHg) after a drug treatment;
11) patients with uncontrolled hyperglycemia, or acute cholelithiasis, or those characterized by the susceptibility to acute pancreatitis;
12) patients with a severe or uncontrollable systemic disease (such as an unstable or uncompensated respiratory, cardiac, hepatic, or renal disease) who were expected to be unable to tolerate the investigational drug treatment, based on the investigators' judgment;
13) patients who had used the following medications within 14 days prior to the first administration or needed to use the following medications during the treatment: medications that pose a risk of QTc interval prolongation and/or ventricular tachycardia;
14) patients with previous antitumor therapy: (1) patients who had received any systemic antitumor therapy within 28 days prior to the first administration of the investigational drug (if the drug had been discontinued for more than 5 half-lives and the compound A could be used within 28 days after the end of the systemic antitumor therapy as assessed by the investigators, the patients could be enrolled into the trial), including systemic chemotherapy, immunotherapy (physiological replacement dose of glucocorticoids [prednisone or equivalent <10 [mg/day] were allowed to use), large molecule monoclonal antibodies, small molecule targeted drugs, etc.; (2) patients who had received radiotherapy or Chinese herbal medicine or proprietary Chinese medicine approved for anti-tumor treatment within 14 days prior to the first administration; (3) patients who had received an antibody or drug treatment targeting T cell co-stimulation or immune checkpoint pathway within the past 28 days, including but not limited to anti-PD-1, anti-PD-L1, anti-PD-L2, anti-CD137 or anti-CTLA-4 antibodies; for the subjects who were removed from the group due to progressive disease in the lower dose group and then received a higher dose level again, there was no limit to the washout period of the systemic anti-tumor treatment before the investigational drug was administered again;
15) subjects who had not recovered from a previous surgical procedure and a previous anti-tumor treatment-related toxic reaction, which were assessed by the investigators as affecting the safety of the subjects;
16) patients with clinically significant active bacterial, fungal or viral infection, including those who were positive for hepatitis B surface antigen and had HBV DNA ≥ ULN (only in the patients who were positive for hepatitis B surface antigen), or those who were positive in the test results of any one or more of the following: hepatitis C (patients who were positive for hepatitis C antibody but negative for HCV RNA polymerase chain reaction (PCR) test were eligible for enrollment), treponema pallidum antibody, or human immunodeficiency virus antibody test, or those with any uncontrolled infection;
17) patients who had taken a strong inducer or inhibitor of CYP3A4, or a CYP3A4 substrate with a narrow therapeutic window, within two weeks prior to the first administration of the investigational drug;
18) pregnant or breastfeeding female subjects;
19) women of childbearing age who were unwilling or unable to use an acceptable method of contraception throughout the entire treatment period of this trial and for 6 months after the last administration of the investigational drug (women of childbearing age included: any woman who had had menarche and had not undergone successful artificial sterilization surgery (hysterectomy, bilateral tubal ligation, or bilateral oophorectomy) or was not menopausal); and fertile male patients who were unwilling or unable to use effective contraception if their partners were women of childbearing age;
20) subjects who were participating in other clinical researches or whose first administration was less than 4 weeks or 5 half-lives of the investigational drug from the end of the previous clinical research, whichever was shorter;
21) patients with any mental or cognitive impairment that might limit their understanding and compliance with the informed consent form; other situations deemed unsuitable for the participation of this research by the investigators, such as poor compliance.

Trial results:
1) As of July 1, 2023, a total of 268 subjects were screened for the dose escalation and expansion phases and the indication expansion phase. Of these, 198 subjects were successful in the screening and 69 subjects failed. The reasons for failure were either not meeting the selection criteria or meeting the exclusion criteria. Among the 198 subjects, 171 subjects were ALK-positive and 27 subjects were ROS1-positive; 114 subjects had brain metastases at enrollment; 192 subjects had a recent tumor stage of IV at enrollment. The baseline information of the subjects' diseases is detailed in Table 1 and Table 2.

**Table 1: The baseline characteristics of the patients during the dose escalation and expansion phases and indication expansion phase**

| Parameter Statistics | Dose escalation and expansion phases (N=42) | Group A, cohort 1 (N=14) | Group A, cohort 2 (N=85) | Group B (N=24) | Group C (N=33) | In total (N=198) |
|---|---|---|---|---|---|---|
| Pathological classification, number of cases (%) | | | | | | |
| Squamous cell carcinoma | 0 | 0 | 0 | 0 | 0 | 0 |
| Adenocarcinoma | 39 (92.9) | 14 (100) | 84 (98.8) | 23 (95.8) | 32 (97.0) | 192 (97.0%) |
| Large-cell carcinoma | 0 | 0 | 1 (1.2) | 1 (4.2) | 1 (3.0) | 3 (1.5) |
| Others | 3 (7.1) | 0 | 0 | 0 | 0 | 3 (1.5) |

| Previous chemotherapy, number of cases (%) | | | | | | |
|---|---|---|---|---|---|---|
| Yes | 20 (47.6) | 5 (35.7) | 34 (40.0) | 12 (50.0) | 11 (33.3) | 82 (41.4) |
| No | 22 (52.4) | 9 (64.3) | 51 (60.0) | 12 (50.0) | 22 (66.7) | 116 (58.6) |

| Previous ALK or ROS1 TKIs treatment, number of cases (%) | | | | | | |
|---|---|---|---|---|---|---|
| None | 0 | 0 | 0 | 0 | 33 (100) | 33 (16.7) |
| 1 type | 14 (33.3) | 14 (100) | 36 (42.4) | 24 (100) | 0 | 88 (44.4) |
| 2 types | 23 (54.8) | 0 | 32 (37.6) | 0 | 0 | 55 (27.8) |
| 3 types and more | 5 (11.9) | 0 | 17 (20.0) | 0 | 0 | 22 (11.1) |

| Whether metastasized, number of cases (%) | | | | | | |
|---|---|---|---|---|---|---|
| Yes | 40 (95.2) | 12 (85.7) | 81 (95.3) | 21 (87.5) | 30 (90.9) | 184 (92.9) |
| No | 2 (4.8) | 2 (14.3) | 4 (4.7) | 3 (12.5) | 3 (9.1) | 14 (7.1) |
| Intracranial metastasis, number of cases (%) | 25 (60.0) | 11 (78.6) | 50 (58.8) | 13 (54.2) | 15 (45.5) | 114 (57.6) |

| ALK/ROS1, number of cases (%) | | | | | | |
|---|---|---|---|---|---|---|
| ALK-positive | 39 (92.9) | 14 (100) | 85 (100) | 0 | 33 (100) | 171 (86.4) |
| ROS 1-positive | 3 (7.1) | 0 | 0 | 24 (100) | 0 | 27 (13.6) |

| ECOG score, number of cases (%) | | | | | | |
|---|---|---|---|---|---|---|
| 0 | 12 (28.6) | 3 (23.1) | 12 (14.1) | 1 (4.2) | 2 (6.1) | 30 (15.2) |
| 1 | 30 (71.4) | 11 (76.9) | 71 (83.5) | 22 (91.7) | 29 (87.9) | 163 (82.3) |
| 2 | 0 | 0 | 2 (2.4) | 1 (4.2) | 2 (6.1) | 5 (2.6) |

| Recent clinical staging, number of cases (%) | | | | | | |
|---|---|---|---|---|---|---|
| I | 0 | 0 | 0 | 0 | 0 | |
| II | 0 | 0 | 0 | 0 | 0 | |
| III | 0 | 0 | 3 (3.5) | 0 | 2 (6.1) | |
| IV | 41 (100)* | 14 (100) | 82 (96.5) | 24 (100) | 31 (93.9) | |

| | | | | | | |
|---|---|---|---|---|---|---|
| *: One subject was missing in the 60mg group. | | | | | | |

**Table 2 The baseline characteristics of the patients during the dose escalation and expansion phases**

| Parameter Statistics | | 5mg (N=1) | 10mg (N=1) | 20mg (N=1) | 40mg (N=10) | 60mg (N=12) | 80mg (N=11) | 100mg (N=3) | 125mg (N=3) | In total (N=42) |
|---|---|---|---|---|---|---|---|---|---|---|
| Pathological classification, number of cases (%) | | 1 | 1 | 1 | 10 | 12 | 11 | 3 | 3 | 42 |
| Squamous cell carcinoma | | 0 | 0 | 0 | 0 | 0 | 0 | 0 | 0 | 0 |
| | Adenocarcinoma | 1 (100) | 1 (100) | 1 (100) | 9 (90.0) | 12 (100) | 9 (81.8) | 3 (100) | 3 (100) | 39 (92.9) |
| | Large-cell carcinoma | 0 | 0 | 0 | 0 | 0 | 0 | 0 | 0 | 0 |
| | Others | 0 | 0 | 0 | 1 (10.0) | 0 | 2 (18.2) | 0 | 0 | 3(7.1) |
| Recent clinical staging of tumors, number of cases (%) | | 1 | 1 | 1 | 10 | 11 (1 case was missing) | 11 | 3 | 3 | 41 (1 case was missing) |
| | I | 0 | 0 | 0 | 0 | 0 | 0 | 0 | 0 | 0 |
| | II | 0 | 0 | 0 | 0 | 0 | 0 | 0 | 0 | 0 |
| | III | 0 | 0 | 0 | 0 | 0 | 0 | 0 | 0 | 0 |
| | IV | 1 (100) | 1 (100) | 1 (100) | 10 (100) | 11 (100) | 11 (100) | 3 (100) | 3 (100) | 41 (100) |
| Previous ALK or ROS1 TKIs treatment | | | | | | | | | | |
| | None | 0 | 0 | 0 | 0 | 0 | 0 | 0 | 0 | 0 |
| | 1 type | 1 (100) | 0 | 0 | 2 (20.0) | 4 (33.3) | 4 (36.4) | 1 (33.3) | 2 (66.7) | **14** (33.3) |
| | 2 types and more | 0 | 1 (100) | 1 (100) | 8 (80.0) | 8 (66.7) | 7 (63.6) | 2 (66.7) | 1 (33.3) | 28 (66.7) |
| Whether metastasized, number of cases (%) | | 1 | 1 | 1 | 10 | 12 | 11 | 3 | 3 | 42 |
| | Yes | 1 (100) | 1 (100) | 1 (100) | 9 (90.0) | 12 (100) | 10 (90.9) | 3 (100) | 3 (100) | 40 (95.2) |
| | No | 0 | 0 | 0 | 1 (10.0) | 0 | 1 (9.1) | 0 | 0 | 2 (4.8) |
| Distribution of the distant metastatic sites, number of cases (%) | | 1 | 1 | 1 | 9 (1) | 12 | 10 (1) | 3 | 3 | 40 (2) |
| | Intracranial metastasis | 1 (100) | 1 (100) | 1 (100) | 6 (66.7) | 5 (41.7) | 7 (70.0) | 3 (100) | 1 (33.3) | 25 (62.5) |
| | Bone metastasis | 0 | 1 (100) | 0 | 3 (33.3) | 5 (41.7) | 6 (60.0) | 2 (66.7) | 3 (100) | 20 (50.0) |
| | Liver metastasis | 0 | 0 | 0 | 0 | 5 (41.7) | 2 (20.0) | 0 | 3 (100) | 10 (25.0) |
| | Adrenal gland metastasis | 0 | 0 | 0 | 0 | 0 | 1 (10.0) | 0 | 1 (33.3) | 2 (5.0) |
| | Others | 0 | 0 | 0 | 5 (55.6) | 8 (66.7) | 8 (80.0) | 2 (66.7) | 2 (66.7) | 25 (62.5) |
| ALK/ROS1, number of cases (%) | | 1 | 1 | 1 | 10 | 12 | 11 | 3 | 3 | 42 |
| | ALK-positive | 1 (100) | 1 (100) | 1 (100) | 9 (90.0) | 12 (100) | 10 (90.9) | 2 (66.7) | 3 (100) | 39 (92.9) |
| | ROS1-positive | 0 | 0 | 0 | 1 (10.0) | 0 | 1 (9.1) | 1 (33.3) | 0 | 3(7.1) |
| | ALK/ROS1 double positive | 0 | 0 | 0 | 0 | 0 | 0 | 0 | 0 | 0 |

2) Among the 42 subjects in the dose escalation and expansion phases, 11 subjects (26.2%) had previously received radiotherapy, and all were treated for NSCLC. All the 42 subjects had previously received an anti-tumor drug treatment, mainly with alectinib, crizotinib, and pemetrexed (see Table 3). 11 subjects (26.2%) had previously received a first-line therapy, 14 subjects (33.3%) had previously received a second-line therapy, 9 subjects (21.4%) had previously received a third-line therapy, 6 subjects (14.3%) had previously received a fourth-line therapy, and 2 subjects (4.8%) had previously received a fifth-line therapy. 6 subjects (14.3%) had undergone an anti-tumor surgery.

**Table 3. Previous anti-tumor drugs in the dose escalation and expansion phases**

| ATC classification level 2 PN | | 5mg (N=1) n (%) | 10mg (N=1) n (%) | 20mg (N=1) n (%) | 40mg (N=10) n (%) | 60mg (N=12) n (%) | 80mg (N=11) n (%) | 100mg (N=3) n (%) | 125mg (N=3) n (%) | In total (N=42) n (%) |
|---|---|---|---|---|---|---|---|---|---|---|
| At least one previous anti-tumor treatment drug | | 1 (100) | 1 (100) | 1 (100) | 10 (100) | 12 (100) | 11 (100) | 3 (100) | 3 (100) | 42 (100) |
| | Anti-tumor drugs | 1 (100) | 1 (100) | 1 (100) | 10 (100) | 12 (100) | 11 (100) | 3 (100) | 3 (100) | 42 (100) |
| | Alectinib | 0 | 0 | 0 | 7 (70.0) | 7 (58.3) | 4 (36.4) | 2 (66.7) | 3 (100) | 23 (54.8) |
| | Crizotinib | 0 | 0 | 0 | 7 (70.0) | 5 (41.7) | 4 (36.4) | 3 (100) | 0 | 19 (45.2) |
| | Pemetrexed | 0 | 0 | 1 (100) | 5 (50.0) | 6 (50.0) | 5 (45.5) | 1 (33.3) | 1 (33.3) | 19 (45.2) |
| | Carboplatin | 0 | 0 | 0 | 4 (40.0) | 5 (41.7) | 3 (27.3) | 1 (33.3) | 1 (33.3) | **14** (33.3) |
| | Anaplastic lymphoma kinase (ALK) inhibitors | 1 (100) | 1 (100) | 1 (100) | 2 (20.0) | 4 (33.3) | 1 (9.1) | 0 | 0 | **10** (23.8) |
| | Bevacizumab | 0 | 0 | 0 | 1 (10.0) | 5 (41.7) | 2 (18.2) | 0 | 1 (33.3) | 9 (21.4) |
| | APG 2449 | 0 | 1 (100) | 1 (100) | 2 (20.0) | 2 (16.7) | 0 | 1 (33.3) | 1 (33.3) | 8 (19.0) |
| | Ceritinib | 0 | 0 | 0 | 1 (10.0) | 2 (16.7) | 4 (36.4) | 0 | 0 | 7 (16.7) |
| | Ensartinib | 0 | 0 | 0 | 2 (20.0) | 1 (8.3) | 3 (27.3) | 0 | 0 | 6 (14.3) |
| | Nedaplatin | 0 | 0 | 1 (100) | 1 (10.0) | 1 (8.3) | 3 (27.3) | 0 | 0 | 6 (14.3) |
| | Cisplatin | 0 | 0 | 0 | 2 (20.0) | 2 (16.7) | 1 (9.1) | 0 | 0 | 5 (11.9) |
| | Paclitaxel | 0 | 0 | 0 | 2 (20.0) | 0 | 1 (9.1) | 0 | 0 | 3 (7.1) |
| | Anlotinib | 0 | 0 | 0 | 1 (10.0) | 1 (8.3) | 0 | 0 | 0 | 2 (4.8) |
| | Brigatinib | 0 | 0 | 0 | 1 (10.0) | 0 | 1 (9.1) | 0 | 0 | 2 (4.8) |
| | Docetaxel | 0 | 0 | 0 | 1 (10.0) | 1 (8.3) | 0 | 0 | 0 | 2 (4.8) |
| | Gefitinib | 0 | 0 | 0 | 0 | 0 | 2 (18.2) | 0 | 0 | 2 (4.8) |
| | Gemcitabine | 0 | 0 | 1 (100) | 0 | 0 | 1 (9.1) | 0 | 0 | 2 (4.8) |
| | Endostatin | 0 | 0 | 0 | 1 (10.0) | 1 (8.3) | 0 | 0 | 0 | 2 (4.8) |
| | Brucea javanica | 0 | 0 | 0 | 0 | 0 | 2 (18.2) | 0 | 0 | 2 (4.8) |
| | Gimeracil; oteracil; tegafur | 0 | 0 | 0 | 0 | 1 (8.3) | 0 | 0 | 0 | 1 (2.4) |
| | Conteltinib | 0 | 0 | 0 | 0 | 0 | 1 (9.1) | 0 | 0 | 1 (2.4) |
| | Lobaplatin | 0 | 0 | 0 | 0 | 1 (8.3) | 0 | 0 | 0 | 1 (2.4) |
| | Cisplatin; pemetrexed | 0 | 0 | 0 | 1 (10.0) | 0 | 0 | 0 | 0 | 1 (2.4) |
| | Immunopotentiator | 0 | 0 | 0 | 0 | 1 (8.3) | 0 | 0 | 0 | 1 (2.4) |
| | Mongolian Astragalus; codonopsis | 0 | 0 | 0 | 0 | 1 (8.3) | 0 | 0 | 0 | 1 (2.4) |

A total of 97 subjects were included in the 60 mg dose group who had previously received at least one second-generation ALK inhibitor, including 85 subjects in the cohort 2 of group A in the indication expansion phase and 12 subjects in the 60 mg dose group in the dose escalation and expansion phases. Among these 97 subjects, 59 (60.8%), 40 (41.2%), 25 (25.8%), 13 (13.4%), and 30 (30.9%) subjects had previously received alectinib, crizotinib, ceritinib, ensartinib, and other ALK tyrosine kinase inhibitors, respectively.

### 3) Cerebrospinal fluid concentration

During the dose escalation phase, analysis of the cerebrospinal fluid concentration was performed on 4 subjects. Due to the low protein concentration in cerebrospinal fluid, the plasma concentration of the compound A was used as the free concentration when calculating the blood-brain barrier permeability. The results are shown in Table 4.

**Table 4. Analysis of the concentration of compound A in cerebrospinal fluid**

| Subject number | Dose group | Plasma concentration (ng/mL) | CSF concentration (ng/mL) | CSF/Plasma |
|---|---|---|---|---|
| S01002 | 5 mg | 15.839 | 9.666 | 61.03% |
| S01009 | 60 mg | 122.025 | 84.120 | 68.94% |
| S01008 | 60 mg | 156.895 | 68.988 | 43.97% |
| S01017 | 80 mg | 157.641 | 74.718 | 47.40% |

### 4) Efficacy analysis

① The overall remission and intracranial remission results of the subjects in the dose escalation and expansion phases are shown in Table 5 and Table 6 below.

**Table 5. Analysis of systemic BOR, ORR, DCR, DOR, and TTR during the dose escalation and expansion phases.**

| | | 5mg (N=1) | 10mg (N=1) | 20mg (N=1) | 40mg (N=10) | 60mg (N=12) | 80mg (N=11) | 100mg (N=3) | 125mg (N=3) |
|---|---|---|---|---|---|---|---|---|---|
| BOR, n (%) | | | | | | | | | |
| | CR | 0 | 0 | 0 | 0 | 0 | 0 | 0 | 0 |
| | PR | 0 | 0 | 1 (100) | 1 (10.0) | 6 (50.0) | 4 (36.4) | 2 (66.7) | 1 (33.3) |
| | SD | 1 (100) | 1 (100) | 0 | 5 (50.0) | 5 (41.7) | 5 (45.5) | 1 (33.3) | 1 (33.3) |
| | PD | 0 | 0 | 0 | 2 (20.0) | 1 (8.3) | 1 (9.1) | 0 | 1 (33.3) |
| | NE | 0 | 0 | 0 | 2 (20.0) | 0 | 1 (9.1) | 0 | 0 |
| Confirmed ORR, n (%) (95% CI) | | 0 (0.0, 97.5) | 0 (0.0, 97.5) | 1 (100) (2.5, 100.0) | 1 (10.0) (0.3, 44.5) | 6 (50.0) (21.1, 78.9) | 4 (36.4) (10.9, 69.2) | 2 (66.7) (9.4, 99.2) | 1 (33.3) (0.8, 90.6) |
| DCR, n (%) (95% CI) | | 1 (100) (2.5, 100.0) | 1 (100) (2.5, 100.0) | 1 (100) (2.5, 100.0) | 6 (60.0) (26.2, 87.8) | 11 (91.7) (61.5, 99.8) | 9 (81.8) (48.2, 97.7) | 3 (100) (29.2, 100.0) | 2 (66.7) (9.4, 99.2) |
| Median DOR, months (95% CI) | | - | - | 6.93 (-, -) | - | 12.45 (2.79, -) | 12.48 (7.98, -) | 5.57 (4.17, -) | 9.53 (-, -) |
| Median TTR, months (range) | | - | - | 1.35 (1.35, 1.35) | 2.73 (2.73, 2.73) | 1.35 (1.35, 6.90) | 1.45 (1.35, 13.96) | 2.05 (1.35, 2.76) | 1.38 (1.38, 1.38) |

**Table 6. Analysis of intracranial BOR, ORR, DCR, DOR, and TTR during the dose escalation and expansion phases**

| | | 5mg (N=0) | 10mg (N=1) | 20mg (N=1) | 40mg (N=4) | 60mg (N=2) | 80mg (N=4) | 100mg (N=2) | 125mg (N=1) |
|---|---|---|---|---|---|---|---|---|---|
| BOR, n (%) | | | | | | | | | |
| | CR | 0 | 0 | 0 | 0 | 0 | 0 | 0 | 0 |
| | PR | 0 | 1 (100) | 1 (100) | 0 | 2 (100) | 1 (25.0) | 0 | 0 |
| | SD | 0 | 0 | 0 | 3 (75.0) | 0 | 2 (50.0) | 2 (100) | 1 (100) |
| | PD | 0 | 0 | 0 | 0 | 0 | 0 | 0 | 0 |
| | NE | 0 | 0 | 0 | 1 (25.0) | 0 | 1 (25.0) | 0 | 0 |
| Confirmed IC-ORR, n (%) (95% CI) | | 0 | 1 (100) (2.5, 100.0) | 1 (100) (2.5, 100.0) | 0 (0.0, 60.2) | 2 (100) (15.8, 100.0) | 1 (25.0) (0.6, 80.6) | 0 (0.0, 84.2) | 0 (0.0, 97.5) |
| IC-DCR, n (%) (95% CI) | | 0 | 1 (100) (2.5, 100.0) | 1 (100) (2.5, 100.0) | 3 (75.0) (19.4, 99.4) | 2 (100) (15.8, 100.0) | 3 (75.0) (19.4, 99.4) | 2 (100) (15.8, 100.0) | 1 (100) (2.5,100.0) |
| Median IC-DOR, months (95% CI) | | - | 5.55 (-, -) | - | - | 17.97 (-, -) | - | - | - |
| Median IC-TTR, months (range) | | - | 1.35 (1.35, 1.35) | 1.35 (1.35, 1.35) | - | 4.11 (1.35, 6.87) | 1.51 (1.51, 1.51) | - | - |

| | | | | | | | | | |
|---|---|---|---|---|---|---|---|---|---|
| ② The overall remission and intracranial remission results of the subjects in the indication expansion phase are shown in Table 7 and Table 8 below. | | | | | | | | | |

**Table 7. Analysis of systemic BOR, ORR, DCR, DOR, and TTR during the indication expansion phase**

| | | Group A, cohort 1 (n=14) | Group A, cohort 2 | Group C (n=33) | In total (ALK-positive) (n=144) | Group B (n=24) |
|---|---|---|---|---|---|---|
| | | | (n=97)* | | | |
| BOR, n (%) | | | | | | |
| | CR | 0 | 0 | 0 | 0 | 0 |
| | PR | 10 (71.4) | 38 (39.2) | 28 (84.8) | 84 (49.1) | 7 (29.2) |
| | SD | 4 (28.6) | 41 (42.3) | 4 (12.1) | 61 (35.7) | 10 (41.7) |
| | PD | 0 | 16 (16.5) | 1 (3.0) | 21 (12.3) | 7 (29.2) |
| | NE | 0 | 2 (2.1) | 0 | 5 (2.9) | 0 |
| Confirmed ORR, n (%) (95% CI) | | 10 (71.4) (41.9, 91.6) | 38 (39.2) (29.4, 49.6) | 28 (84.8) (68.1, 94.9) | 84 (49.1) (41.4, 56.9) | 7 (29.2) (12.6,51.1) |
| DCR, n (%) (95% CI) | | 14 (100) (76.8, 100.0) | 79 (81.4) (72.3, 88.6) | 32 (97.0) (84.2, 99.9) | 145 (84.8) (78.5, 89.8) | 17 (70.8) (48.9, 87.4) |
| Median DOR, months (95% CI) | | NR (5.585, NR) | 12.48 (12.45, NR) | NR (9.69, NR) | 12.48 (12.45, -) | NR (4.370, -) |
| Median TTR, months (range) | | 2.71 (1.25, 9.66) | 1.38 (1.28, 9.72) | 1.35 (1.12, 4.21) | 1.38 (1.12, 13.96) | 1.41 (1.31, 7.03) |

| | | | | | | |
|---|---|---|---|---|---|---|
| *: 85 subjects in the cohort 2 of group A in the indication expansion phase and 12 subjects in the 60 mg dose group in the dose escalation and expansion phases were included. | | | | | | |

**Table 8. Analysis of BOR, ORR, DCR, DOR, and TTR of intracranial measurable lesions during the indication expansion phase**

| | | Group A, cohort 1 (n=4) | Group A, cohort 2 (n=27) | Group C (n=8) | In total (ALK-positive) (n=52) | Group B (n=6) |
|---|---|---|---|---|---|---|
| BOR, n (%) | | | | | | |
| | CR | 1 (25.0) | 5 (18.5) | 0 | 6 (11.5) | 0 |
| | PR | 1 (25.0) | 14 (51.9) | 6 (75.0) | 24 (46.2) | 3 (50.0) |
| | SD | 2 (50.0) | 7 (25.9) | 2 (25.0) | 19 (36.5) | 2 (33.3) |
| | PD | 0 | 0 | 0 | 1 (1.9) | 1 (16.7) |
| | NE | 0 | 1 (3.7) | 0 | 2 (3.8) | 0 |
| Confirmed ORR, n (%) (95% CI) | | 2 (50.0) (6.8, 93.2) | 19 (70.4) (49.8, 86.2) | 6 (75.0) (34.9, 96.8) | 30 (57.7) (43.2, 71.3) | 3 (50.0) (11.8, 88.2) |
| DCR, n (%) (95% CI) | | 4 (100) (39.8, 100.0) | 26 (96.3) (81.0, 99.9) | 8 (100) (63.1, 100) | 49 (94.2) (84.1, 98.8) | 5 (83.3) (35.9, 99.6) |
| Median DOR, months (95% CI) | | NR (NR, NR) | 17.97 (6.64, NR) | NR (4.17, NR) | 17.97 (17.97, -) | - (4.17, -) |
| Median TTR, months (range) | | 1.36 (1.35, 1.38) | 1.41 (1.31, 9.72) | 1.41 (1.35, 1.45) | 1.40 (1.31, 9.72) | 1.41 (1.35, 1.41) |

In the 14 patients who had previously received crizotinib with or without chemotherapy, the ORR was 71.4% (95% CI: 41.9% to 91.6%); 10 patients (71.4%) achieved a confirmed PR, and 4 patients (28.6%) achieved SD. The median DOR was not reached (95% CI: 5.585 to [NR]), and the median TTR was 2.71 months (range: 1.25 to 9.66). The best percentage change in disease status from the baseline after the treatment with compound A is shown in Fig. 10b. Four patients had measurable central nervous system lesions at baseline, with an intracranial ORR of 50.0% (1 CR and 1 PR). The intracranial median DOR was not reached, and the median TTR was 1.36 months.

In the 97 patients who had previously received a second-generation TKI treatment, the overall ORR was 39.2% (95% CI: 29.4% to 49.6%), the median DOR was 12.48 months (95% CI: [12.45] to [NE]), and the median TTR was 1.38 months (range: 1.28 to 9.72). The best percentage change in disease status from the baseline after the treatment with compound A is shown in Fig. 10a. In the 27 patients with measurable central nervous system lesions at baseline, 5 patients achieved CR and 14 patients achieved PR. The IC-ORR was 70.4% (95% CI: 49.8% to 86.2%). The median intracranial DOR was 17.97 months (95% CI: 6.64 to [NR]), and the median intracranial TTR was 1.41 months (range: 1.31 to 9.72).

A total of 33 ALK TKI-treatment naive patients were analyzed, with an ORR of 84.8% (95% CI: 68.1% to 94.9%), of whom 28 patients achieved PR and 4 patients achieved SD. At a median follow-up of 10.45 months, the median DOR was not reached (95% CI: [9.69] to [NR]), and the median TTR was 1.35 months (range: 1.12 to 4.21). The best percentage change in disease status from the baseline after the treatment with compound A is shown in Fig. 10c. For the 8 patients with measurable central nervous system lesions at baseline, the intracranial ORR was 75% (95% CI, 34.9% to 96.8%), and 6 patients achieved a confirmed PR. The median intracranial DOR was not reached (95% CI, 4.17 to [NR]), and the median TTR was 1.41 months (range: 1.35 to 1.45).

In the 24 ROS1-positive NSCLC patients who had previously received a treatment with crizotinib, the ORR was 29.2% (95% CI: 12.6% to 51.1%). The median DOR was not reached (95% CI: 4.370 to [NR]), and the median TTR was 1.41 months (range: 1.31 to 7.03). Six of these patients had measurable central nervous system lesions at baseline, with an intracranial ORR of 50.0% (3 PR). The intracranial median DOR was not reached, and the median TTR was 1.41 months.

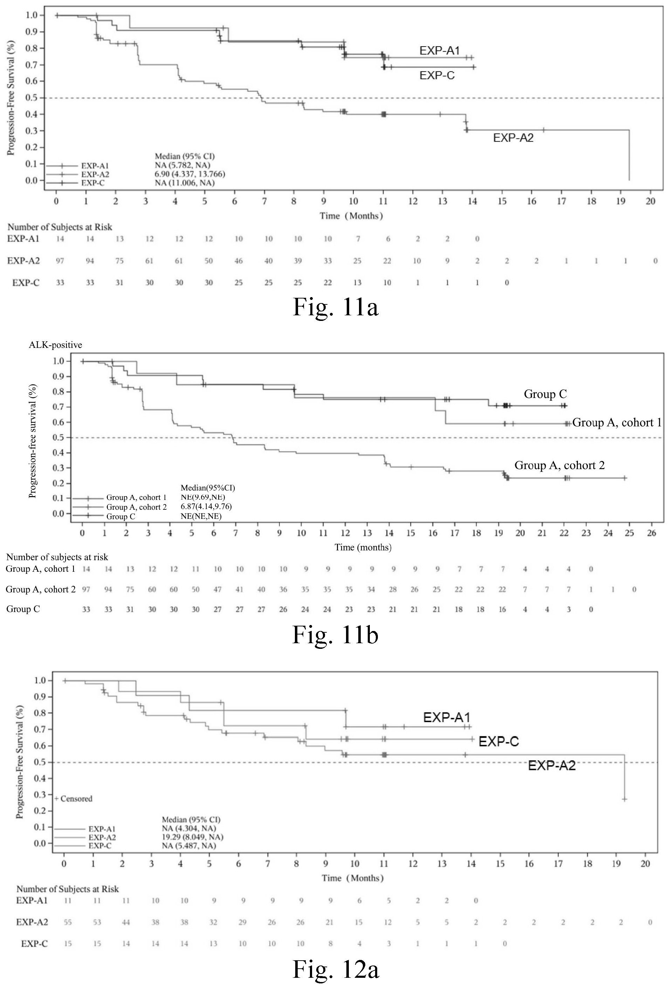

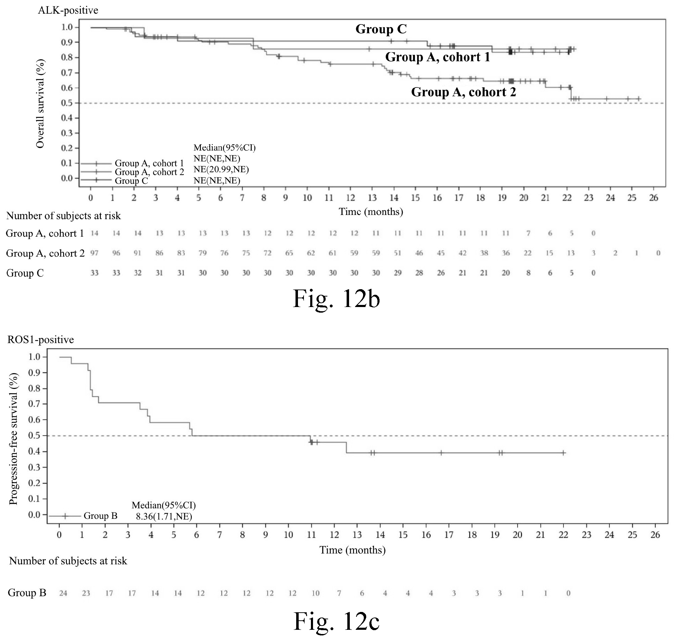

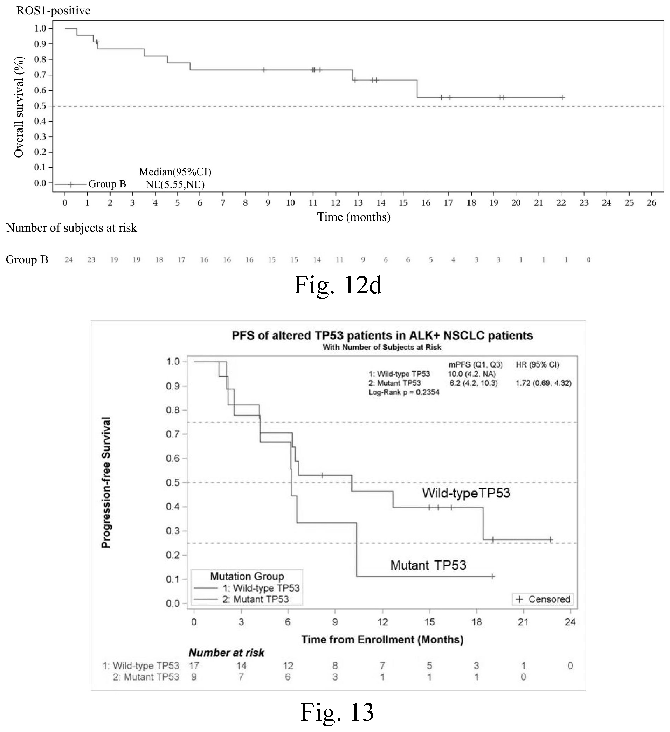

The results of systemic progression-free survival and intracranial progression-free survival in the ALK-positive subjects during the indication expansion phase are shown in Fig. 11 and Fig. 12, respectively.

As of April 17, 2024, based on FAS, the median PFS in the cohort 2 of group A was 6.87 months (95% CI: 4.14, 9.76), and the median PFS in group B was 8.36 months (95% CI: 1.71, -). The results of systemic progression-free survival and overall survival in the ALK-positive subjects in the indication expansion phase are shown in Fig. 11b and Fig. 12b, respectively. The results of systemic progression-free survival and overall survival in the ROS1-positive subjects in the indication expansion phase are shown in Fig. 12c and Fig. 12d, respectively.

③ The overall remission results of the subjects in the cohort 2 of group A, who had previously received a treatment with one, two, three or more second-generation ALK inhibitors, after being administered with the compound A of the present disclosure are shown in Table 9 below.

**Table 9. Analysis of BOR, ORR, DCR, DOR, and TTR in the cohort 2 of group A**

| | | Previously treated with one second-generation ALK inhibitor | | Previously treated with two second-generation ALK inhibitors | | Previously treated with 3 or more second-generation ALK inhibitors | |
|---|---|---|---|---|---|---|---|
| | | No chemotherapy treatment received (N=26) | Chemotherapy treatment received (N=14) | No chemotherapy treatment received | Chemotherapy treatment received | No chemotherapy treatment received (N=7) | Chemotherapy treatment received |
| | | | | (N=24) | (N=15) | | (N=11) |
| BOR, n (%) | | | | | | | |
| | CR | 0 | 0 | 0 | 0 | 0 | 0 |
| | PR | 9 (34.6) | 6 (42.9) | 11 (45.8) | 6 (40.0) | 3 (42.9) | 3 (27.3) |
| | SD | 11 (42.3) | 3 (21.4) | 10 (41.7) | 7 (46.7) | 3 (42.9) | 7 (63.6) |
| | PD | 6 (23.1) | 4 (28.6) | 3 (12.5) | 2 (13.3) | 0 | 1 (9.1) |
| | NE | 0 | 1 (7.1) | 0 | 0 | 1 (14.3) | 0 |
| Confirmed ORR, n (%) (95% CI) | | 9 (34.6) (17.2, 55.7) | 6 (42.9) (17.7, 71.1) | 11 (45.8) (25.6, 67.2) | 6 (40.0) (16.3, 67.7) | 3 (42.9) (9.9, 81.6) | 3 (27.3) (6.0, 61.0) |
| DCR, n (%) (95% CI) | | 20 (76.9) (56.4, 91.0) | 9 (64.3) (35.1, 87.2) | 21 (87.5) (67.6, 97.3) | 13 (86.7) (59.5, 98.3) | 6 (85.7) (42.1, 99.6) | 10 (90.9) (58.7, 99.8) |
| Median DOR, months (95% CI) | | - (2.793, -) | - | 12.48 (2.76, -) | 12.45 (5.39, -) | - (2.66, -) | - (4.14, -) |
| Median TTR, months (range) | | 1.38 (1.28, 6.80) | 2.07 (1.35, 6.90) | 1.38 (1.35, 9.72) | 1.40 (1.31, 2.79) | 1.35 (1.35, 1.45) | 1.35 (1.28, 7.00) |

6) The PK parameters of the compound A at five dose levels (40 mg, 60 mg, 80 mg, 100 mg, 125 mg) after a single or multiple administrations to the subjects are shown in Table 10 below:

**Table 10. PK parameters for the single and multiple oral administrations of the compound A at doses of 40 mg, 60 mg, 80 mg, 100 mg, and 125 mg, and lorlatinib at a dose of 100 mg.**

| PK param eters | Unit | Statist ics | 40 mg | 60 mg | 80 mg | 100 mg | 125 mg | 100 mg of lorlatinib ^{a} |
|---|---|---|---|---|---|---|---|---|
| Single administration | | | | | | | | |
| Cmax | ng/mL | GM (%CV ) [n] | 392(49.0 ) [3]* | 875(18.0) [3] | 524(60.1) [4] | 774(29. 8) [3] | 1261(27. 1) [3] | 907.2(24) [7] |
| Tmax | hour | Media n (min-max) [n] | 0.98(0.5, 1.0) [3] | 0.5(0.5, 0.52) [3] | 0.7(0.47,1. 08) [4] | 1.02(0.5 , 2.0) [3] | 1.02(0.48 , 2.0) [3] | 2.0(0.50,4.02 ) [7] |
| AUC0 -t | h*ng/ mL | GM (%CV ) [n] | 4440(31. 5) [3] | 11310(49.2 ) [3] | 9135(7.2) [4] | 13826(5 4.6) [3] | 17778(15 .6) [3] | / |
| AUC0 -inf | h*ng/ mL | GM (%CV ) [n] | 4710(35. 6) [3] | 12194(50.6 ) [3] | 10100(12.2 ) [4] | 14644(5 6.1) [3] | 18688(18 .7) [3] | 9590(11)[7] |
| T1/2 | hour | GM (%CV ) [n] | 27.8(46. 1) [3] | 33.6(26.6) [3] | 34.2(42.0) [4] | 30.5(20. 1) [3] | 29.2(30.1 ) [3] | 20.0(22)[7] |

| Multiple administrations | | | | | | | | |
|---|---|---|---|---|---|---|---|---|
| Cmax, SS | ng/mL | GM (%CV ) [n] | 387(19.5 ) [7] | 644(30.4) [12] | 589(34.8) [10] | 798(46. 9) [3] | 987(52.1) [3] | 644.8(32)[11] |
| Tmax | hour | Media n (min-max) [n] | 1.0(0.5, 3.07) [7] | 0.533(0.42, 2.02) [12] | 1.56(0.48,3 .02) [10] | 1.97(0.5 8, 2.0) [3] | 0.5(0.48, 1.0) [3] | 2.00(1.00,4.0 0)[11] |
| AUC0 -τ | h*ng/ mL | GM (%CV ) [n] | 3878 (38.6) [7] | 5437 (24.1) [12] | 5497(25.4) [10] | 7021(53 .9) [3] | 7700(23. 5) [3] | 5946(46)[11] |
| AR | NA | GM (%CV ) [n] | 1.044(25 .1) [3] | 0.913(44.1 ) [3] | 1.256(45.9) [4] | 0.732(6 6.4) [3] | 0.606(36. 1) [3] | 0.718(55)[11] |

| | | | | | | | | |
|---|---|---|---|---|---|---|---|---|
| Note: 'a' indicates that the data in this column comes from column 3 of Table 4 on page 1322 of Joseph Chen et al., Pharmacokinetics of Lorlatinib After Single and Multiple Dosing in Patients with Anaplastic Lymphoma Kinase (ALK)-Positive Non-Small Cell Lung Cancer: Results from a Global Phase I/II Study. Clinical Pharmacokinetics (2021) 60:1313-1324 (Asian subjects); *[n] represents n subjects. | | | | | | | | |

7) Adverse events related to the investigational drug during the treatment are shown in Table 11, and adverse events related to the CNS are shown in Table 12.

**Table 11 Centralized summary of TRAE in safety analysis**

| | In total n=198 | | 60mg dose group n=168 | |
|---|---|---|---|---|
| | Number of cases at any level (%) | ≥3, number of cases (%) | Number of cases at any level (%) | ≥3, number of cases (%) |
| Any TRAE | 184 (92.9%) | 73 (36.9%) | 159 (94.6) | 61 (36.3%) |

| TRAE with an incidence of ≥ 5% | | | | |
|---|---|---|---|---|
| Hypercholesterolemia | 154 (77.8) | 18 (9.1) | 131 (78.0) | 17 (10.1) |
| Hypertriglyceridemia | 147 (74.2) | 38 (19.2) | 124 (73.8) | 31 (18.5) |
| Weight gain | 91 (46.0) | 14 (7.1) | 77 (45.8) | 11 (6.5) |
| Increased LDL cholesterol | 51 (25.8) | 0 | 45 (26.8) | 0 |
| Edema | 51(25.8) | 3(1.5) | 45(22.7) | 2 (1.2) |
| Peripheral neuropathy | 38(19.2) | 1(0.5) | 34(20.2) | 1 (0.6) |
| Anemia | 27 (13.6) | 4 (2.0) | 26 (15.5) | 4 (2.4) |
| Hyperuricemia | 27 (13.6) | 4 (2.0) | 26 (15.5) | 4 (2.4) |
| Increased AST | 20 (10.1) | 0 | 18 (10.7) | 0 |
| Elevated alanine aminotransferase | 18 (9.1) | 0 | 14 (8.3) | 0 |
| Increased lipase | 19 (9.6) | 1 (0.5) | 12 (7.1) | 1 (0.6) |
| Increased glutamine transferase | 10 (5.1) | 2 (1.0) | 9 (5.4) | 2 (1.2) |
| Hypoproteinemia | 10 (5.1) | 0 | 10 (6.0) | 0 |
| Positive for occult blood in urine | 13 (6.6) | 0 | 13 (7.7) | 0 |
| Elevated creatinine | 12 (6.1) | 0 | 11 (6.5) | 0 |
| Increased creatine phosphokinase | 11 (5.6) | 0 | 9 (5.4) | 0 |
| Proteinuria | 10 (5.1) | 0 | 7 (7.2) | 0 |
| Diarrhea | 13 (6.6) | 0 | 8 (8.2) | 0 |

**Table 12 CNS-related AE**

| | Any level, number of cases (%) | ≥3, number of cases (%) |
|---|---|---|
| **Any CNS-related AE** | **18 (9.1)** | **2 (1.0)** |
| **Impact on emotion** | **4 (2.0)** | |
| Depression | 3 (1.5) | 0 |
| Anger | 1 (0.5) | 0 |
| **Impact on cognition** | **5 (2.5)** | **1 (0.5)** |
| Memory impairment | 3 (1.5) | 0 |
| Mental disorder | 1 (0.5) | 1 (0.5) |
| Cognitive impairment | 1 (0.5) | 0 |
| **Impact on language** | **2 (1.0)** | **1 (0.5)** |
| Speech disorder | 1 (0.5) | 1 (0.5) |
| Disorganized speech | 1 (0.5) | 0 |
| **Impact on sleep** | **7 (4.5)** | |
| Poor sleep quality | 6 (4.0) | 0 |
| Insomnia | 1 (0.5) | 0 |

A total of 198 patients were included, of whom 184 (92.9%) patients experienced treatment-related adverse events (TRAEs), with hypercholesterolemia, hypertriglyceridemia, or weight gain being the most common. 1.0% of the patients experienced grade ≥ 3 treatment-related central nervous system (CNS) AEs; no dose interruption or dose reduction was observed. No DLT was observed, therefore the MTD was not reached. The compound A was well tolerated, with a low incidence of grade ≥ 3 TRAEs and CNS AEs.

### 8) MTD and RP2D

No dose-limiting toxicity was observed in compound A during the dose escalation phase, and it was also shown to be safe and well-tolerated during the dose expansion and indication expansion phases, indicating that compound A has good safety and tolerability.

Based on the assessments of the data results of PK, safety, and efficacy of the compound A during the dose escalation and dose expansion phases, the incidence of other AE was significantly lower in the 60 mg dose group than that in the 80 mg dose group. Following the discussion and resolution at the SRC meeting, it was decided that the recommended dose for indication expansion was 60 mg.

### Biomarker analysis

26 subjects who had previously received a treatment with at least one ALK inhibitor were included in the biomarker analysis (Table 13), including 24 subjects who had previously received a treatment with a second-generation ALK-TKI and 2 subjects who had experienced progressive disease after a treatment with crizotinib.

Among the 26 subjects, 11 subjects achieved PR. The ORR for the subjects with variant 1 and variant 3 were 33.3% (2/6) and 62.5% (5/8), respectively. The compound A was effective in the subjects with common drug resistance mutations in ALK fusions. The ORR for the subjects with G1202R, V1180L, and L1196M mutations were 50% (3/6), 100% (1/1), and 50% (1/2), respectively. There was no difference in the PFS between the subjects with mutant TP53 and the subjects with wild-type TP53 (P=0.235) (Fig. 13, which shows the PFS of patients with mutant TP53 or wild-type TP53 treated with compound A in the ALK TKI resistance subgroup).

**Table 13 Biomarker analysis of 26 subjects who had previously received a treatment with at least one ALK inhibitor**

| | **Partial response** | **Stable disease** | **Progressive disease** | **Overall response rate (CR + PR)** | **Disease control rate (CR + PR + SD)** |
|---|---|---|---|---|---|
| **ALK fusion** | 11/26 | 9/26 | 6/26 | 11/26 | 20/26 |
| **V1** | **2/6** | 2/6 | 2/6 | 2/6 | 4/6 |
| **V3** | **5/8** | 2/8 | 1/8 | 5/8 | 7/8 |
| V2 | 1/3 | 2/3 | 0/3 | 1/3 | 3/3 |
| V5 | 1/1 | 0/1 | 0/1 | 1/1 | 1/1 |
| others^{†} | 2/8 | 3/8 | 3/8 | 2/8 | 5/8 |
| **ALK mutation** | 5/12 | 5/12 | 2/12 | 5/12 | 10/12 |
| p.L1196M | 1/2 | 1/2 | 0/2 | 1/2 | 2/2 |
| **p.G1202R** | **3/6** | 2/6 | 1/6 | 3/6 | 5/6 |
| p.V1180L | 1/1 | 0/1 | 0/1 | 1/1 | 1/1 |
| p.I1171N | 0/1 | 1/1 | 0/1 | 0/1 | 1/1 |
| p.I1171T | 0/1 | 1/1 | 0/1 | 0/1 | 1/1 |
| p.E1210K p.D1203N | 0/1 | 0/1 | 1/1 | 0/1 | 0/1 |

| **Alterations in the bypass signaling pathways** | | | | | |
|---|---|---|---|---|---|
| **CDKN2A** | 0/1 | 1/1 | 0/1 | 0/1 | 1/1 |
| BRCA1 | 0/1 | 0/1 | 1/1 | 0/1 | 0/1 |
| RB1 | 0/1 | 0/1 | 1/1 | 0/1 | 0/1 |
| **EGFR** | 0/1 | 1/1 | 0/1 | 0/1 | 1/1 |
| IGF1R | 0/1 | 0/1 | 1/1 | 0/1 | 0/1 |
| CCNE1 | 1/1 | 0/1 | 0/1 | 1/1 | 1/1 |
| BRCA2 | 1/1 | 0/1 | 0/1 | 1/1 | 1/1 |
| MET | 1/2 | 1/2 | 0/2 | 1/2 | 2/2 |
| KIT | 1/1 | 0/1 | 0/1 | 1/1 | 1/1 |
| **TP53** | **4/9** | 3/9 | 2/9 | 4/9 | 7/9 |

Example 5: A phase II, multicenter, single-arm clinical trial evaluating the efficacy and safety of monotherapy with compound A in the patients with advanced ALK-positive non-small cell lung cancer who had failed a treatment with a second-generation ALK inhibitor

### Primary purpose and primary estimated target:

### Primary purpose:

To evaluate the clinical efficacy of the monotherapy with compound A in patients with ALK-positive advanced NSCLC who had previously experienced progressive disease or intolerance after a continuous treatment with a second-generation ALK inhibitor using the objective response rate (ORR) as the endpoint, as assessed by an independent review committee (IRC).

### Primary estimated target:

Target population: the ALK-positive advanced NSCLC patients who had previously experienced progressive disease or intolerance after a continuous treatment with a second-generation ALK inhibitor;
treatment: 60 mg once daily (QD), for 3 weeks as an administration cycle.

Variable of the research: whether the subjects achieved objective response (CR/PR) during the treatment, as assessed by the independent review committee (IRC).

Group-level summary: ORR during the treatment.

### Secondary purpose:

To evaluate the clinical efficacy of the monotherapy with compound A in patients with ALK-positive advanced NSCLC using other efficacy endpoints;
to evaluate the safety of the compound A in the treatment of patients with ALK-positive advanced NSCLC;
to evaluate the population pharmacokinetic (PK) characteristics of the compound A in ALK-positive advanced NSCLC patients;
to evaluate the effect of the compound A on the PK characteristics of simvastatin tablets in ALK-positive advanced NSCLC patients.

### Research design

The research was divided into four phases: screening period, treatment period, safety follow-up period, and survival follow-up period.

### Screening period

After signing the informed consent form, the subjects entered the screening period, which lasted from day -28 to day -1. The subjects participated in the DDI study were screened from day -28 to day -4. All the subjects were required to complete the screening period-related examinations or observations before the enrollment to determine their disease status, treatment history, etc. The relevant examinations included vital signs, physical examination, laboratory tests, pregnancy tests, ECOG score, tumor imaging evaluation, electrocardiogram, echocardiography, etc. Among these, the complete blood count, blood biochemistry, coagulation function, urinalysis, 12-lead electrocardiogram, and pregnancy tests during the screening period must be completed within one week before the first administration of the investigational drug.

### Treatment period

The subjects meeting the screening criteria entered the treatment period. During the treatment period, all the subjects received a long-term treatment with the administration regimen of compound A, which was administered orally on an empty stomach, 60 mg once daily (QD), for 3 weeks as one administration cycle, until the occurrence of progressive disease, intolerable toxicity, death, withdrawal of the informed consent, or termination of the research as determined by the IRC, whichever occurred first. During the treatment period (starting from C1D1), all the subjects underwent imaging examinations and antitumor efficacy assessments every 6/12 weeks (every 6 weeks for C1 to C17, and every 12 weeks thereafter). During the treatment period, all the subjects underwent laboratory tests, vital signs, and physical examinations, etc. every 6/12 weeks (every 6 weeks for C1 to C17, and every 12 weeks thereafter) to assess the safety of the treatment. During the medication, the investigators might add additional examinations for the subjects based on their clinical symptoms or status. Disease status was assessed by the investigators according to the Response Evaluation Criteria in Solid Tumors (RECIST version 1.1), and AE was evaluated and recorded according to CTCAE 5.0.

In addition, approximately 12 subjects were selected for the DDI assessment of the effect of the compound A on simvastatin. The subjects received a single oral dose of 40 mg of simvastatin tablet on an empty stomach during cycle 1 (C1)D-2 (C1D-2), and PK sampling was performed up to 32 h after the administration (C1D-1). The subjects began taking compound A at C1D1 according to the administration regimen developed during the treatment period. On C1D15, after an oral administration of compound A on an empty stomach, subjects simultaneously received a single oral dose of 40 mg of simvastatin tablet, and PK sampling was performed up to 32 h after the administration (C1D16). The subjects continued taking the compound A according to the administration regimen developed during the treatment period until the occurrence of progressive disease, intolerable toxicity, death, withdrawal of the informed consent, or termination of the research as determined by the IRC, whichever occurred first.

In this research, a population PK study was conducted to investigate the PK characteristics and potential influencing factors in the ALK-positive NSCLC patients.

An independent review committee (IRC) was established in this research. The IRC evaluated all the tumor imaging data to determine the objective response and tumor progression. After the investigator determined progressive disease in a subject, the IRC confirmed the subject's tumor imaging data. If the IRC assessment indicated progressive disease in the subject, the treatment would be terminated on the subject. If the investigator determined progressive disease, but the IRC determined that progressive disease had not been achieved, the sponsors and investigators needed to discuss and reach a consensus on whether the subject should continue to receive the study treatment from a perspective of risk and benefit of the subject. If the subject continued to receive the study treatment, the investigators continued the tumor assessment according to the protocol (and unplanned tumor assessments might be added when there was a clinical indication) until the IRC's assessment indicated that the patient had progressive disease. The results of the IRC assessment were used for the analysis of the efficacy indicators, and the results of the IRC assessment were also used to guide the treatment decisions for the subjects.

A subject was treated until the subject met the criteria for the discontinuation of the treatment/withdrawal from the treatment, at which point the study treatment was terminated on the subject. The subject must complete the last treatment visit within 7 days from the termination of the study treatment, after which the subject entered the safety follow-up period. If the IRC assessed that a subject had progressive disease and the investigators determined that continued use of the investigational drug would benefit the subject based on the subject's condition, the subject might continue the trial treatment after a discussion with the sponsors. If any adverse event occurred or any concomitant medication or treatment was used during the continued medication, the subject must report it to the investigators immediately. If necessary, the subject might come to the hospital for necessary examinations or treatments. The investigators followed up based on the subject's disease status.

### Safety follow-up period

Once a subject met the criteria for the discontinuation of the treatment/withdrawal from the treatment and discontinued the medication, the subject entered a 4-week safety follow-up period. If other anti-tumor therapies were used during the safety follow-up period, a safety follow-up visit and inspection must be completed before starting the other anti-tumor therapies. For the unresolved adverse event or abnormal laboratory test, the follow-up continued until the event was resolved, the subject returned to the baseline status, the condition stabilized, a reasonable explanation was obtained, or the subject was lost to follow-up or died.

### Survival follow-up period

The survival follow-up (by telephone) was conducted every 3 months ± 7 days starting from the last study administration to collect all the subsequent anti-tumor treatments and patient survival status until the end of the trial, the achievement of survival, or refusal of follow-up, whichever occurred first. Unless a subject discontinued the medication or started a new anti-tumor therapy due to the progressive disease or death, the subject must come to the hospital every 12 weeks (calculated from the first administration) for tumor imaging examination and efficacy assessment examination according to the RECIST version 1.1 during the long-term follow-up period, as originally scheduled, until the progressive disease, start of a new anti-tumor therapy, death, refusing to come to the hospital for a follow-up, or end of the trial. The IRC evaluated the tumor imaging data to determine the tumor progression.

### Selection criteria:

Patients meeting all of the following criteria were considered for enrollment:
1) agreeing to follow the treatment protocol and visitation plan of the trial, voluntarily enrolled, and having signed a written informed consent form;
2) age ≥ 18 years old on the day of signing the informed consent form, regardless of gender;
3) the patients with ALK-positive advanced (defined as stage IIIB, IIIC, or IV) inoperable NSCLC who had experienced progressive disease or intolerance after a treatment with a second-generation ALK inhibitor (regardless of whether they had previously used crizotinib, those using a second-generation drug in the trial phase were also eligible for enrollment);
4) during the screening, a previous test result of a subject was acceptable (a test report confirming ALK positivity using the nationally approved Ventana D5F3 immunohistochemistry, FISH, PCR, or next-generation sequencing (NGS)); if there was no previous test report, a tissue sample must be provided for the confirmation of ALK gene; a fresh tissue sample or archived paraffin-embedded tumor tissue block or pathological biopsy section within the past 6 months should be obtained whenever possible, but the availability of the sample did not affect the enrollment;
5) the patients with central nervous system metastases at the time of screening who met the following criteria were eligible for the enrollment of this trial: a. asymptomatic: not requiring corticosteroid treatment, or the dose of prednisone/equivalent had been reduced to ≤ 10 mg QD and the treatment was maintained stable for at least 2 weeks; or b. for the patients who had previously received a treatment for the central nervous system metastases (radiotherapy or surgery), the acute effects of radiotherapy or surgery had been fully recovered before the first administration, and the corticosteroid treatment for the metastases had been discontinued for at least 4 weeks, with stable neurological symptoms and signs at the same time;
6) before the first administration of the investigational drug, the patients who had previously received a treatment with an ALK inhibitor must had discontinued the drug for ≥ 5 half-lives;
7) according to the criteria in RECIST version 1.1, there must be at least one measurable lesion; the lesion which had been previously treated with radiotherapy could not be considered as a target lesion unless the lesion had significantly progressed;
8) eastern Cooperative Oncology Group (ECOG) Performance Status (PS): score 0 to 2; (appendix 2);
9) adequate bone marrow, liver, kidney, coagulation, and pancreatic function (referring to the upper limit of normal at each clinical trial center):
   bone marrow (no blood transfusions or use of corrective therapy with cytokine drugs such as granulocyte colony-stimulating factor (G-CSF) within 2 weeks prior to the examination):
   absolute neutrophil count (ANC) ≥ 1.5 × 10⁹/L;
   platelet count ≥ 100 × 10⁹/L;
   hemoglobin ≥ 90 g/L;
   liver function:
   serum total bilirubin ≤ 1.5 times the upper limit of normal (ULN);
   in the absence of a liver metastasis, alanine aminotransferase (ALT), aspartate aminotransferase (AST), and alkaline phosphatase (ALP) ≤ 2.5 times the ULN; in the presence of a liver metastasis, ALT, AST, and ALP ≤ 5 times the ULN;
   kidney function: serum creatinine ≤ 1.5 times the ULN;
   coagulation function: international normalized ratio (INR) ≤ 1.5 × ULN, the activated partial thromboplastin time (APTT) ≤ 1.5 × ULN;
   pancreatic function:
   serum total amylase ≤ 1.5 times the ULN;
   serum lipase ≤ 1.5 times the ULN;
10) expected survival ≥ 3 months;
11) men with fertility and women of childbearing age were willing to take effective contraception from the date of signing the informed consent form until 6 months after the last administration of the investigational drug; the women of childbearing age included the premenopausal women and women within two years of menopause.

### Exclusion criteria:

Patients meeting any one of the following criteria were ineligible for enrollment:
1) subjects who had previously received a third-generation ALK inhibitor other than the compound A;
2) subjects with a known allergy to any active ingredient or excipient of compound A, or a history of specific allergic reactions (asthma, rheumatism, eczema), or a history of other severe allergic reactions that the investigator determined were unsuitable for the treatment with compound A;
3) subjects with another malignant tumor other than lung cancer; except for the cervical cancer in situ and non-melanoma skin cancer that had received curative treatment and had not recurred within 5 years;
4) subjects who had undergone a major surgery within 4 weeks prior to the first administration; minor surgical procedures (e.g., port implantation) were not exclusion criteria, but sufficient time was required for adequate wound healing;
5) spinal cord compression (caused by a tumor), unless the subject had achieved good pain control through treatment and neurological function had fully recovered within 4 weeks prior to the first administration;
6) gastrointestinal dysfunction, primarily including inability to take oral medications, need for intravenous nutrition, previous surgical procedures (including total gastrectomy or gastric banding surgery) affecting the absorption, active inflammatory gastrointestinal disease, chronic diarrhea, symptomatic diverticulosis, treatment of active peptic ulcer disease within the past 6 months, malabsorption syndrome, etc.;
7) patients with a history of active pneumonia or clinically significant interstitial lung disease, or patients with a radiation or drug-induced pulmonary disease requiring treatment; the patients with radiation pneumonitis who were 3 months post-radiotherapy and had no clinical symptoms could be enrolled;
8) patients with cardiac dysfunction, including but not limited to those with a left ventricular ejection fraction (LVEF) <50%, a history of congestive heart failure or a history of ventricular arrhythmias requiring treatment, hypokalemia, hereditary long QT syndrome, or a history of myocardial infarction or unstable angina within 6 months prior to the screening; patients with heart failure classified by the New York Heart Association as class ≥2;
9) patients with clinically significant abnormalities in QTc in the electrocardiogram (ECG) examination (QTc >450 msec [male] or QTc >470 msec [female] obtained in the ECG examination at rest), or those requiring concomitant use of any medication known to prolong the QT interval and cause torsades de pointes ventricular tachycardia (such medications must also be discontinued before the first administration; see Appendix 6 for specific medications and requirements for the time of discontinuation);
10) patients with uncontrolled hypertension (systolic blood pressure ≥ 160 mmHg or diastolic blood pressure ≥ 100 mmHg) after a drug treatment;
11) patients with uncontrolled hyperglycemia, or acute cholelithiasis, or those characterized by the susceptibility to acute pancreatitis;
12) patients with a severe or uncontrollable systemic disease (such as an unstable or uncompensated respiratory, cardiac, hepatic, or renal disease) who were expected to be unable to tolerate the investigational drug treatment, based on the investigators' judgment;
13) patients who had used the following medications within 14 days prior to the first administration or needed to use the following medications during the treatment: medications that pose a risk of QTc interval prolongation and/or ventricular tachycardia;
14) patients with previous antitumor therapy: (1) patients who had received any systemic antitumor therapy within 28 days prior to the first administration of the investigational drug (if the drug had been discontinued for more than 5 half-lives and the compound A could be used within 28 days after the end of the systemic antitumor therapy as assessed by the investigators, the patients could be enrolled into the trial), including systemic chemotherapy, immunotherapy (physiological replacement dose of glucocorticoids [prednisone or equivalent <10 [mg/day] were allowed to use), large molecule monoclonal antibodies, small molecule targeted drugs, etc.; (2) patients who had received radiotherapy or Chinese herbal medicine or proprietary Chinese medicine approved for anti-tumor treatment within 14 days prior to the first administration; (3) patients who had received an antibody or drug treatment targeting T cell co-stimulation or immune checkpoint pathway within the past 28 days, including but not limited to anti-PD-1, anti-PD-L1, anti-PD-L2, anti-CD137 or anti-CTLA-4 antibodies;
15) subjects who had not recovered from a previous surgical procedure and a previous anti-tumor treatment-related toxic reaction, which were assessed by the investigators as affecting the safety of the subjects;
16) patients with clinically significant active bacterial, fungal or viral infection, including those who were positive for hepatitis B surface antigen and had HBV DNA ≥ ULN (ULN was defined as 2000 IU/ml)(only in the patients who were positive for hepatitis B surface antigen), or those who were positive in the test results of any one or more of the following: hepatitis C (those who were positive for hepatitis C antibody but had the HCV RNA polymerase chain reaction (PCR) < ULN were eligible for enrollment) or human immunodeficiency virus antibody test, or those with any uncontrolled infection;
17) patients who had taken a strong inducer or inhibitor of CYP3A4, or a CYP3A4 substrate with a narrow therapeutic window, within two weeks prior to the first administration of the investigational drug;
18) pregnant or breastfeeding female subjects;
19) women of childbearing age who were unwilling or unable to use an acceptable method of contraception throughout the entire treatment period of this trial and for 6 months after the last administration of the investigational drug (women of childbearing age included: any woman who had had menarche and had not undergone successful artificial sterilization surgery (hysterectomy, bilateral tubal ligation, or bilateral oophorectomy) or was not menopausal); and fertile male patients who were unwilling or unable to use effective contraception if their partners were women of childbearing age;
20) subjects who were participating in other clinical researches (excluding the non-intervention phase of the interventional clinical researches, such as the survival follow-up period) or whose first administration was less than 4 weeks or 5 half-lives of the investigational drug from the end of receiving other investigational drugs, whichever was shorter;
21) patients with any mental or cognitive impairment that might limit their understanding and compliance with the informed consent form; other situations deemed unsuitable for the participation of this research by the investigators, such as poor compliance;

### Trial results:

A total of 204 subjects were screened in this trial, of which 164 subjects were successful in the screening and enrolled. 163 (99.4%) subjects received the treatment with compound A, and 40 subjects failed in the screening. The main reason for the failure in screening was not meeting the selection criteria or meeting the exclusion criteria (37 subjects, 18.1%).

### ① Demographic data

Based on the full analysis set meeting the key selection and exclusion criteria, among the 158 subjects with a history of anti-NSCLC drug treatment, the pathological classification was adenocarcinoma in 151 subjects (95.6%), squamous cell carcinoma in 3 subjects (1.9%), other types of carcinoma in 3 subjects (1.9%), and large-cell carcinoma in 1 subject (0.6%). Among all the subjects at enrollment, 2 subjects (1.3%) had tumors at clinical stage of III B, 3 subjects (1.9%) had tumors at stage of III C, 151 subjects (95.6%) had tumors at stage of IV, and 2 subjects had tumors at other stages. All the subjects were ALK-positive. 153 subjects (96.8%) had metastases at enrollment, at the following metastatic sites: intracranial metastases in 86 subjects (56.2%), bone metastases in 73 subjects (47.7%), liver metastases in 28 subjects (18.3%), adrenal gland metastases in 20 subjects (13.1%), and metastases at other sites in 130 subjects (85.0%). 28 subjects (17.7%) received an ECOGPS score of 0, 127 subjects (80.4%) received a score of 1, and 3 subjects (1.9%) received a score of 2.

The therapy types for the 158 subjects were as follows: targeted therapy in 158 subjects (100.0%), other therapies in 55 subjects (34.8%, mainly including chemotherapy combined with targeted therapy and/or immunotherapy, chemotherapy combined with targeted therapy and proprietary Chinese medicine treatment, targeted therapy combined with proprietary Chinese medicine treatment, traditional Chinese medicine or proprietary Chinese medicine treatment and adjuvant therapy), and chemotherapy in 43 subjects (27.2%). Classified by the alternative name, the most commonly used antitumor drugs were alectinib (89 subjects, 56.3%), crizotinib (55 subjects, 34.8%), and pemetrexed (49 subjects, 31.0%). The usage of second-generation ALK inhibitors was as follows: alectinib in 89 subjects (56.3%), ensartinib in 39 subjects (24.7%), ceritinib in 20 subjects (12.7%), and brigatinib in 7 subjects (4.4%). The usage of the second-generation ALK inhibitors in clinical trials was as follows: XZP-3621 tablet in 10 subjects (6.3%), TQ-B3139 capsule in 10 subjects (6.3%), and WX-0593 tablet in 7 subjects (4.4%).

Among them, XZP-3621 was an ALK inhibitor independently developed by Xuanzhu Biopharmaceutical for the treatment of non-small cell lung cancer, and it was currently in Phase III clinical trials; TQ-B3139 was also known as envonalkib, and WX-0593 was also known as iruplinalkib, and their structures are shown below:

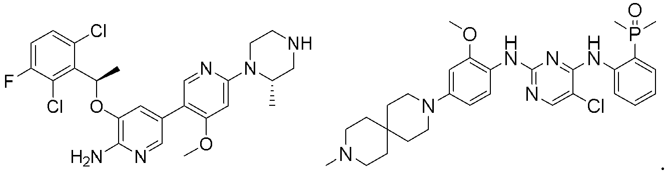

### ② Efficacy results

### a) Therapeutic effects on the whole-body tumors

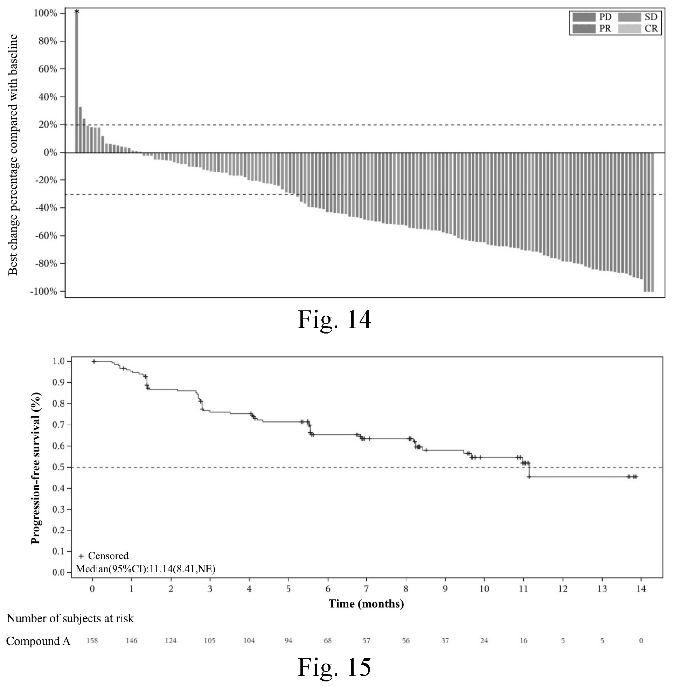

As of the data cutoff date (May 20, 2024), based on the full analysis set of 158 subjects, the best objective response of a total of 67 subjects (42.4%) achieved PR as assessed by the IRC, and the ORR was 67 subjects (42.4%, 95% CI: 34.59, 50.51). The median DOR was not yet reached, the median TTR was 1.38 months (95% CI: 1.38, 1.41), and the median PFS was 11.14 months (95% CI: 8.41, -). The detailed analyses of the whole-body BOR, ORR, and DCR of the subjects assessed by the IRC are shown in Table 14. The waterfall chart of the best percentage change in the sum of the whole-body tumor diameters relative to the baseline is shown in Fig. 14, and the Kaplan-Meier curve (survival curve) of PFS is shown in Fig. 15.

**Table 14 The analysis of whole-body BOR, ORR and DCR (IRC assessment results) - full analysis set**

| | In total (N=158) |
|---|---|
| BOR, n (%) | |
| CR | 0 |
| PR | 67 (42.4) |
| SD | 64 (40.5) |
| PD | 25 (15.8) |
| NE | 2 (1.3) |
| ORR, n (%) | 67 (42.4) |
| 95% CI^{[1]} | 34.59, 50.51 |
| DCR, n (%) | 131 (82.9) |
| 95% CI^{[1]} | 76.12, 88.43 |

The above results indicated that the compound A showed good efficacy in the subjects who had failed the treatment with a second-generation ALK-TKI, with a shorter time achieving tumor remission, can effectively control the disease progression, and was expected to prolong the progression-free survival of the subjects.

### b) Therapeutic effects on the intracranial tumors

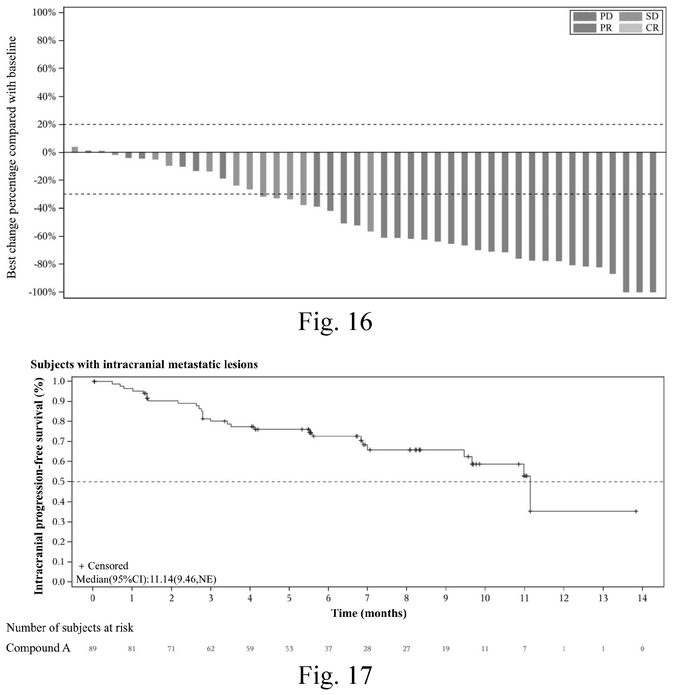

As of the data cutoff date (May 20, 2024), based on the full analysis set of 158 subjects, 44 subjects had intracranial measurable lesions. No subjects had a best objective response achieved CR according to the IRC assessment, and 24 subjects (54.5%) achieved PR. The IC-ORR of them was 24 subjects (54.5%, 95% CI: 38.85, 69.61), and the IC-DCR of them was 37 subjects (84.1%, 95% CI: 69.93, 93.36). The median IC-DOR was 9.76 months (95% CI: 8.28, -), the median IC-TTR was 1.41 months (95% CI: 1.35, 1.41), and the median IC-PFS was 9.66 months (95% CI: 6.83, -). For detailed analysis of the subjects' IC-BOR, IC-ORR and IC-DCR, see Table 15. The waterfall chart of the changes in the sum of intracranial tumor diameters relative to the baseline is shown in Fig. 16. The Kaplan-Meier curve of IC-PFS is shown in Fig. 17.

**Table 15 The analysis of intracranial BOR, ORR and DCR (IRC assessment results) - full analysis set**

| | | | In total (N=158) |
|---|---|---|---|
| Subjects with intracranial measurable target lesions | | | 44 |
| | BOR, n (%) | | |
| | | CR | 0 |
| | | PR | 24 (54.5) |
| | | SD | 13 (29.5) |
| | | PD | 7 (15.9) |
| | | NE | 0 |
| | IC-ORR, n (%) | | 24 (54.5) |
| | | 95% CI | 38.85, 69.61 |
| | IC-DCR, n (%) | | 37 (84.1) |
| | | 95% CI | 69.93, 93.36 |

The above results indicated that the compound A showed good efficacy in the subjects who had failed the treatment with a second-generation ALK-TKI and had intracranial target lesions, with a shorter time achieving tumor remission, and can effectively control the intracranial disease progression.

### c) ORR of the subjects in the "efficacy subgroup"

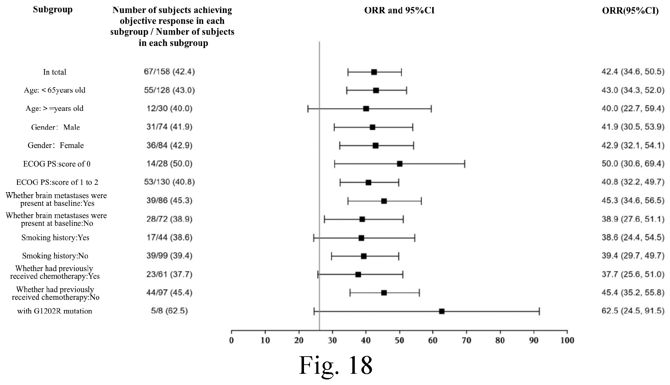

The ORR of subjects assessed by the IRC based on FAS in each subgroup is shown in Fig. 18. The data showed that there was essentially no difference in the ORR of the subjects among the following subgroups: age < or ≥ 65 years old (ORR: 43.0% vs. 40.0%), male or female (ORR: 41.9% vs. 42.9%), and with or without a smoking history (ORR: 38.6% vs. 39.4%). However, the ORR of the subjects with an ECOGPS score of 0 at baseline was slightly higher than that of the subjects with an ECOG PS score of 1 to 2 at baseline (50.0% vs. 40.8%), the ORR of the subjects with brain metastases at baseline was slightly higher than that of the subjects without brain metastases at baseline (45.3% vs. 38.9%), and the ORR of the subjects who had never received chemotherapy was slightly higher than that of the subjects who had previously received chemotherapy (45.4% vs. 37.7%). The ORR in the subjects with G1202R mutation was 62.5% (95% CI: 24.5%, 91.5%).

The subgroup analysis results showed that the compound A had a better efficacy in the subjects with an ECOGPS score of 0 at baseline, with brain metastases at baseline, and without prior chemotherapy; it also showed significant efficacy in the subjects with G1202R mutation, and the ORR can be up to 62.5%.

### ③ Safety analysis

### The common TRAEs of compound A were hypercholesterolemia, hypertriglyceridemia, weight gain, anemia, and peripheral edema. The TRAEs of the most subjects were grade 1 to 2, and the incidence of TRAEs leading to dose reduction, temporary discontinuation of medication, or permanent discontinuation of medication was low. For the subjects with grade 3 or higher level TRAEs, most could recover through measures such as temporarily suspending the administration, reducing the dose, or symptomatic treatment. The adverse event classification was similar to that of the similar drugs. The safety risks were manageable, and the overall safety was good.

Example 6: A multicenter, randomized controlled, open-label phase III research comparing the efficacy and safety of compound A with crizotinib in the treatment of patients with ALK-positive advanced or metastatic non-small cell lung cancer (NSCLC)

This trial was a multicenter, randomized, open-label, positive-controlled phase III clinical trial primarily evaluating the clinical efficacy and safety of the compound A versus crizotinib in the treatment of patients with advanced or metastatic ALK-positive NSCLC. The primary purpose was to compare the clinical efficacy of the compound A tablets versus the crizotinib capsules in the treatment of patients with ALK-positive advanced or metastatic NSCLC, using the progression-free survival (PFS) as the endpoint as assessed by an independent review committee (IRC). The secondary purposes were evaluating the clinical efficacy of the compound A tablets versus the crizotinib capsules in the treatment of patients with ALK-positive advanced or metastatic NSCLC using other efficacy endpoints, and comparing the safety of the compound A tablets versus the crizotinib capsules in the treatment of patients with ALK-positive advanced or metastatic NSCLC.

This research was divided into four phases: screening period, treatment period, safety follow-up period, and survival follow-up period.

### Screening period

After signing the informed consent form, the subjects entered the screening period, which lasted from day -28 to day -1. All the subjects were required to complete the screening period-related examinations or observations before the enrollment to determine their disease status, treatment history, etc. The relevant examinations included vital signs, physical examination, laboratory tests, pregnancy tests, ECOG score, tumor imaging evaluation, electrocardiogram, echocardiography, etc. Among these, the complete blood count, blood biochemistry, coagulation function, urinalysis, 12-lead electrocardiogram, and pregnancy tests during the screening period must be completed within one week before the first administration of the investigational drug.

### Treatment period

The subjects who met the screening criteria were randomly assigned to the assay group (compound A) and the control group (crizotinib), with an assay group:control group ratio of 2:1. This research employed stratified randomization, stratified based on the factors of the presence of central nervous system metastases at baseline and whether chemotherapy had been previously used for advanced NSCLC. All the subjects in the assay group received a long-term treatment with the administration regimen of compound A, which was administered orally on an empty stomach, 60 mg once daily (QD) for 28 days as one administration cycle. The subjects in the control group received crizotinib, a positive control drug, at 250 mg twice daily (BID) for 28 days as one administration cycle. All the subjects received the medication treatment according to their randomized groups until the occurrence of progressive disease, intolerable toxicity, death, withdrawal of the informed consent, or termination of the research as determined by the IRC, whichever occurred first.

During the treatment period (starting from C1D1), all the subjects underwent imaging examinations and antitumor efficacy assessments every 8/12 weeks (every 8 weeks for C1 to C17, and every 12 weeks thereafter). During the treatment period, all the subjects underwent laboratory tests, vital signs, and physical examinations, etc. every 4/8/12 weeks (every 4 weeks for C1 to C3, every 8 weeks for C4 to C17, and every 12 weeks thereafter) to assess the safety of the treatment. During the medication, the investigators might add additional safety assessment examinations for the subjects based on their clinical symptoms or status. Disease status was assessed by the investigators according to the Response Evaluation Criteria in Solid Tumors (RECIST v1.1), and AE was evaluated and recorded according to CTCAE v5.0. A population PK study and exposure-effect relationship study of the compound A were conducted in all the subjects of the assay group to explore the population pharmacokinetic characteristics and potential influencing factors of the compound A in the ALK-positive NSCLC subjects.

For a subject in the crizotinib group who was assessed by the IRC as having progressive disease, after the discontinuation of crizotinib (cross-over was not allowed for the subjects who received other anti-tumor treatments after the discontinuation of crizotinib), cross-over to the compound A group for the medication treatment was permitted with the investigators' assessment and the subject's consent, and the subject was followed up for clinical efficacy and safety. Cross-over from the compound A group to the crizotinib group was not permitted. For the subject crossing over to the compound A for medication, the imaging assessment result indicating a progressive disease according to the IRC assessment was used as the baseline for the crossover treatment period. After starting to take compound A tablets, the subject underwent imaging examinations and antitumor efficacy assessments every 8/12 weeks (every 8 weeks for C1 to C17, and every 12 weeks thereafter), and underwent laboratory tests, vital signs, and physical examinations, etc. every 4/8/12 weeks (every 4 weeks for C1 to C3, every 8 weeks for C4 to C17, and every 12 weeks thereafter) to assess the safety of the treatment. The investigators might add additional safety assessment examinations for the subject based on the subject's clinical symptoms or status until the occurrence of progressive disease, intolerable toxicity, death, withdrawal of the informed consent, or termination of the research, whichever occurred first.

An IRC was established in this research. The IRC evaluated all the tumor imaging data during the randomization phase to determine the objective response and tumor progression. After the investigator determined progressive disease in a subject, the IRC confirmed the subject's tumor imaging data. If the investigator determined progressive disease, but the IRC determined that progressive disease had not been achieved, the subject continued to receive the study treatment, and the investigators continued the tumor assessment according to the protocol (and unplanned tumor assessments might be added when there was a clinical indication) until the IRC's assessment indicated that the subject had progressive disease. The results of the IRC assessment were used for the analysis of efficacy indicators, and the results of the IRC assessment were also used to guide the treatment decisions for the subjects.

A subject was treated until the subject met the criteria for the discontinuation of the treatment/withdrawal, at which point the study treatment was terminated on the subject. The subject must complete the last treatment visit within 7 days from the termination of the study treatment, after which the subject entered the safety follow-up period. If the IRC assessed that a subject had progressive disease and the investigators determined that continued use of the investigational drug would benefit the subject based on the subject's condition, the subject might continue the trial treatment after a discussion with the sponsors. Follow-up visits were conducted according to the previous visit schedule during the continued medication period. If any adverse event occurred or any concomitant medication or treatment was used during the medication, the subject must report it to the investigators immediately. If necessary, the subject might come to the hospital for necessary examinations or treatments. The investigators followed up based on the subject's disease status.

### Safety follow-up period

Once the subjects met the criteria for the discontinuation of the treatment/withdrawal and discontinued the medication, they entered a 4 weeks ± 7 days safety follow-up period, starting from the last study administration. If other anti-tumor therapies were used during the safety follow-up period, a safety follow-up visit and inspection must be completed before starting the other anti-tumor therapies. For the unresolved adverse event or abnormal laboratory test, the follow-up continued until the event was resolved, the subject returned to the baseline status, the condition stabilized, a reasonable explanation was obtained, or the subject was lost to follow-up or died.

### Survival follow-up period

Subjects withdrew from the treatment due to progressive disease. The survival follow-up (by telephone) was conducted every 12 weeks (±14 days) starting from the last study administration to collect all the subsequent anti-tumor treatments and subject survival data until the end of the trial, the achievement of survival, or refusal of follow-up, whichever occurred first.

Unless a subject discontinued the medication due to the progressive disease or death, the subject must come to the hospital every 12 weeks (±14 days) (calculated from the first administration) for tumor imaging examination and efficacy assessment examination according to the RECIST version 1.1 during the long-term follow-up period, as originally scheduled, until the progressive disease, death, refusing to come to the hospital for a follow-up, or end of the trial.

Patients meeting all of the following criteria were considered for enrollment:
1) agreeing to follow the treatment protocol and visitation plan of the trial, voluntarily enrolled, and having signed a written informed consent form;
2) age ≥ 18 years old on the day of signing the informed consent form, regardless of gender;
3) patients who were definitely diagnosed with stage IIIB-IV ALK-positive NSCLC by histology or cytology and were not eligible for curative treatment (surgery, radiotherapy) (International Association for the Study of Lung Cancer 8th edition lung cancer staging).
4) during the screening, a previous test result of a subject in the local area was acceptable (nationally approved Ventana D5F3 immunohistochemistry, FISH, PCR, or next-generation sequencing (NGS)); if there was no previous test report, a tissue sample must be provided for the central confirmation of ALK gene before randomization;
5) patients who had not previously received a treatment with an ALK inhibitor; or who had experienced progressive disease or intolerance after receiving at most a first-line chemotherapy regimen;
6) patients with asymptomatic brain metastases at the time of screening could be enrolled, with untreated brain metastases, and no need to use the corticosteroids; if a patient had neurological symptoms or signs due to CNS metastases, the patient needed to complete the whole brain radiotherapy or Gamma Knife treatment at least 14 days before the first administration and achieve symptom stability (using the corticosteroid at a fixed dose or tapering dose at baseline);
7) according to the criteria in RECIST version 1.1, there must be at least one measurable target lesion (the longest diameter of a non-lymph node ≥10 mm, the shortest diameter of a lymph node ≥15 mm); the lesion which had been previously treated with radiotherapy could not be considered as a target lesion unless the lesion had significantly progressed;
8) eastern Cooperative Oncology Group (ECOG) Performance Status (PS): score 0 to 2;
9) adequate bone marrow, liver, kidney, coagulation, and pancreatic function:
   bone marrow (no blood transfusions or use of corrective therapy with cytokine drugs such as granulocyte colony-stimulating factor (G-CSF) within 2 weeks prior to the screening examination):
   absolute neutrophil count (ANC) ≥ 1.5 × 10⁹/L;
   platelet count ≥ 100 × 10⁹/L;
   hemoglobin ≥ 90 g/L;
   liver function:
   serum total bilirubin ≤ 1.5 times the upper limit of normal (ULN);
   in the absence of a liver metastasis, alanine aminotransferase (ALT), aspartate aminotransferase (AST), or alkaline phosphatase (ALP) ≤ 2.5 times the ULN; in the presence of a liver metastasis, ALT, AST, or ALP ≤ 5 times the ULN;
   kidney function: serum creatinine ≤ 1.5 times the ULN;
   coagulation function: international normalized ratio (INR) ≤ 1.5 × ULN, the activated partial thromboplastin time (APTT) ≤ 1.5 × ULN;
   pancreatic function:
   serum total amylase ≤ 1.5 times the ULN;
   serum lipase ≤ 1.5 times the ULN;
10) expected survival ≥ 3 months;
11) men with fertility and women of childbearing age were willing to take effective contraception from the date of signing the informed consent form until 6 months after the last administration of the investigational drug; the women of childbearing age included the premenopausal women and women within one year of menopause. The result of a pregnancy test conducted ≤7 days prior to the first administration of the investigational drug must be negative for the women of childbearing age.

Patients meeting any one of the following criteria were ineligible for enrollment:
1) subjects with a known allergy to any active ingredient or excipient of compound A and a crizotinib capsule, or a clear history of specific allergic reactions (asthma, rheumatism, eczema), or a history of other severe allergic reactions;
2) subjects with another malignant tumor other than lung cancer in the past 5 years (except for the basal cell carcinoma of the skin, or the cervical carcinoma in situ that had been treated with a curative treatment, or other cancers that were curable and did not affect the current PFS and OS of NSCLC);
3) subjects who had received radiotherapy within 14 days prior to the first administration;
4) subjects who had received other systemic antitumor drug treatment within 4 weeks prior to the first administration, or were still within 5 half-lives of the drug; subjects who had received Chinese herbal medicine or proprietary Chinese medicine treatment for antitumor treatment within 14 days prior to the first administration;
5) subjects who had undergone a major surgery therapy within 4 weeks prior to the first administration;
6) spinal cord compression (caused by a tumor), unless the subject had achieved good pain control through treatment and neurological function had fully recovered within 4 weeks prior to the first administration;
7) gastrointestinal dysfunction, including inability to take oral medications, need for intravenous nutrition, previous surgical procedures (including total gastrectomy or gastric banding surgery) affecting the absorption, active inflammatory gastrointestinal disease, chronic diarrhea, symptomatic diverticulosis, active peptic ulcer requiring a treatment within the past 6 months, malabsorption syndrome, etc.;
8) patients with a history of active pneumonia or clinically significant interstitial lung disease, or patients with a radiation or drug-induced pulmonary disease requiring an interventional treatment; the patients with radiation pneumonitis who were 3 months post-radiotherapy and had no clinical symptoms could be enrolled;
9) patients with cardiac dysfunction, including but not limited to those with a left ventricular ejection fraction (LVEF) <50%, a history of congestive heart failure or a history of ventricular arrhythmias requiring treatment, hypokalemia, hereditary long QT syndrome, or a history of myocardial infarction or unstable angina within 6 months prior to the screening; patients with heart failure classified by the New York Heart Association as class ≥2;
10) patients with clinically significant abnormalities in QTc in the electrocardiogram (ECG) examination (QTc >450 msec [male] or QTc >470 msec [female] in the ECG examination at rest), or those requiring concomitant use of any medication known to prolong the QT interval and cause torsades de pointes ventricular tachycardia;
11) patients with poorly controlled hypertension (systolic blood pressure ≥ 160 mmHg or diastolic blood pressure ≥ 100 mmHg) after a drug treatment;
12) patients with uncontrolled hyperglycemia, or acute cholelithiasis, or those characterized by the susceptibility to acute pancreatitis;
13) patients with a severe or uncontrollable systemic disease (such as an unstable or uncompensated respiratory, cardiac, hepatic, or renal disease) who were expected to be unable to tolerate the investigational drug treatment, based on the investigators' judgment;
14) subjects who had not recovered from a previous surgical procedure and a previous anti-tumor treatment-related toxic reaction, which were assessed by the investigators as affecting the safety of the subjects;
15) patients with clinically significant active bacterial, fungal or viral infection, including those who were positive for hepatitis B surface antigen and had HBV DNA ≥ 2000 IU/ml (only in the patients who were positive for hepatitis B surface antigen), or those with positive results in the hepatitis C (those who were positive for hepatitis C antibody but had the HCV RNA polymerase chain reaction (PCR) < ULN were eligible for enrollment) or human immunodeficiency virus antibody test, or those with any uncontrolled infection;
16) patients who had taken a strong inducer or inhibitor of CYP3A4, or a CYP3A4 substrate with a narrow therapeutic window, within two weeks prior to the first administration of the investigational drug;
17) pregnant or breastfeeding female subjects;
18) women of childbearing age who were unwilling or unable to use an acceptable method of contraception throughout the entire treatment period of this trial and for 6 months after the last administration of the investigational drug (women of childbearing age included: any woman who had had menarche and had not successfully undergone artificial sterilization surgery (hysterectomy, bilateral tubal ligation, or bilateral oophorectomy) or was not menopausal); and fertile male patients who were unwilling or unable to use effective contraception if their partners were women of childbearing age;
19) subjects who were participating in other clinical researches or whose first administration was less than 4 weeks or 5 half-lives of the investigational drug from the end of receiving other investigational drugs, whichever was shorter;
20) patients with any mental illness or cognitive impairment that might limit their understanding and compliance with the informed consent form;
21) other situations deemed unsuitable for the participation of this research by the investigators.

### Criteria for the termination of treatment

The investigators might terminate the treatment for a subject if any of the following occurred:
the subject voluntarily requested to stop the treatment;
the investigators believed that the subject should discontinue the treatment with the investigational drug due to safety concerns (such as the occurrence of an intolerable adverse event in the subject).
pregnancy of the subject;
death;
the subject exhibited serious protocol violation, and the investigators believed that the protocol violation significantly affected the evaluation of the primary endpoint of the trial;
if the investigators assessed that a subject had progressive disease, the IRC needed to conduct a rapid review on the progressive disease; if the IRC assessed that the subject had progressive disease, the subject should discontinue the treatment; if the IRC assessed that the subject did not have progressive disease, the sponsors and investigators needed to discuss and reach a consensus, and should assess whether the subject should continue to receive the study treatment from a perspective of the risk and benefit of the subject.

Criteria for the withdraw from the research:
a subject might withdraw from the research for the following reasons:
   1. the subject died;
   2. the subject requested to withdraw from the research;
   3. the subject was lost to the follow-up;
   4. the sponsors terminated the research;
a subject might withdraw from the research at any stage of the research at the subject's own discretion.

A clear distinction should be made between the subjects permanently discontinuing the study drug (i.e., termination of the treatment) and withdrawing from the research. The subjects who permanently discontinued the study drug should remain in the research to complete the safety visits and survival follow-ups. For the subjects withdrawing from the research, they were encouraged to complete the relevant examinations and survival follow-ups within 7 days of deciding to withdraw, referring to the EOT visit (see the trial flowchart). A subject's withdrawal from the research should be recorded on the relevant page of the eCRF and in the subject's medical record, along with the reason for withdrawal.

Criteria for the withdraw from the research:
a subject might withdraw from the research for the following reasons:
   1. the subject died;
   2. the subject requested to withdraw from the research;
   3. the subject was lost to the follow-up;
   4. the sponsors terminated the research;
a subject might withdraw from the research at any stage of the research at the subject's own discretion.

A clear distinction should be made between the subjects permanently discontinuing the study drug (i.e., termination of the treatment) and withdrawing from the research. The subjects who permanently discontinued the study drug should remain in the research to complete the safety visits and survival follow-ups. For the subjects withdrawing from the research, they were encouraged to complete the relevant examinations and survival follow-ups within 7 days of deciding to withdraw, referring to the EOT visit (see the trial flowchart). A subject's withdrawal from the research should be recorded on the relevant page of the eCRF and in the subject's medical record, along with the reason for withdrawal.

The above content is a further detailed description of the present disclosure in conjunction with specific alternative embodiments, and it cannot be considered that the specific implementation of the present disclosure is limited to these descriptions. For those skilled in the art to which the present disclosure belongs, some simple deductions or substitutions can be made without departing from the concept of the present disclosure, and should be regarded as belonging to the scope of protection of the present disclosure.

## Claims

1. Use of a compound A or a pharmaceutically acceptable salt thereof in the manufacture of a medicament for the treatment of non-small cell lung cancer, wherein the compound A has the following structure:

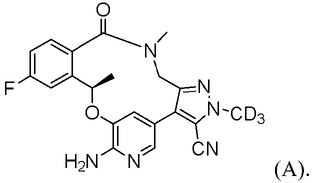

2. A method of treating non-small cell lung cancer in a patient, comprising administering to the patient a therapeutically effective amount of a compound A or a pharmaceutically acceptable salt thereof, wherein the compound A has the following structure:

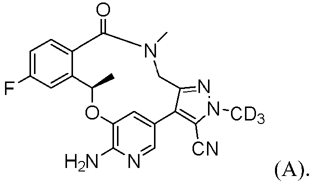

3. A compound A or a pharmaceutically acceptable salt thereof, for use in the treatment of non-small cell lung cancer, wherein the compound A has the following structure:

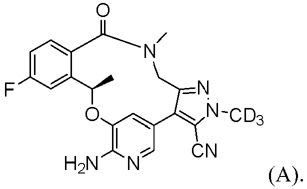

4. The use according to claim 1, or the method according to claim 2, or the compound for use according to claim 3, wherein the non-small cell lung cancer is ALK-positive non-small cell lung cancer; alternatively, wherein the non-small cell lung cancer is advanced ALK-positive non-small cell lung cancer; alternatively, wherein the ALK is ALK L1196M or ALK G1202R mutation; alternatively, wherein the non-small cell lung cancer is TP53-positive (including mutant and wild-type) ALK-positive non-small cell lung cancer; alternatively, the non-small cell lung cancer is TP53-mutated ALK-positive non-small cell lung cancer.

5. The use, method, or compound for use according to any one of claims 1 to 4, wherein the non-small cell lung cancer is adenocarcinoma.

6. The use, method, or compound for use according to any one of claims 1 to 5, wherein the non-small cell lung cancer is metastatic non-small cell lung cancer; alternatively, wherein the non-small cell lung cancer is CNS metastatic non-small cell lung cancer (e.g., intracranial metastatic non-small cell lung cancer).

7. The use, method, or compound for use according to any one of claims 1 to 6, wherein the non-small cell lung cancer is untreated.

8. The use, method, or compound for use according to any one of claims 1 to 6, wherein the non-small cell lung cancer has reached progressive disease after a treatment with another therapy;
alternatively, wherein the another therapy is selected from radiotherapy, pharmacotherapy and surgical therapy;
alternatively, wherein the pharmacotherapy is chemotherapy, targeted therapy, or immunotherapy;
alternatively, wherein the pharmacotherapy is chemotherapy;
alternatively, wherein the pharmacotherapy is targeted therapy;
alternatively, wherein the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody.

9. The use, method, or compound for use according to claim 8, wherein the non-small cell lung cancer has reached progressive disease after a treatment with an ALK inhibitor.

10. The use, method, or compound for use according to claim 9, wherein the ALK inhibitor is a first-generation or a second-generation ALK inhibitor; alternatively, the ALK inhibitor is selected from crizotinib, alectinib, ceritinib, ensartinib and brigatinib.

11. The use, method, or compound for use according to any one of claims 1 to 10, wherein the non-small cell lung cancer is in clinical stage III or IV.

12. The use, method, or compound for use according to any one of claims 1 to 11, wherein the patient has received a first-line or a higher-line therapy; alternatively, wherein the patient has received a second-line or a higher-line therapy; alternatively, the patient has received a first-line, a second-line, a third-line, a fourth-line, or a fifth-line therapy.

13. The use, method or compound for use according to claim 12, wherein the patient has experienced progressive disease or intolerance after receiving a first-line or a higher-line therapy; alternatively, wherein the patient has experienced progressive disease or intolerance after receiving a first-line or a second-line therapy.

14. The use, method, or compound for use according to claim 12 or 13, wherein the first-line or the higher-line therapy is selected from radiotherapy, pharmacotherapy and surgical therapy;
alternatively, wherein the pharmacotherapy is chemotherapy, targeted therapy, or immunotherapy;
alternatively, wherein the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody.

15. The use, method, or compound for use according to claim 14, wherein the patient has previously received chemotherapy; alternatively, wherein the chemotherapy drug is selected from carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, and pyridoxine, alternatively carboplatin, cisplatin, nedaplatin, lobaplatin, pemetrexed, docetaxel, endostatin, sodium cantharidate, or pyridoxine.

16. The use, method, or compound for use according to claim 14, wherein the patient has previously received targeted therapy; alternatively, the patient has previously received a treatment with an ALK inhibitor; alternatively, the patient has previously received a treatment with a second-generation ALK inhibitor, such as alectinib, ceritinib, ensartinib, or brigatinib; alternatively, the patient has previously received a treatment with crizotinib as the only ALK inhibitor.

17. The use, method, or compound for use according to claim 14, wherein the patient has not previously received a treatment with an ALK inhibitor (e.g., a third-generation ALK inhibitor).

18. The use, method, or compound for use according to any one of claims 1 to 17, wherein the dosage of the compound A is approximately 5 mg/day to approximately 180 mg/day;
alternatively, the dosage of the compound A is approximately 5 mg/day to approximately 125 mg/day;
alternatively, the dosage of the compound A is selected from approximately 5 mg/day, approximately 10 mg/day, approximately 20 mg/day, approximately 40 mg/day, approximately 60 mg/day, approximately 80 mg/day, approximately 100 mg/day, and approximately 125 mg/day;
alternatively, the dosage of the compound A is selected from approximately 40 mg/day, approximately 60 mg/day, approximately 80 mg/day, approximately 100 mg/day, and approximately 125 mg/day;
alternatively, the dosage of the compound A is selected from approximately 40 mg/day, approximately 60 mg/day, and approximately 80 mg/day;
alternatively, the dosage of the compound A is approximately 60 mg/day.

19. The use, method, or compound for use according to any one of claims 1 to 18, wherein the compound A is administered orally.

20. The use, method, or compound for use according to any one of claims 1 to 19, wherein the compound A is administered orally once daily, twice daily, or three times daily;
alternatively, the compound A is administered orally once daily.

21. The use, method, or compound for use according to any one of claims 1 to 20, wherein the compound A is administered until progressive disease, intolerable toxicity, or death.

22. The use, method, or compound for use according to any one of claims 1 to 21, wherein the patient has a CSF/plasma concentration ratio greater than approximately 40% of the compound A after the administration of the compound A; alternatively, has a CSF/plasma concentration ratio greater than approximately 50% of the compound A; alternatively, has a CSF/plasma concentration ratio greater than approximately 60% of the compound A.

23. The use according to claim 1, or the method according to claim 2, or the compound for use according to claim 3, wherein the non-small cell lung cancer is ROS1-positive non-small cell lung cancer; alternatively, wherein the non-small cell lung cancer is advanced ROS1-positive non-small cell lung cancer; alternatively, wherein the ROS1 is ROS1 G2032R mutation.

24. The use, method, or compound for use according to any one of claims 1 to 3 and 23, wherein the non-small cell lung cancer is adenocarcinoma.

25. The use, method, or compound for use according to any one of claims 1 to 3, 23 and 24, wherein the non-small cell lung cancer is metastatic non-small cell lung cancer; alternatively, wherein the non-small cell lung cancer is CNS metastatic non-small cell lung cancer (e.g., intracranial metastatic non-small cell lung cancer).

26. The use, method, or compound for use according to any one of claims 1 to 3 and 23 to 25, wherein the non-small cell lung cancer is untreated.

27. The use, method, or compound for use according to any one of claims 1 to 3 and 23 to 26, wherein the non-small cell lung cancer has reached progressive disease after a treatment with another therapy;
alternatively, wherein the another therapy is selected from radiotherapy, pharmacotherapy and surgical therapy;
alternatively, wherein the pharmacotherapy is chemotherapy, targeted therapy, or immunotherapy;
alternatively, wherein the pharmacotherapy is chemotherapy;
alternatively, wherein the pharmacotherapy is targeted therapy;
alternatively, wherein the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody.

28. The use, method, or compound for use according to claim 27, wherein the non-small cell lung cancer has reached progressive disease after a treatment with a ROS1 inhibitor.

29. The use, method, or compound for use according to claim 28, wherein the ROS1 inhibitor is a first-generation ROS1 inhibitor; alternatively, the ROS1 inhibitor is selected from crizotinib, alectinib, ceritinib, ensartinib and brigatinib.

30. The use, method, or compound for use according to any one of claims 1 to 3 and 23 to 29, wherein the non-small cell lung cancer is in clinical stage III or IV, alternatively clinical stage IV.

31. The use, method, or compound for use according to any one of claims 1 to 3 and 23 to 30, wherein the patient has received a first-line or a higher-line therapy; alternatively, wherein the patient has received a second-line therapy.

32. The use, method or compound for use according to claim 31, wherein the patient has experienced progressive disease or intolerance after receiving a first-line or a higher-line therapy; alternatively, wherein the patient has experienced progressive disease or intolerance after receiving a second-line therapy.

33. The use, method, or compound for use according to claim 31 or 32, wherein the first-line or the higher-line therapy is selected from radiotherapy, pharmacotherapy and surgical therapy;
alternatively, wherein the pharmacotherapy is chemotherapy, targeted therapy, or immunotherapy;
alternatively, wherein the drug of the pharmacotherapy is selected from one or more of the following: carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, pyridoxine, crizotinib, ceritinib, alectinib, ensartinib, brigatinib, anlotinib, gefitinib, conteltinib, ALK inhibitors, APG2499, bevacizumab, sintilimab, anti-PD-1 antibody, anti-PD-L1 antibody, anti-PD-L2 antibody, anti-CD137 antibody, and anti-CTLA-4 antibody.

34. The use, method, or compound for use according to claim 33, wherein the patient has previously received chemotherapy; alternatively, wherein the chemotherapy drug is selected from carboplatin, cisplatin, nedaplatin, lobaplatin, pemetrexed, paclitaxel, docetaxel, gemcitabine, endostatin, brucea javanica, gimeracil, oteracil, tegafur, sodium cantharidate, and pyridoxine, alternatively carboplatin, cisplatin, nedaplatin, lobaplatin, pemetrexed, docetaxel, endostatin, sodium cantharidate, or pyridoxine.

35. The use, method, or compound for use according to claim 33, wherein the patient has previously received targeted therapy; alternatively, the patient has previously received a treatment with a ROS1 inhibitor, such as alectinib, ceritinib, ensartinib, or brigatinib; alternatively, the patient has previously received a treatment with crizotinib as the only ROS1 inhibitor.

36. The use, method, or compound for use according to claim 33, wherein the patient has not previously received a treatment with a ROS1 inhibitor (e.g., a second-generation ROS1 inhibitor).

37. The use, method, or compound for use according to any one of claims 1 to 3 and 23 to 36, wherein the dosage of the compound A is approximately 5 mg/day to approximately 180 mg/day;
alternatively, the dosage of the compound A is approximately 5 mg/day to approximately 125 mg/day;
alternatively, the dosage of the compound A is selected from approximately 5 mg/day, approximately 10 mg/day, approximately 20 mg/day, approximately 40 mg/day, approximately 60 mg/day, approximately 80 mg/day, approximately 100 mg/day, and approximately 125 mg/day;
alternatively, the dosage of the compound A is selected from approximately 40 mg/day, approximately 60 mg/day, approximately 80 mg/day, approximately 100 mg/day, and approximately 125 mg/day;
alternatively, the dosage of the compound A is selected from approximately 40 mg/day, approximately 60 mg/day, and approximately 80 mg/day;
alternatively, the dosage of the compound A is approximately 60 mg/day.

38. The use, method, or compound for use according to any one of claims 1 to 3 and 23 to 37, wherein the compound A is administered orally.

39. The use, method, or compound for use according to any one of claims 1 to 3 and 23 to 38, wherein the compound A is administered orally once daily, twice daily, or three times daily;
alternatively, the compound A is administered orally once daily.

40. The use, method, or compound for use according to any one of claims 1 to 3 and 23 to 39, wherein the compound A is administered until progressive disease, intolerable toxicity, or death.

41. The use, method, or compound for use according to any one of claims 1 to 3 and 23 to 40, wherein the patient has a CSF/plasma concentration ratio greater than approximately 40% of the compound A after the administration of the compound A; alternatively, has a CSF/plasma concentration ratio greater than approximately 50% of the compound A; alternatively, has a CSF/plasma concentration ratio greater than approximately 60% of the compound A.

42. A method of determining the concentration of the compound A in the CNS fluid of a subject with ALK-positive or ROS1-positive CNS metastatic non-small cell lung cancer, comprising:
a. collecting a cerebrospinal fluid sample; and
b. measuring the concentration of the compound A in the CSF sample to determine the concentration of the compound A present in the CNS fluid.
